Europäisches Patentamt
European Patent Office
Office européen des brevets

(19)

(11) Veröffentlichungsnummer: **0 111 266**
**A2**

# (12) EUROPÄISCHE PATENTANMELDUNG

(21) Anmeldenummer: **83112100.9**

(22) Anmeldetag: **01.12.83**

(51) Int. Cl.³: **C 07 C 103/52**
C 12 Q 1/38, A 61 K 37/02

(30) Priorität: **03.12.82 CH 7047/82**
**01.07.83 CH 3635/83**

(43) Veröffentlichungstag der Anmeldung:
**20.06.84 Patentblatt 84/25**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Riniker, Bernhard, Dr.**
**Adlergasse 14**
**CH-4402 Frenkendorf(CH)**

(72) Erfinder: **Bühlmayer, Peter, Dr.**
**Im Baumgarten 3**
**CH-4144 Arlesheim(CH)**

(72) Erfinder: **Fuhrer, Walter, Dr.**
**Munzacherweg 11**
**CH-4402 Frenkendorf(CH)**

(74) Vertreter: **Zumstein, Fritz, Dr. et al,**
**Bräuhausstrasse 4**
**D-8000 München 2(DE)**

(54) **Substituierte Tetrapeptide.**

(57) Beschrieben sind Tetrapeptide der Formel I,

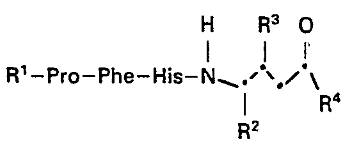

(I)

worin R¹ Wasserstoff oder Acyl, R² Alkyl oder Aralkl, R³ freies oder funktionell abgewandeltes Hydroxy, R⁴ freies oder substituiertes Amino oder freies oder verethertes Hydroxy und -Pro-, -Phe- sowie -His- die bivalenten Reste der Aminosäuren prolin, Phenylalanin beziehungsweise Histidin oder ihrer (D)-Isomeren bedeuten, Salze solcher Verbindungen mit salzbildenden Gruppen und Verfahren zu ihrer Herstellung.

Die Verbindungen hemmen die Wirkung des Enzyms Renin und können als Antihypertensiva und zur Behandlung der Herzinsuffizienz verwendet werden.

EP 0 111 266 A2

Croydon Printing Company Ltd.

CIBA-GEIGY AG

Basel (Schweiz)

4-14197/1+2/+

Substituierte Tetrapeptide

Die Erfindung betrifft substituierte Tetrapeptide der Formel I,

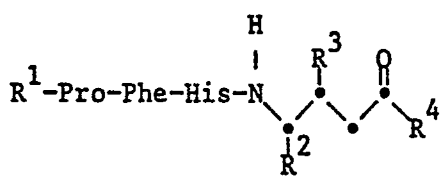

(I)

worin $R^1$ Wasserstoff oder Acyl, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, Salze solcher Verbindungen mit salzbildenden Gruppen, Verfahren zur Herstellung dieser Verbindungen, pharmazeutische Präparate, die diese Verbindungen enthalten, ihre Verwendung zur Herstellung von pharmazeutischen Präparaten oder als pharmakologisch wirksame Verbindungen und neue Zwischenprodukte zur Herstellung der Verbindungen der Formel I.

Acyl als Rest $R^1$ hat in erster Linie bis zu 80 C-Atome und ist hauptsächlich aliphatisches, aromatisches, aromatisch-aliphatisches, heterocyclisches oder heterocyclisch-aliphatisches Acyl. Insbesondere ist Acyl als Rest $R^1$ der Acylrest einer in der Natur vorkommenden (L)-Aminosäure, wie Histidin, oder ihres (D)-Isomeren, der Acylrest eines Dipeptids, bestehend aus zwei in der Natur vorkommenden

(L)-Aminosäuren oder deren D-Isomeren, der Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder ein anderer aliphatischer, aromatischer oder aromatisch-aliphatischer Acylrest mit bis zu 18 C-Atomen. Bevorzugterweise steht $R^1$ für den Dipeptidylrest H-Arg-Arg-, H-Pro-His- oder H-Ile-His-, worin jeweils beide Aminosäuren unabhängig voneinander die (D)- oder (L)-Konfiguration aufweisen können, in erster Linie jedoch die (L)-Konfiguration besitzen. Ein Oligopeptidrest $R^1$ weist am Carboxylende vorzugsweise einen Histidylrest auf, der wiederum vorzugsweise an einen Prolyl- oder Isoleucylrest gebunden sein kann. Die weitere Aminosäuresequenz ist im wesentlichen beliebig. Als Beispiele für einen Oligopeptidrest $R^1$ können somit der Rest H-Asp-Arg-Val-Tyr-Ile(oder Pro)-His- oder Teile davon genannt werden, die durch das Fehlen von 1-3 Aminosäuren vom Aminoende her charakterisiert sind.

Alkyl als Rest $R^2$ hat insbesondere 1-18 C-Atome und ist in erster Linie Niederalkyl, wobei das Präfix "Nieder"- hier und im folgenden einen Rest von und mit 1 bis und mit 7, hauptsächlich von und mit 1 bis und mit 4, C-Atomen bezeichnet. Bevorzugterweise steht ein Alkylrest $R^2$ für verzweigtes Niederalkyl, in allererster Linie für 2-Methyl-propyl. Aralkyl als Rest $R^2$ weist vornehmlich nicht mehr als 18 C-Atome auf und steht insbesondere für unsubstituiertes oder im Phenylrest substituiertes Phenylniederalkyl, in erster Linie für Benzyl. Als Arylsubstituenten seien insbesondere Niederalkyl, freies oder funktionell abgewandeltes Hydroxy und freies oder verestertes Carboxy genannt. Ein Arylrest kann einen oder mehrere, z.B. zwei oder drei und in der Regel nicht mehr als fünf, gleiche oder verschiedene Substituenten tragen.

Funktionell abgewandeltes Hydroxy als Arylsubstituent ist verestertes oder verethertes Hydroxy. Verestertes Hydroxy ist durch eine organische oder anorganische Säure verestertes Hydroxy, z.B. Acyloxy, Sulfonyloxy oder Halogen. Acyloxy ist insbesondere niederaliphatisches Acyloxy, cycloaliphatisches Acyloxy mit 3-6, vornehmlich 5 oder 6, Ringgliedern und höchstens 12 C-Atomen, monocyclisches Aroyloxy mit höchstens 12 C-Atomen oder aromatisch-aliphatisches Acyloxy mit höchstens 12 C-Atomen. Verethertes Hydroxy als Arylsubstituent ist insbesondere formal mit einem aliphatischen, aromatischen oder aromatisch-aliphatischen Alkohol mit höchstens 12 C-Atomen verethertes Hydroxy, in erster Linie Niederalkoxy.

Verestertes Carboxy als Arylsubstituent ist insbesondere formal mit einem aliphatischen, aromatischen oder aromatisch-aliphatischen Alkohol mit höchstens 12 C-Atomen verestertes Hydroxy, in erster Linie Niederalkoxycarbonyl.

Funktionell abgewandeltes Hydroxy als Rest $R^3$ ist insbesondere mit einer organischen Carbonsäure verestertes Hydroxy, daneben auch verethertes Hydroxy. Als veresternde oder verethernde Reste kommen hauptsächlich im menschlichen oder tierischen Organismus abspaltbare Reste in Frage, die nach der Abspaltung in der betreffenden Konzentration pharmakologisch unbedenkliche Spaltprodukte ergeben. Verestertes Hydroxy als Rest $R^3$ ist insbesondere Acyloxy mit bis zu 18 C-Atomen und vornehmlich niederaliphatisches Acyloxy, cycloaliphatisches Acyloxy mit 3-6, hauptsächlich 5 oder 6, Ringgliedern und höchstens 12 C-Atomen oder monocyclisches Aroyloxy oder aromatisch-aliphatisches Acyloxy mit jeweils höchstens 12 C-Atomen.

Verethertes Hydroxy als Rest $R^3$ ist insbesondere formal mit einem aliphatischen, aromatischen oder aromatisch-aliphatischen Alkohol mit höchstens 12 C-Atomen verethertes Hydroxy.

Substituiertes Amino als Rest $R^4$ ist z.B. durch Niederalkyl und/oder Arylniederalkyl mono- oder disubstituiertes Amino oder der amidisch gebundene Rest einer in der Natur vorkommenden Aminosäure oder ihres (D)-Isomeren oder eines Peptids mit 2 bis 6 Aminosäuren und höchstens 60 C-Atomen, das aus in der Natur vorkommenden Aminosäuren und/oder ihren (D)-Isomeren aufgebaut ist, wobei freie funktionelle Gruppen in diesen Aminosäure- oder Peptidresten gegebenenfalls in geschützter Form vorliegen, und ist in allererster Linie der Rest eines Peptids, mit 2 bis 6, vorzugsweise 2 oder 3, Aminosäuren, das am N-terminalen Ende eine der folgenden Aminosäuresequenzen aufweist
-Val-Tyr-Lys-, -Ile-His-Lys-, -Ile-His-Ser-, -Val-Tyr-Ser- oder -Ala-Sta-.

Verethertes Hydroxy als Rest $R^4$ ist formal mit einem aliphatischen, aromatischen oder aromatisch-aliphatischen Alkohol mit höchstens 12-C-Atomen verethertes Hydroxy, in erster Linie Niederalkoxy, oder insbesondere der Rest einer Hydroxycarbonsäure, die ihrerseits gegebenenfalls mit einer Aminosäure oder einem Dipeptid amidiert ist.

Die Verbindungen der Formel I können am $\underline{C}$-$R^2$ und $\underline{C}$-$R^3$ unabhängig voneinander die (R)- oder (S)-Konfiguration aufweisen, besitzen jedoch vorzugsweise die (S,S)-Konfiguration.

Die vor- und nachstehend verwendeten Allgemeinbegriffe haben im Rahmen der vorliegenden Beschreibung vorzugsweise die folgenden Bedeutungen:
Niederalkyl ist vor allem Methyl, Ethyl, n-Propyl, Isopropyl, n-Butyl oder Isobutyl, daneben z.B. auch sek.-Butyl oder tert.-Butyl, ferner n-Pentyl, Neopentyl, n-Hexyl oder n-Heptyl.
Halogen ist insbesondere Chlor oder Brom, daneben auch Fluor oder Jod.

In der Natur vorkommende Aminosäuren sind in erster Linie jene 20 Aminosäuren, die regelmässig in Proteinen vorkommen, nämlich Glycin (H-Gly-OH), Alanin (H-Ala-OH), Prolin (H-Pro-OH), Serin (H-Ser-OH), Cystein (H-Cys-OH), Tyrosin (H-Tyr-OH), Asparagin (H-Asn-OH), Glutamin (H-Gln-OH), Asparaginsäure (H-Asp-OH), Glutaminsäure (H-Glu-OH), Arginin (H-Arg-OH), Histidin (H-His-OH) und jene 8 Aminosäuren, die für den Menschen essentiell sind, nämlich Valin (H-Val-OH), Leucin (H-Leu-OH), Isoleucin (H-Ile-OH), Lysin (H-Lys-OH), Phenylalanin (H-Phe-OH), Tryptophan (H-Trp-OH), Methionin (H-Met-OH) und Threonin (H-Thr-OH). Von den anderen in der Natur vorkommenden Aminosäuren sind hier z.B. Hydroxyprolin, Sarkosin, (H-Sar-OH), β-Aminocarbonsäuren, z.B. β-Alanin (H-β-Ala-OH), γ-Aminocarbonsäuren, z.B. γ-Amino-buttersäure, α,γ-Diaminocarbonsäuren, z.B. Ornithin, und vor allem 4-Amino-3-hydroxy-6-methyl-heptancarbonsäure (Statin, hier abgekürzt "H-Sta-OH") zu nennen. Wenn nicht anders angegeben, bezeichnen die genannten Abkürzungen die Aminosäuren in ihrer natürlichen Konfiguration.

Eine Hydroxycarbonsäure ist in erster Linie eine Monohydroxymonocarbonsäure, insbesondere eine α-Hydroxy-monocarbonsäure, z.B. Glykolsäure oder Milchsäure (abgekürzt: H-Lac-OH).

Aromatische Reste sind in erster Linie gegebenenfalls substituierte Phenylreste. Substituenten aromatischer Reste sind insbesondere Niederalkyl, freies oder funktionell abgewandeltes Hydroxy und/oder freies oder verestertes Carboxy.

Aliphatische Reste sind in erster Linie acyclische, gesättigte oder durch ein oder zwei Doppelbindungen ungesättigte, unsubstituierte oder substituierte Kohlenwasserstoffreste ohne cyclische Substituenten, bei denen die freie Valenz von einem C-Atom ausgehen muss, das nicht durch Oxo substituiert ist, und sind in erster Linie unsubstituierte oder substituierte Alkylreste, z.B. Niederalkylreste. Bevorzugte Substituenten solcher aliphatischen Reste sind freies oder funktionell abgewandeltes Hydroxy oder Mercapto, freies oder verestertes Carboxy und/oder freies oder substituiertes Amino.

Heterocyclische Reste sind hauptsächlich mono- oder bicyclisch, vornehmlich mit 3-7 Ringgliedern pro geschlossener Ring, weisen als Heteroatome in erster Linie Sauerstoff, Schwefel und/oder Stickstoff auf und können bis zu vier Heteroatome enthalten. Im Falle von $R^1$ weisen sie vornehmlich 5-6, in allererster Linie 5, Ringglieder in einem monocyclischen heterocyclischen Ring auf, der als Heteroatome ausschliesslich ein oder zwei Stickstoffatome enthält und benzoannelliert sein kann.

Die Verbindungen der Formel I können als reine, optisch aktive Isomere oder als Isomerengemische vorliegen. Salzbildende Gruppen in einer Verbindung der Formel I sind entweder saure Gruppen, wie in erster Linie Carboxylgruppen oder daneben auch Sulfonsäuregruppen, oder basische Gruppen, wie in erster Linie solche, die basische Stickstoffatome enthalten, z.B. Aminogruppen. Salze von Verbindungen der Formel I mit salzbildenden Gruppen sind insbesondere pharmazeutisch verwendbare, nicht toxische Salze, wie Salze von sauren Verbindungen der Formel I mit Basen, insbesondere geeignete Alkalimetall-, wie Natrium- oder Kalium-, oder Erdalkalimetallsalze, z.B. Magnesium- oder Calciumsalze, ferner Zinksalze, oder Ammoniumsalze, inkl. solche mit organischen Aminen, wie gegebenenfalls durch Hydroxy substituierten mono-, di- oder tri-Alkylaminen, z.B. Diethylamin, Di-(2-hydroxyethyl)-amin, Triethylamin, N,N-Dimethyl-N-(2-hydroxyethyl)-amin, Tri-(2-hydroxy-ethyl)-amin oder N-Methyl-D-glucamin, oder Salze von basischen Verbindungen der Formel I mit Säuren, wie Salze mit anorganischen Säuren, z.B. Salzsäure, Schwefelsäure oder Phosphorsäure, oder Salze mit organischen Carbon-, Sulfon- oder Sulfosäuren, z.B. Essigsäure, Propionsäure, Glykolsäure, Bernsteinsäure, Maleinsäure, Hydroxymaleinsäure, Methylmaleinsäure, Fumarsäure, Aepfelsäure, Weinsäure, Zitronensäure, Benzoesäure, Zimtsäure, Mandelsäure, Salicylsäure, 4-Amino-salicylsäure, 2-Phenoxybenzoesäure, 2-Acetoxybenzoesäure, Embonsäure, Nicotinsäure oder Isonicotinsäure, ferner Aminosäuren, wie z.B. den obgenannten, sowie Methansulfonsäure, Ethansulfonsäure, 2-Hydroxyethansulfonsäure, Ethan-1,2-disulfonsäure, Benzolsulfonsäure, 4-Methyl-benzolsulfonsäure oder Naphthalin-2-sulfonsäure, oder mit anderen sauren organischen Verbindungen, wie Ascorbinsäure. Verbindungen der Formel I mit sauren und basischen Gruppen können auch innere Salze bilden.

Zur Isolierung oder Reinigung können auch pharmazeutisch ungeeignete Salze Verwendung finden. Zur therapeutischen Anwendung gelangen nur die pharmazeutisch verwendbaren, nicht-toxischen Salze, die deshalb bevorzugt werden.

Die Verbindungen der vorliegenden Erfindung weisen Enzym-hemmende Wirkungen auf; insbesondere hemmen sie die Wirkung des natürlichen Enzyms Renin. Letzteres gelangt aus den Nieren in das Blut und bewirkt dort die Spaltung eines Blutglycoproteins (Angiotensinogen) unter Bildung des Dekapeptids Angiotensin I, das dann in der Lunge, den Nieren und anderen Organen zum Octapeptid Angiotensin II gespalten wird. Letzteres erhöht den Blutdruck sowohl direkt durch arterielle Konstriktion, als auch indirekt durch die Freisetzung des Natrium zurückhaltenden Hormons Aldosteron aus den Nebennieren, womit ein Anstieg des extrazellulären Flüssigkeitsvolumens verbunden ist. Dieser Anstieg ist auf die Wirkung von Angiotensin II selber oder des daraus als Spaltprodukt gebildeten Heptapeptids Angiotensin III zurückzuführen. Hemmer der enzymatischen Aktivität von Renin bewirken eine Hemmung der Bildung von Angiotensin I. Als Folge davon entsteht weniger Angiotensin II und die verminderte Konzentration dieses aktiven Peptidhormons ist letztlich verantwortlich für die pharmakologische Wirkung von Renin-Hemmern.

Die Wirkung von Renin-Hemmern wird unter anderem mittels in vitro-Methoden erfasst, wobei die Verminderung der Bildung von Angiotensin I in verschiedenen Systemen (Humanplasma, gereinigtes humanes Renin zusammen mit synthetischem oder natürlichem Reninsubstrat) gemessen wird. Unter anderem wird der folgende in vitro Test verwendet: Ein Extrakt von menschlichem Renin aus der Niere (0,5 m GU [Milli-Goldblatt-Einheiten]/ml) wird eine Stunde lang bei 37°C und pH 7.2 in 1molarer wässeriger 2-N-(Tris-hydroxymethyl-methyl)-amino-ethansulfonsäure-Pufferlösung, die 23 µg/ml synthetisches Tetradecapeptid-Substrat (H-Asp-Arg-Val-Tyr-Ile-His-Pro-Phe-His-Leu-Leu-Val-Tyr-Ser-OH) enthält, inkubiert. Die Menge des gebildeten Angiotensin I wird mit einem Radioimmunoassay ermittelt. Die Hemmstoffe werden dem Inkubationsgemisch jeweils in verschiedenen Konzentrationen zugefügt. Als $IC_{50}$ wird diejenige Konzentration des jeweiligen Hemmstoffes bezeichnet, die die Bildung von Angiotensin I um 50% reduziert. Die Verbindungen der vorliegenden Erfindung zeigen in den vitro-Systemen Hemmwirkungen bei minimalen Dosen von etwa $10^{-7}$ bis etwa $10^{-10}$ Mol/l.

An salzverarmten Tieren bewirken Renin-Hemmer einen Blutdruckabfall. Da sich das menschliche Renin von dem anderer Spezies unterscheidet, werden zur Prüfung von Hemmern des humanen Renins Primaten (Marmosets, Callithrix jacchus) verwendet. Unter anderem wird der folgende in vivo Test benutzt:
Die Testverbindungen werden an normotensiven Marmosets beider Geschlechter mit einem Körpergewicht von etwa 300 g, die bei Bewusstsein sind, evaluiert. Blutdruck und Herzfrequenz werden mittels eines Katheters in der Oberschenkelarterie gemessen. Die Testsubstanzen werden über ein Katheter in die laterale Schwanzvene injiziert. Die endogene Freisetzung von Renin wird durch die intravenöse Injektion von Furosemid (5 mg/kg) angeregt. 30 Minuten nach der Injektion von Furosemid werden die Testsubstanzen entweder durch einmalige Injektion oder durch kontinuierliche Infusion verabreicht und ihre Wirkung auf den Blutdruck und die Herzfrequenz wird

evaluiert. Die Verbindungen der vorliegenden Erfindung sind in den in vivo-Systemen bei Dosen von etwa 0,1 bis etwa 1,0 mg/kg i.v. wirksam.

Die Verbindungen der vorliegenden Erfindung können als Antihypertensiva, ferner zur Behandlung von Herzinsuffizienz sowie zur Diagnostik der Ursachen von Bluthochdruck oder zu hoher Aldosteronspiegel Verwendung finden.

Pharmazeutisch nicht verwendbare Verbindungen der Formel I, in denen funktionelle Gruppen, wie Carboxyl-, Amino-, Hydroxy- oder Mercaptogruppen in geschützter Form vorliegen, sind in erster Linie Zwischenprodukte zur Herstellung der pharmazeutisch verwendbaren Verbindungen der Formel I und ebenfalls Gegenstand dieser Erfindung.

Verbindungen der Formel I, in denen funktionelle Gruppen, insbesondere Carboxyl- und Aminogruppen, vorzugsweise sämtliche Carboxyl- und Aminogruppen, durch pharmazeutisch verwendbare Schutzgruppen, z.B. Carboxylgruppen als Niederalkyl-, z.B. Methylester, und Aminogruppen als tert.-Butyloxycarbonyl- oder Benzyloxycarbonylamino, geschützt sind, sind als Verbindungen mit gegenüber den entsprechenden ungeschützten Verbindungen verlängerter Wirkungsdauer pharmazeutisch verwendbar und bevorzugter Gegenstand der Erfindung.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten

als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere

Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acylniederalk-1-en-1-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls, z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl, oder Propionyl, Halogenniederalkanoyl, wie 2-Halogenacetyl, insbesondere 2-Chlor-, 2-Brom-, 2-Jod-, 2,2,2-Trifluor- oder 2,2,2-Trichloracetyl, gegebenenfalls, z.B. durch Halogen, Niederalkoxy oder Nitro substituiertes Benzoyl, z.B. Benzoyl, 4-Chlorbenzoyl, 4-Methoxybenzoyl oder 4-Nitrobenzoyl, oder in 1-Stellung der Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B.

Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Jodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit z.B. bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes Phenyloxy-phenyl-phosphonyl, z.B. Phenyloxyphenyl-phosphonyl, Diniederalkylphosphinyl, z.B. Diäthylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylamino darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-en-1-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxy-carbonyl-prop-1-en-2-yl, z.B. 1-Aethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichloräthoxycarbonyl, ferner Trityl oder Formyl.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder

2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder -cycloaliphatische Kohlenwasserstoffreste, in erster Linie 1-Niederalkoxy-niederalkyl oder 1-Niederalkylthio-niederalkyl, z.B. Methoxymethyl, 1-Methoxyethyl, 1-Ethoxy-ethyl, 1-Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Eine Mercaptogruppe, wie z.B. in Cystein, kann insbesondere durch S-Alkylierung mit gegebenenfalls substituierten Alkylresten, Thioacetalbildung, S-Acylierung oder durch das Erstellen asymmetrischer Disulfid-Gruppierungen geschützt werden. Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls im Phenylteil, z.B. durch Methoxy, substituiertes Diphenylmethyl, wie 4,4'-Dimethoxydiphenyl-methyl, Triphenylmethyl, Trimethylsilyl, Benzyl-thiomethyl, Tetrahydropyranyl, Acylaminomethyl, Benzoyl, Benzyloxycarbonyl oder Aminocarbonyl, wie Ethylaminocarbonyl.

Der Schutz von Amidgruppen ist nur selten nötig. Bevorzugte Amidschutzgruppen sind z.B. 4-, 2,4-Di- oder 2,4,6-Trimethoxy-benzyl oder gegebenenfalls im Phenylrest durch Methyl oder Methoxy substituiertes Diphenylmethyl, z.B. 4,4'-Dimethoxy-diphenyl-methyl, wobei diese Gruppen an das Stickstoffatom gebunden sind.

Die Erfindung betrifft insbesondere Verbindungen der Formel I, worin $R^1$ Wasserstoff, einen aliphatischen, aromatischen oder aromatisch-aliphatischen Acylrest mit bis zu 18 C-Atomen, einen heterocyclischen oder heterocyclisch-aliphatischen Acylrest mit jeweils fünf oder

sechs Ringgliedern und ein oder zwei Stickstoffatomen im gegebenenfalls benzoannellierten heterocyclischen Ring und insgesamt nicht mehr als 18 C-Atomen oder den Acylrest eines Oligopeptids mit mehr als 18 und höchstens 60 C-Atomen, das aus höchstens 6 in der Natur vorkommenden Aminosäuren und/oder ihren (D)-Isomeren aufgebaut ist, wobei in solchen Aminosäuren vorhandene freie funktionelle Gruppen gegebenenfalls in geschützter Form vorliegen, $R^2$ Alkyl oder Aralkyl mit jeweils nicht mehr als 18 C-Atomen, $R^3$ freies oder verestertes Hydroxy mit höchstens 18 C-Atomen und $R^4$ freies oder durch Arylniederalkyl mit höchstens 18 C-Atomen oder Niederalkyl substituiertes Amino, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, oder freies oder verethertes Hydroxy mit höchstens 12 C-Atomen bedeuten, und Salze von solchen Verbindungen mit salzbildenden Gruppen.

Hauptsächlich betrifft die Erfindung Verbindungen der Formel I, worin $R^1$ Wasserstoff, den Rest einer in der Natur vorkommenden Aminosäure oder ihres (D)-Isomeren, oder den Rest eines aus höchstens 6 in der Natur vorkommenden Aminosäuren und/oder ihren (D)-Isomeren aufgebauten Peptids, wobei in diesen Resten vorhandene freie funktionelle Gruppen gegebenenfalls in geschützter Form vorliegen, $R^2$ Niederalkyl oder Phenylniederalkyl, $R^3$ freies Hydroxy und $R^4$ freies Amino oder Amino, das durch gegebenenfalls durch Phenyl substituiertes Niederalkyl substituiert ist, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, freies Hydroxy oder Niederalkoxy bedeuten, und Salze von solchen Verbindungen mit salzbildenden Gruppen.

Ganz besonders betrifft die Erfindung die obengenannten Verbindungen der Formel I, worin Amino und/oder Carboxy entweder als solches oder als gegebenenfalls substituiertes Benzyloxycarbonylamino oder $C_1$-$C_{18}$-Alkanoylamino beziehungsweise Alkoxycarbonyl mit 1-18 C-Atomen vorliegen, und Salze von solchen Verbindungen mit salzbildenden Gruppen.

Vornehmlich betrifft die Erfindung Verbindungen der Formel I, worin $R^1$ Wasserstoff, den Acylrest einer in der Natur vorkommenden Aminosäure, den Acylrest eines aus in der Natur vorkommenden Aminosäuren aufgebauten Dipeptids oder Niederalkanoyl, $R_2$ verzweigtes Niederalkyl, $R^3$ freies Hydroxy und $R^4$ freies oder durch Niederalkyl oder Benzyl substituiertes Amino, oder den N-terminalen Rest einer in der Natur vorkommenden Aminosäure oder eines aus solchen Aminosäuren aufgebauten Di- oder Tripeptids bedeuten, und Salze von solchen Verbindungen mit salzbildenden Gruppen.

Hervorzuheben sind Verbindungen der Formel I, worin $R^1$ den Acylrest eines aus in der Natur vorkommenden Aminosäuren aufgebauten Dipeptids, $R^2$ 2-Methyl-propyl, $R^3$ freies Hydroxy und $R^4$ den N-terminalen Rest eines aus in der Natur vorkommenden Aminosäuren aufgebauten Di- oder Tripeptids bedeuten, mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, und pharmazeutisch verwendbare Salze dieser Verbindungen.

Besonders hervorzuheben sind die obengenannten Verbindungen der Formel I, worin in den Resten $R^1$ und $R^4$ vorkommende Aminosäurereste sich von jenen 20 obengenannten Aminosäuren in ihrer natürlichen Konfiguration ableiten, die regelmässig in Proteinen vorkommen.

Ganz besonders hervorzuheben sind Verbindungen der Formel I, worin $R^1$ den Rest H-Pro-His- oder H-Ile-His-, $R^2$ 2-Methyl-propyl, $R^3$ freies Hydroxy und $R^4$ den Rest
-Val-Tyr-Lys-OH, -Ile-His-Lys-OH, -Ile-His-Ser-OH, -Val-Tyr-Ser-OH,

-Val-Tyr-OH, -Ile-His-OH, -Val-Tyr-$NH_2$, -Ile-His-$NH_2$ oder -Ala-Sta-OH bedeuten, und pharmazeutisch verwendbare Salze dieser Verbindungen.

In erster Linie betrifft die Erfindung die obengenannten Verbindungen der Formel I, worin die in Formel I verwendeten Abkürzungen -Pro-, -Phe- und -His- für die Reste der entsprechenden (L)-Aminosäuren stehen, und Salze von solchen Verbindungen mit salzbildenden Gruppen.

Bevorzugt sind auch die folgenden Verbindungen der Formel I und Salze von solchen Verbindungen mit mindestens einer salzbildenden Gruppe, worin

a) $R^1$ den Rest H-Ile-His-, $R^2$ 2-Methyl-propyl, $R^3$ freies Hydroxy und $R^4$ den Rest -Val-Tyr-Lys-OH, -Ile-His-Lys-OH, -Ile-His-Ser-OH, -Val-Tyr-Ser-OH, -Val-Tyr-OH, -Ile-His-OH, -Val-Tyr-$NH_2$, -Ile-His-$NH_2$ oder -Ala-Sta-OH bedeuten,

b) $R^1$ den Rest eines aus höchstens 6 in der Natur vorkommenden Aminosäuren und/oder ihren (D)-Isomeren aufgebauten Peptids, wobei in diesen Resten vorhandene freie funktionelle Gruppen gegebenenfalls in geschützter Form vorliegen, $R^2$ Niederalkyl oder Phenylniederalkyl, $R^3$ freies Hydroxy und $R^4$ freies Amino oder Amino, das durch gegebenenfalls durch Phenyl substituiertes Niederalkyl substituiert ist, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, freies Hydroxy oder Niederalkoxy bedeuten, und Salze von solchen Verbindungen mit salzbildenden Gruppen mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$,

c) $R^1$ den Acylrest eines aus in der Natur vorkommenden Aminosäuren aufgebauten Dipeptids oder Niederalkanoyl, $R_2$ verzweigtes Niederalkyl, $R^3$ freies Hydroxy und $R^4$ freies oder durch Niederalkyl oder

Benzyl substituiertes Amino, oder den N-terminalen Rest einer in der Natur vorkommenden Aminosäure oder eines aus solchen Aminosäuren aufgebauten Di- oder Tripeptids bedeuten, mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$,

d) $R^1$ den Acylrest eines Dipeptids, bestehend aus zwei in der Natur vorkommenden (L)-Aminosäuren oder deren (D)-Isomeren, den Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder einen anderen aliphatischen, aromatischen oder aromatisch-aliphatischen Acylrest mit bis zu 18 C-Atomen, der verschieden ist von dem Acylrest von N-unsubstituiertem oder N-substituiertem (L)- oder (D)-Histidin oder Sarkosin, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, mit Ausnahme der Verbindungen (H, Boc oder N-Isovaleryl)-Pro-His-Pro-Phe-His-Sta-Leu-Phe-$NH_2$ und (H, Boc, N-Acetyl oder N-Phenoxyacetyl)-Pro-Phe-His-Sta-Leu-Phe-$NH_2$,

e) $R^1$ den Acylrest eines Dipeptids, bestehend aus zwei in der Natur vorkommenden (L)-Aminosäuren oder deren D-Isomeren, den Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder einen anderen aliphatischen, aromatischen oder aromatisch-aliphatischen Acylrest mit bis zu 18 C-Atomen, der verschieden ist von dem Acylrest von N-unsubstituiertem oder N-substituiertem (L)- oder (D)-Histidin oder Sarkosin, $R^2$ Alkyl oder Aralkyl mit jeweils nicht mehr als 18 C-Atomen, $R^3$ freies oder verestertes Hydroxy mit höchstens 18 C-Atomen und $R^4$ freies oder durch Arylniederalkyl mit höchstens 18 C-Atomen oder Niederalkyl substituiertes Amino, den N-terminalen Rest einer in der Natur vorkommenden

Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, oder freies oder verethertes Hydroxy mit höchstens 12 C-Atomen bedeuten, mit Ausnahme der Verbindungen (H, Boc oder N-Isovaleryl)-Pro-His-Pro-Phe-His-Sta-Leu-Phe-$NH_2$ und (H, Boc, N-Acetyl oder N-Phenoxyacetyl)-Pro-Phe-His-Sta-Leu-Phe-$NH_2$, sowie Verbindungen der Formel I aus den obengenannten Gruppen und Salze von solchen Verbindungen mit salzbildenden Gruppen, die mindestens zwei Aminosäuren enthalten, die von α-Aminosäuren verschieden sind, insbesondere zwei Statinmoleküle, z.B. solche Verbindungen, die die Sequenz -Sta-Ala-Sta-, -Sta-Gly-Sta-, -Sta-Ile-Sta-, -Sta-Sta- oder -Sta-Sar-Sta- aufweisen, oder Verbindungen, die ß-Alanin, z.B. die Sequenz -Sta-ß-Ala-His, oder γ-Amino-buttersäure enthalten, sowie Verbindungen der Formel I, die mindestens eine Hydroxycarbonsäure, z.B. L-Milchsäure oder Glykolsäure an Stelle einer Aminosäure aufweisen, z.B. im Rest $R^4$ im Anschluss an die γ-Aminosäure, sowie Verbindungen der Formel I, worin $R^1$ für den Rest H-Arg-Arg- steht, dessen N-terminale Aminosäure gegebenenfalls in geschützter Form vorliegt, z.B. Verbindungen, worin $R^1$ den Rest Z-Arg-Arg- bedeutet.

In allererster Linie betrifft die Erfindung die in den Beispielen genannten Verbindungen der Formel I, vor allem Z-Arg-Arg-Pro-Phe-His-Sta-Ile-His-Lys(Boc)-$OCH_3$, und ihre pharmazeutisch verwendbaren Salze.

Die erfindungsgemässen Verbindungen der Formel I und Salze von solchen Verbindungen mit mindestens einer salzbildenden Gruppe werden nach an sich bekannten Verfahren erhalten, z.B. indem man

a) eine Amidbindung einer Verbindung der Formel I durch Umsetzung eines entsprechenden Bruchstücks mit einer freien Carboxylgruppe oder eines reaktionsfähigen Säurederivats davon mit einem komplementierenden Bruchstück mit einer freien Aminogruppe oder einem reaktionsfähigen Derivat davon mit aktivierter Aminogruppe, wobei in

den Reaktionskomponenten vorhandene freie funktionelle Gruppen mit Ausnahme der an der obengenannten Reaktion teilnehmenden Gruppen gegebenenfalls in geschützter Form vorliegen, herstellt und, wenn nötig, vorhandene Schutzgruppen abspaltet oder

b) zur Herstellung von Verbindungen der Formel I, worin $R^3$ freies Hydroxy bedeutet, die Ketogruppe in einer Verbindung der Formel II,

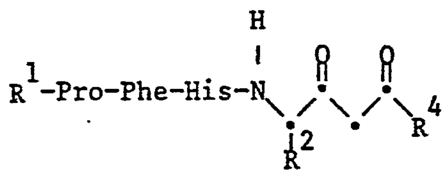

(II)

worin die Substituenten die obengenannten Bedeutungen haben, mit der Massgabe, dass in der Verbindung vorhandene freie funktionelle Gruppen mit Ausnahme der an der Reaktion teilnehmenden Ketogruppe gegebenenfalls in geschützter Form vorliegen, durch Umsetzung mit einem regioselektiven Reduktionsmittel zu einer Hydroxygruppe reduziert und, wenn nötig, vorhandene Schutzgruppen abspaltet oder

c) an die C=C-Doppelbindung einer Verbindung der Formel III,

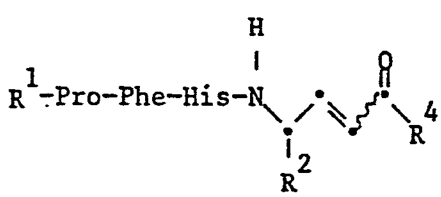

(III)

worin die Substituenten die obengenannten Bedeutungen haben, mit der Massgabe, dass in der Verbindung vorhandene freie funktionelle Gruppen mit Ausnahme der an der Reaktion teilnehmenden Doppelbindung gegebenenfalls in geschützter Form vorliegen, regioselektiv eine Verbindung der Formel $R^3$-H, worin $R^3$ die obengenannte Bedeutung hat, addiert und, wenn nötig, vorhandene Schutzgruppen abspaltet oder

d) in einer Verbindung der Formel IV,

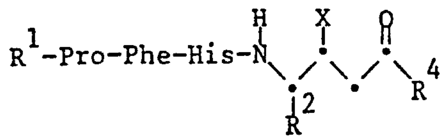

(IV)

worin X für eine gute Abgangsgruppe steht und die übrigen Substituenten die obengenannten Bedeutungen haben mit der Massgabe, dass in der Verbindung vorhandene freie funktionelle Gruppen mit Ausnahme der an der Reaktion teilnehmenden Gruppe gegebenenfalls in geschützter Form vorliegen, den Substituenten X mit einem, den Substituenten $R^3$ in nucleophiler Form bereitstellenden Reagenz gegen $R^3$ austauscht und, wenn nötig, vorhandene Schutzgruppen abspaltet oder

e) zur Herstellung einer Verbindung der Formel I, worin $R^4$ für freies oder substituiertes Amino steht, die Cyanogruppe in einer Verbindung der Formel V,

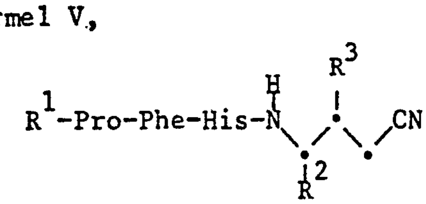

(V)

worin die Substituenten die obengenannten Bedeutungen haben, in eine gegebenenfalls N-substituierte Amidgruppe überführt oder

f) zur Herstellung einer Verbindung der Formel I, worin $R^3$ für freies Hydroxy steht, ein Epoxid der Formel VI,

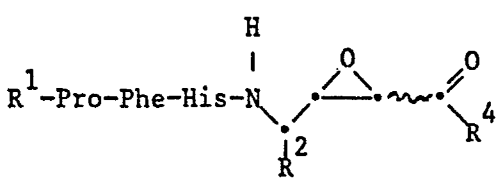

(VI)

worin die Substituenten die obengenannten Bedeutungen haben, mit der Massgabe, dass in der Verbindung vorhandene freie funktionelle Gruppen mit Ausnahme der an der Reaktion teilnehmenden Epoxygruppe gegebenenfalls in geschützter Form vorliegen, mit einem regioselektiven Reduktionsmittel zum entsprechenden Alkohol reduziert und, wenn nötig, vorhandene Schutzgruppen abspaltet oder

g) eine Verbindung der Formel VII,

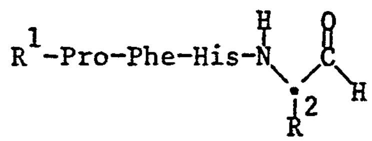

(VII)

worin die Substituenten die obengenannten Bedeutungen haben, mit der Massgabe, dass in der Verbindung vorhandene freie funktionelle Gruppen mit Ausnahme der an der Reaktion teilnehmenden Aldehydgruppe gegebenenfalls in geschützter Form vorliegen, mit einer Verbindung der Formel VIII,

$$R^5-CH_2-\overset{O}{\overset{\|}{C}}-R^4 \qquad \text{(VIII)},$$

worin $R^5$ für Halogen mit einem Atomgewicht zwischen 35 und 127 steht und $R^4$ die obengenannte Bedeutung hat, mit der Massgabe, dass in einer Verbindung der Formel VIII vorhandene freie funktionelle Gruppen mit Ausnahme der an der Reaktion beteiligten Gruppe gegebenenfalls in geschützter Form vorliegen, nach Aktivierung mit Zink (analog einer Reformatsky-Reaktion) umsetzt und, wenn nötig, vorhandene Schutzgruppen abspaltet oder

h) in einer Verbindung der Formel I, worin die Substituenten die obengenannten Bedeutungen haben, mit der Massgabe, dass in einer Verbindung der Formel I mindestens eine freie Amino-, Hydroxy-, Mercapto- oder Carboxygruppe vorhanden ist und die übrigen funktionellen Gruppen gegebenenfalls in geschützter Form vorliegen, mindestens eine freie Amino-, Hydroxy- oder Mercaptogruppe mit einer den einzuführenden Rest enthaltenden Carbonsäure oder einem reaktionsfähigen Derivat davon acyliert oder mindestens eine freie Carboxygruppe oder ein reaktionsfähiges Derivat davon verestert und, wenn nötig, vorhandene Schutzgruppen abspaltet oder

i) in einer Verbindung der Formel I, worin die Substituenten die obengenannten Bedeutungen haben, mit der Massgabe, dass in einer Verbindung der Formel I mindestens eine freie Amino-, Hydroxy- oder Mercaptogruppe vorhanden ist und die übrigen funktionellen Gruppen gegebenenfalls in geschützter Form vorliegen, mindestens eine freie Aminogruppe alkyliert, oder eine freie Hydroxy- oder Mercaptogruppe verethert und, wenn nötig, vorhandene Schutzgruppen abspaltet oder

j) zur Herstellung einer Verbindung der Formel I, worin $R^4$ für den N-terminalen Rest einer gegebenenfalls entsprechend substituierten Aminosäure steht, ein Lacton der Formel IX,

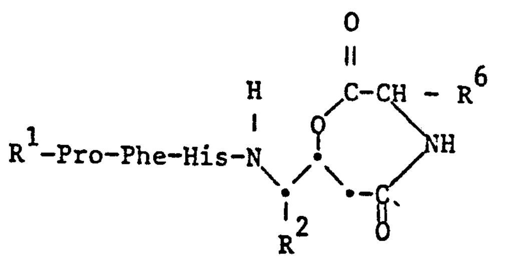

(IX),

worin $R^1$ und $R^2$ die obengenannten Bedeutungen haben und $R^6$ den entsprechenden Rest in einer in der Natur vorkommenden Aminosäure der Formel X oder ihrem (D)-Isomeren

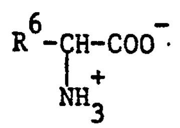

(X)

die über eine Carboxyl- oder Aminogruppe im Rest $R^6$ amidisch mit einer weiteren in der Natur vorkommenden Aminosäure oder mit einem aus in der Natur vorkommenden Aminosäuren oder ihren (D)-Isomeren aufgebauten Peptid mit 2-5 Aminosäuren verbunden sein kann, wobei freie funktionelle Gruppen in diesen Aminosäureresten gegebenenfalls in abgewandelter Form vorliegen, bedeutet, aufspaltet oder

k) zur Herstellung einer Verbindung mit mindestens einer freien funktionellen Gruppe in einer Verbindung der Formel I, worin die Substituenten die obengenannten Bedeutungen haben, mit der Massgabe, dass in einer Verbindung der Formel I mindestens eine funktionelle Gruppe durch eine leicht abspaltbare Schutzgruppe geschützt ist, die vorhandenen Schutzgruppen, gegebenenfalls stufenweise, abspaltet, und, wenn erwünscht, nach Ausführung eines der vorstehend genannten Verfahren a-k) oder eines beliebigen anderen Verfahrens zur Herstellung einer Verbindung der Formel I eine erhaltene Verbindung der Formel I mit mindestens einer salzbildenden Gruppe in ihr Salz oder ein erhaltenes Salz in die freie Verbindung oder in ein anderes Salz überführt und/ oder gegebenenfalls erhaltene Stereoisomerengemische auftrennt und/ oder eine erhaltene Verbindung der Formel I epimerisiert.

Die Abspaltung einer Schutzgruppe am Schluss der Verfahrensvarianten a-d) und f-i) ist nötig, wenn das gewünschte Produkt die betreffende Schutzgruppe nicht enthält.

Die Erfindung betrifft auch die nach irgendeinem der obengenannten Verfahren erhältlichen Verbindungen und ihre Salze.

Verfahren a) (Herstellung einer Amidbindung):

Reaktionsfähige Carbonsäurederivate eines Bruchstücks einer Verbindung der Formel I mit freier Carboxylgruppe sind in erster Linie aktivierte Ester oder reaktionsfähige Anhydride, ferner reaktionsfähige cyclische Amide; dabei können reaktionsfähige Säurederivate auch <u>in situ</u> gebildet werden.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronenanziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol, 2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. durch Nitro, substituierte Phenyl-thioester (die man z.B. durch Behandeln der entsprechenden Säure mit gegebenenfalls, z.B. durch Nitro, substituierten Thiophenolen, u.a. mit Hilfe der Anhydrid- oder Carbodiimid-Methode, erhalten kann; Methode der aktivierten Thiolester), oder Amino- oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid, N-Hydroxy-5-norbornen-2,3-dicarbonsäureimid oder 1-Hydroxybenztriazol, z.B. nach der Anhydrid- oder Carbodiimid-Methode, erhalten kann; Methode der aktivierten N-Hydroxyester).

Anhydride von Säuren können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden

Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln der entsprechenden Säure mit Phosphoroxychlorid erhalten kann; Phosphoroxychloridmethode) Anhydride mit anderen Phosphorsäurederivaten (z.B. solchen, die man mit Phenyl-N-phenyl-phosphoramidochloridat erhalten kann) oder mit Phosphorigsäurederivaten, oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischen Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Ein komplementierendes Bruchstück mit freier Aminogruppe ist Ammoniak oder ein primäres oder sekundäres Amin.

Die an der Reaktion teilnehmende Aminogruppe in einem komplementierenden Bruchstück einer Verbindung der Formel I liegt bevorzugterweise in freier Form vor, insbesondere wenn die damit reagierende Carboxygruppe in reaktionsfähiger Form vorliegt, sie kann aber auch selbst derivatisiert sein, d.h. z.B. durch Reaktion mit einem Phosphit, wie Diäthylchlorphosphit, 1,2-Phenylen-chlorphosphit, Ethyl-dichlor-phosphit, Ethylen-chlor-phosphit oder Tetraethylpyrophosphit, aktiviert worden sein. Eine reaktionsfähige Form eines solchen komplementierenden Bruchstücks ist z.B. auch ein Carbaminsäurehalogenid oder ein Isocyanat, wobei die an der Reaktion teilnehmende Aminogruppe an Halogencarbonyl, z.B. Chlorcarbonyl, gebunden ist beziehungsweise als Isocyanatgruppe vorliegt, wobei im letzteren Falle nur Verbindungen der Formel I zugänglich sind, die am Stickstoffatom der durch die Reaktion gebildeten Amidgruppe ein Wasserstoffatom tragen.

Ist das komplementierende Bruchstück mit freier Aminogruppe Ammoniak oder ein durch Niederalkyl oder Arylniederalkyl mono- oder disubstituiertes Amin, so stellt auch ein entsprechender Harnstoff eine reaktionsfähige Form dar. Beispielsweise erhält man beim Erhitzen äquimolarer Mengen eines solchen Harnstoffs und der Komponente mit freier Carboxylgruppe entsprechende Verbindungen der Formel I in guter Ausbeute.

Ist das komplementierende Bruchstück Dimethylamin, so stellt z.B. auch Dimethylformamid eine reaktionsfähige Form dafür dar.

Schutzgruppen von gegebenenfalls vorhandenen funktionellen Gruppen sind z.B. die obengenannten Schutzgruppen.

Die Reaktion kann in an sich bekannter Weise durchgeführt werden, wobei die Reaktionsbedingungen in erster Linie davon abhängen, ob

und wie die an der Reaktion teilnehmende Carboxylgruppe aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +200°C, in einem geschlossenen Reaktionsgefäss und/oder in der Atmosphäre eines Inertgases, z.B. Stickstoff.

Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Wie erwähnt können reaktionsfähige Säurederivate auch in situ gebildet werden. So kann man z.B. N,N'-disubstituierte Amidinoester in situ bilden, indem man das Gemisch des Bruchstücks mit freier Carboxylgruppe und des komplementierenden Bruchstücks mit freier Aminogruppe in Gegenwart eines geeigneten N,N'-disubstituierten Carbodiimids, z.B. N,N'-Dicyclohexylcarbodiimid, zur Reaktion bringt. Ferner kann man Amino- oder Amidoester von solchen Säuren in Gegenwart der zu acylierenden Aminokomponente bilden, indem man das Gemisch der entsprechenden Säure- und Amino-Ausgangsstoffe in Gegenwart eines N,N'-disubstituierten Carbodiimids, z.B. N,N'-Dicyclohexyl-carbodiimid, und eines N-Hydroxy-amins oder N-Hydroxy-amids, z.B. N-Hydroxysuccinimid, gegebenenfalls in Anwesenheit einer

geeigneten Base, z.B. 4-Dimethylamino-pyridin, umsetzt.

Die Ausgangsstoffe zur Durchführung von Verfahren a) sind bekannt oder können nach an sich bekannten Verfahren hergestellt werden, z.B. aus den betreffenden Aminosäuren analog dem vorstehend beschriebenen Verfahren.

Verfahren b) (Reduktion einer Ketogruppe):

Solche regioselektiven Reduktionsmittel können verwendet werden, die unter den Reaktionsbedingungen eine isolierte Ketogruppe genügend schneller als Amidgruppen reduzieren.

In erster Linie zu nennen sind geeignete Borhydride, wie Alkalimetallborhydride, insbesondere Natriumborhydrid, Lithiumborhydrid oder Natriumcyanborhydrid, oder geeignete Aluminiumhydride, wie sterisch gehinderte Alkalimetallniederalkoxyaluminiumhydride, z.B. Lithium-tris-tert.butoxy-aluminiumhydrid.

Die Reduktion kann auch mit Wasserstoff in Gegenwart geeigneter Schwermetallkatalysatoren, z.B. Raney-Nickel, Platin- oder Palladiumkatalysatoren, oder nach Meerwein-Ponndorf-Verley mit Hilfe von Aluminiumalkanolaten, bevorzugt Aluminium-2-propanolat oder -ethanolat, durchgeführt werden.

Die Reduktion kann vorzugsweise mit stöchiometrischen Mengen oder, wenn es aufgrund unerwünschter Nebenreaktionen, z.B. mit dem Lösungsmittel, notwendig ist, einem sinnvoll bemessenen Ueberschuss des Reduktionsmittels in einem inerten Lösungsmittel bei Temperaturen zwischen -80°C und +180°C, vorzugsweise zwischen -20°C und +100°C, wenn nötig, unter Schutzgas, z.B. Argon, durchgeführt werden.

Die Ausgangsketone der Formel II sind nach an sich bekannten Verfahren, z.B. durch Claisen-Kondensation eines Esters, z.B. Ethylesters, einer Säure der Formel XI,

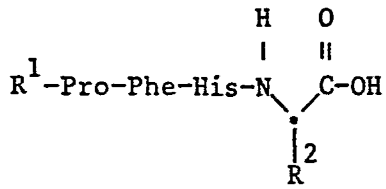

(XI)

worin die Substituenten die obengenannten Bedeutungen haben, mit der Massgabe, dass in der Verbindung vorhandene freie funktionelle Gruppen mit Ausnahme der an der Reaktion teilnehmende Estergruppe gegebenenfalls in geschützter Form vorliegen, mit einem Essigsäureester, Amidierung des resultierenden Esters mit einem Amin $R^4$-H und nachfolgende Abspaltung der Schutzgruppen, erhältlich.

Alternativ kann man auch eine entsprechende, durch Claisen-Kondensation erhältliche γ-Amino-β-oxo-carbonsäure nach den bei Verfahren a) beschriebenen Methoden in die Peptidkette einbauen.

Verfahren c) (Addition an ein Olefin):

Bei der Verbindung der Formel $R^3$-H handelt es sich je nach der Bedeutung von $R^3$ z.B. um Wasser, einen Alkohol, eine Carbonsäure oder eine Halogenwasserstoffsäure. Die Reaktion wird vorzugsweise unter saurer oder basischer Katalyse durchgeführt.

Das Ausgangsolefin der Formel III ist nach an sich bekannten Methoden zugänglich, z.B. durch Einbau einer α,β-ungesättigten Aminosäure der Formel XII,

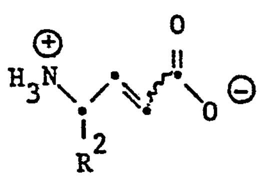

(XII)

worin $R^2$ die obengenannte Bedeutung hat, in die Peptidkette nach den bei Verfahren a) beschriebenen Methoden.

Die Aminosäure der Formel XII ist z.B. aus einem gegebenenfalls in geschützter Form vorliegenden α-Aminoaldehyd und einem Malonsäurederivat, z.B. Malodinitril oder Malonsäurediethylester, nach Knoevenagel-Doebner erhältlich.

Verfahren d) (nucleophile Substitution):

Eine Abgangsgruppe X ist insbesondere mit einer starken anorganischen oder organischen Säure verestertes Hydroxy, wie mit einer Mineralsäure, z.B. Halogenwasserstoffsäure, wie Chlorwasserstoff-, Bromwasserstoff- oder Jodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon- oder p-Toluolsulfonsäure verestertes Hydroxy oder mit Stickstoffwasserstoffsäure verestertes Hydroxy.

Ein den Substituenten $R^3$ in nucleophiler Form bereitstellendes Reagenz ist je nach der Bedeutung von $R^3$ z.B. Wasser, ein Alkohol oder das Salz einer Carbonsäure.

Vorzugsweise werden die Reaktionsbedingungen so gewählt, dass die Reaktion im wesentlichen als nucleophile Substitution zweiter Ordnung abläuft. z.B. kann man eine Verbindung der Formel IV, worin X für eine Abgangsgruppe mit hoher Polarisierbarkeit, z.B. für Jod, steht, in einem dipolar aprotischen Lösungsmittel, z.B. Aceton, Acetonitril, Nitromethan oder Dimethylformamid, mit dem Cäsiumsalz einer Carbonsäure umsetzen.

Verfahren e) (Ueberführung einer Cyanogruppe in eine Amidgruppe):

Die Ueberführung kann durch partielle Hydrolyse, im Sinne einer Graf-Ritter-Reaktion oder auf dem Weg über Carbonsäureesterimid-Salze erfolgen. Die Bedingungen zur Hydrolyse einer Verbindung der Formel V müssen so gewählt werden, dass die Reaktion auf der Stufe des Amids angehalten werden kann und nicht in die freie Carbonsäure gebildet wird. Zu diesem Zweck am generellsten geeignet ist die Hydrolyse mit Säuren, wobei man je nach den in einer Verbindung der Formel V vorhandenen Substituenten insbesondere wählen kann zwischen 80%iger Schwefelsäure (unter Erwärmen), Polyphosphorsäure (bei 110-150°), Bromwasserstoff/Eisessig (Raumtemperatur), Ameisensäure (ohne Lösungsmittel), Chlorwasserstoffgas in ätherischer Lösung gefolgt von Zugabe von Wasser oder wässeriger Salzsäure, oder Borhalogeniden/1 Aequivalent Wasser.

In einigen Fällen gelingt auch die alkalische Hydrolyse, insbesondere nach der Methode von Radziszewski mit Wasserstoffperoxid in Gegenwart von Alkalien bei mässiger Temperatur.

Mit Hilfe der Graf-Ritter-Reaktion gelingt die Herstellung N-substituierter Amide aus den Nitrilen der Formel V. Hierzu setzt man die Nitrile in Gegenwart einer starken Säure, vornehmlich 85-90%iger Schwefelsäure, oder auch Polyphosphorsäure, Ameisensäure, Bortrifluorid oder anderen Lewis-Säuren, nicht jedoch Aluminiumchlorid, mit Verbindungen um, die in dem sauren Medium Carbeniumionen bilden können, also z.B. mit Olefinen, wie Propylen, oder Alkoholen, wie Benzylalkohol.

Entsprechend einer Variante der Graf-Ritter-Reaktion kann man auch mit Quecksilber(II)-nitrat katalysieren und anschliessend mit Natriumborhydrid reduzieren.

Die Carbonsäureesterimide erhält man z.B. durch säurekatalysierte Anlagerung von Alkoholen an die Nitrile. Aus den Esterimiden erhält man die Amide im Sinne einer Pinner-Spaltung durch thermischen Zerfall der Esterimid-Salze bei Temperaturen oberhalb von etwa 80°C.

Nitrile der Formel V können beispielsweise durch eine Kolbe-Synthese aus den entsprechenden primären Halogeniden mit Cyanidionen im Sinne einer nucleophilen Substitution hergestellt werden. Alternativ kann man eine Verbindung der Formel XIII,

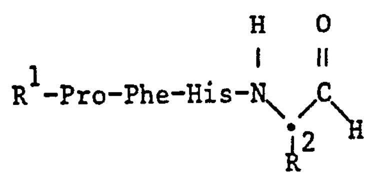

(XIII)

worin die Substituenten die obengenannten Bedeutungen haben, mit der Massgabe, dass in der Verbindung vorhandene freie funktionelle Gruppen mit Ausnahme der an der Reaktion teilnehmenden Aldehydgruppe gegebenenfalls in geschützter Form vorliegen, mit vorzugsweise einem Ueberschuss von Acetonitril in Gegenwart vorzugsweise katalytischer Mengen einer starken Base umsetzen.

Verfahren f) (Reduktion eines Epoxids):

Solche regioselektiven Reduktionsmittel können verwendet werden, die unter den Reaktionsbedingungen eine Epoxygruppe genügend schneller als Amidgruppen reduzieren und das Epoxid so öffnen, dass ein genügend und möglichst grosser Anteil der Reaktionsprodukte die neugebildete Hydroxylgruppe in der der Formel I entsprechenden Position trägt, z.B. Lithiumborhydrid oder Natriumcyanborhydrid/Bortrifluorid-Etherat.

Mit dem letztgenannten Reagenz lässt sich die Reaktion z.B. so durchführen, dass man zu 1 Mol der Verbindung der Formel VI und einem Ueberschuss, z.B. 1,4-3 Mol, Natriumcyanborhydrid in Tetrahydrofuran bei erhöhter Temperatur, z.B. unter Rückfluss, eine Lösung von Bortrifluorid-etherat, $BF_3 \cdot O(C_2H_5)_2$, in Tetrahydrofuran so zugibt, dass der pH-Wert der Reaktionslösung in der Nähe des Umschlagpunktes des Indikators Bromkresolgrün gehalten wird.

Das Epoxid der Formel VI kann nach an sich bekannten Methoden, z.B. durch Epoxidierung einer ungesättigten Aminosäure der Formel XII und Einbau der erhaltenen Aminosäure in die Peptidkette gemäss Verfahren a) hergestellt werden.

Verfahren g) (Reformatsky-Reaktion):

Die Reaktion kann z.B. so durchgeführt werden, dass man zunächst eine Verbindung der Formel VIII, z.B. eine solche, worin $R^5$ für Brom steht, mit vorzugsweise stöchiometrischen Mengen Zink in einem Alkohol bei erhöhter Temperatur, z.B. in siedendem Methanol, aktiviert und gegebenenfalls nach Zugabe von Tetrahydrofuran zur Erhöhung der Löslichkeit bei einer Temperatur zwischen etwa -30°C und +60°C, vorzugsweise zwischen 0°C und +20°C mit dem Aldehyd der Formel VII umsetzt.

Die Ausgangsprodukte der Formel VII sind nach an sich bekannten Methoden erhältlich, z.B. durch Reduktion eines entsprechenden Säurechlorids, z.B. mit Lithium-tris-tert.-butoxy-aluminiumhydrid oder nach Rosenmund. Man kann auch zunächst, z.B. ausgehend von der entsprechenden Aminosäure, den endständigen Aminoaldehyd herstellen und diesen dann nach den bei Verfahren a) beschriebenen Methoden in die Peptidkette einbauen.

Die Ausgangsstoffe der Formel VIII sind ebenfalls nach an sich bekannten Verfahren, z.B. durch Amidierung der entsprechenden α-Halogen-Essigsäure, wie bei Verfahren a) beschrieben, erhältlich.

Verfahren h) (Acylierung):

Das reaktionsfähige Derivat einer als Acylierungsmittel verwendeten Carbonsäure ist z.B. eines der bei Verfahren a) genannten.

Das reaktionsfähige Derivat einer zu veresternden Carboxylgruppe in einer Verbindung der Formel I ist z.B. eines der bei Verfahren a) genannten oder ein reaktionsfähiges Salz, z.B. ein Cäsiumsalz.

Zur Veresterung einer Carboxylgruppe in einer Verbindung der Formel I kann man entweder die freie Säure oder vorzugsweise eines der bei Verfahren a) genannten reaktionsfähigen Carbonsäurederivate mit einem Alkohol umsetzen oder die freie Säure oder ein reaktionsfähiges Carbonsäuresalz mit einem Veresterungsmittel, z.B. einem reaktionsfähigen Derivat eines Alkohols, umsetzen. Z.B. kann man das Cäsiumsalz einer Carbonsäure mit einem Halogenid umsetzen.

Geeignete Mittel zur Veresterung einer Carboxylgruppe in einer Verbindung der Formel I sind beispielsweise entsprechende Diazoverbindungen, wie gegebenenfalls substituierte Diazoniederalkane, z.B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers,wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veresterung einer Carboxylgruppe in einer Verbindung der Formel I sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate,

wie Dimethylsulfat, ferner Fluorsulfonsäureester, wie -niederalkylester, z.B. Fluorsulfonsäuremethylester, oder gegebenenfalls Halogensubstituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organischen Basen, wie üblicherweise sterisch gehinderte Triniederalkylamine, z.B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20°C bis etwa +50°C und, wenn notwendig, in einem geschlossenen Gefäss und/oder in einer Inertgas-, z.B. Stickstoffatmosphäre, gearbeitet wird.

Weitere Mittel zur Veresterung einer Carboxylgruppe in einer Verbindung der Formel I sind entsprechende trisubstituierte Oxonium-Salze (sogenannte Meerweinsalze), oder disubstituierte Carbenium- oder Haloniumsalze, worin die Substituenten die veresternden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat, -hexafluorphosphat oder tetrafluorborat, Dimethoxycarbeniumhexafluorphosphat oder Dimethylbromoniumhexafluorantimonat. Man verwendet diese Veresterungsmittel vorzugsweise in einem inerten Lösungsmittel, wie einem Ether oder einem halogenierten Kohlenwasserstoff, z.B. Diethylether, Tetrahydrofuran oder Methylenchlorid, oder in einem

Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa +50°C, wenn notwendig, in einem geschlossenen Gefäss und/oder in einer Inertgas-, z.B. Stickstoffatmosphäre.

Die Veresterung von freiem Carboxyl mit dem gewünschten freien Alkohol wird in Gegenwart eines geeigneten Kondensationsmittels durchgeführt. Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Die Kondensationsreaktion wird vorzugsweise in einem wasserfreien Reaktionsmedium, vorzugsweise in Gegenwart eines Lösungs- oder Verdünnungsmittels, z.B. Methylenchlorid, Dimethylformamid, Acetonitril oder Tetrahydrofuran und, wenn notwendig, unter Kühlen oder Erwärmen und/oder in einer Inertgasatmosphäre durchgeführt.

Zur Acylierung einer Amino-, Hydroxy- oder Mercaptogruppe in einer Verbindung der Formel I behandelt man das Ausgangsmaterial der Formel I mit einem, den gewünschten Acylrest einer organischen Carbonsäure einführenden Acylierungsmittel. Dabei verwendet man die entsprechende Carbonsäure oder ein reaktionsfähiges Derivat davon, insbesondere ein Anhydrid, inkl. ein gemischtes oder inneres Anhydrid einer solchen Säure. Gemischte Anhydride sind z.B. diejenigen mit Halogenwasserstoffsäuren, d.h. die entsprechenden Säurehalogenide, insbesondere -chloride, ferner mit Cyanwasserstoffsäure,oder dann diejenigen mit geeigneten Kohlensäurehalbderivaten, wie entsprechenden -halbestern (wie die z.B. mit einem Halogen-ameisensäure-niederalkyl, wie Chlorameisensäure-ethylester oder -isobutylester, gebildeten gemischten Anhydride) oder mit gegebenenfalls substituierten, z.B. Halogen, wie

Chlor, enthaltenden Niederalkancarbonsäuren (wie die mit Pivalinsäurechlorid oder Trichloressigsäurechlorid gebildeten gemischten Anhydride). Innere Anhydride sind z.B. diejenigen von organischen Carbonsäuren, d.h. Ketene, wie Keten oder Diketen, oder diejenigen von Carbamin- oder Thiocarbaminsäuren, d.h. Isocyanate oder Isothiocyanate. Weitere reaktionsfähige, als Acylierungsmittel verwendbare Derivate von organischen Carbonsäuren sind aktivierte Ester, wie geeignet substituierte Niederalkyl-, z.B. Cyanmethylester, oder geeignet substituierte Phenyl-, z.B. Pentachlorphenyl- oder 4-Nitrophenylester. Die Veresterung kann, wenn notwendig, in Gegenwart von geeigneten Kondensationsmitteln, bei Verwendung von freien Carbonsäuren, z.B. in Gegenwart von Carbodiimidverbindungen, wie Dicyclohexylcarbodiimid, oder Carbonylverbindungen, wie Diimidazolylcarbonyl, und bei Verwendung von reaktionsfähigen Säurederivaten z.B. in Gegenwart von basischen Mitteln, wie Triniederalkylaminen, z.B. Triethylamin, oder heterocyclischen Basen, z.B. Pyridin, durchgeführt werden. Die Acylierungsreaktion kann in Abwesenheit oder in Gegenwart eines Lösungsmittels oder Lösungsmittelgemisches, unter Kühlen, bei Raumtemperatur oder unter Erwärmen, und, wenn notwendig, in einem geschlossenen Gefäss und/oder in einer Inertgas-, z.B. Stickstoffatmosphäre, durchgeführt werden. Geeignete Lösungsmittel sind z.B. gegebenenfalls substituierte, insbesondere gegebenenfalls chlorierte, aliphatische, cycloaliphatische oder aromatische Kohlenwasserstoffe, wie Benzol oder Toluol, wobei man geeignete Veresterungsreagentien, wie Essigsäureanhydrid, auch als Verdünnungsmittel verwenden kann.

Verfahren i) (Alkylierung oder Veretherung):

Geeignete Mittel zur Alkylierung einer Aminogruppe oder zur Veretherung einer freien Hydroxy- oder Mercaptogruppe in einer Verbindung der Formel I sind z.B. entsprechende Diazoverbindungen, wie gegebenenfalls substituierte Diazoniederalkane, z.B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan,

Cyclohexan, Benzol oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mitel sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Jodwasserstoffsäure, ferner Schwefelsäure, oder Halogenschwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogensubstituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, aliphatischen, cycloaliphatischen oder aromatischen Kohlenwasserstoffs, z.B. Methylenchlorid, eines Ethers, wie Dioxan oder Tetrahydrofuran, oder eines Gemisches verwendet. Dabei wendet man vorzugsweise geeignete Kondensationsmittel, wie Alkalimetallcarbonate oder hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin (vorzugsweise zusammen mit Halogensulfonsäure-niederalkyl-estern oder gegebenenfalls halogensubstituierten Methansulfonsäureniederalkylestern) an, wobei unter Kühlen,

bei Raumtemperatur oder unter Erwärmen, z.B. bei Temperaturen von etwa -20°C bis etwa 50°C und, wenn notwendig, in einem geschlossenen Gefäss und/oder in einer Inertgas-, z.B. Stickstoffatmosphäre, gearbeitet wird.

Durch Phasentransfer-Katalyse [siehe z.B. Dehmlow, Angewandte Chemie, 86, 187 (1974)] kann die oben beschriebene Veretherungsreaktion wesentlich beschleunigt werden. Als Phasentransfer-Katalysatoren können quartäre Phosphoniumsalze und insbesondere quartäre Ammoniumsalze, wie gegebenenfalls substituierte Tetraalkylammoniumhalogenide, z.B. Tetrabutylammoniumchlorid, -bromid oder -jodid, oder auch Benzyltriethylammoniumchlorid, in katalytischen oder bis zu äquimolaren Mengen verwendet werden. Als organische Phase kann irgendeines der mit Wasser nicht mischbaren Lösungsmittel dienen, beispielsweise einer der gegebenenfalls halogenierten, wie chlorierten, niederaliphatischen, cycloaliphatischen oder aromatischen Kohlenwasserstoffe, wie Tri- oder Tetrachlorethylen, Tetrachlorethan, Tetrachlorkohlenstoff, Chlorbenzol, Toluol oder Xylol. Die als Kondensationsmittel geeigneten Alkalimetallcarbonate oder -hydrogencarbonate, z.B. Kalium- oder Natriumcarbonat oder -hydrogencarbonat, Alkalimetallphosphate, z.B. Kaliumphosphat, und Alkalimetallhydroxide, z.B. Natriumhydroxid, können bei basenempflindlichen Verbindungen der Reaktionsmischung titrimetrisch, z.B. mittels eines Titrierautomaten, zugesetzt werden, damit der pH-Wert während der Veretherung zwischen etwa 7 und etwa 8,5 bleibt.

Weitere Mittel sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche Reagentien sind z.B. Trimethyloxonium- oder Triethyloxonium-hexafluorantimonat, -hexachlorantimonat,

-hexafluorphosphat oder -tetrafluorborat, Dimethoxycarbeniumhexafluorphosphat oder Dimethylbromoniumhexafluorantimonat. Man verwendet diese Veretherungsmittel vorzugsweise in einem inerten Lösungsmittel, wie einem Ether oder einem halogenierten Kohlenwasserstoff, z.B. Diethylether, Tetrahydrofuran oder Methylenchlorid,oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Weitere geeignete Veretherungsmittel sind schliesslich entsprechende 1-substituierte 3-Aryltriazenverbindungen, worin der Substituent den verethernden Rest, und Aryl vorzugsweise gegebenenfalls substituiertes Phenyl, z.B. Niederalkylphenyl, wie 4-Methylphenyl, bedeutet. Solche Triazenverbindungen sind 3-Aryl-1-niederalkyltriazene, z.B. 3-(4-Methylphenyl)-1-methyl-triazen, 3-(4-Methylphenyl)-1-ethyl-triazen oder 3-(4-Methylphenyl)-1-isopropyl-triazen. Diese Reagentien werden üblicherweise in Gegenwart von inerten Lösungsmitteln, wie gegebenenfalls halogenierten Kohlenwasserstoffen oder Ethern, z.B. Benzol, oder Lösungsmittelgemischen, und unter Kühlen, bei Raumtemperatur und vorzugsweise bei erhöhter Temperatur, z.B. bei etwa 20°C bis etwa 100°C, wenn notwendig in einem geschlossenen Gefäss und/oder in einer Inertgas-, z.B. Stickstoffatmosphäre, verwendet.

Verfahren j) (Lactonöffnung):

Die Ringöffnung eines Lactons der Formel IX erreicht man durch milde saure oder alkalische Behandlung, z.B. mit einem Alkalimetallcyanid, wie Natriumcyanid, oder einem Alkalimetallhydrogensulfit, z.B. Natriumhydrogensulfit.

Die Ausgangsstoffe der Formel IX können nach an sich bekannten Methoden erhalten werden, z.B. durch Einbau eines entsprechenden Bruchstückes, z.B. eines der Formel XIV

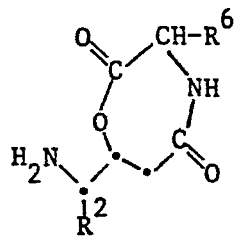

(XIV)

in die Peptidkette nach den bei Verfahren a) beschriebenen Methoden. Die Verbindung der Formel XIV wiederum kann z.B durch Zyklisierung einer entsprechenden Hydroxycarbonsäure, vorzugsweise in stark verdünnter Lösung, oder ausgehend von einem entsprechenden Keton der Formel XV,

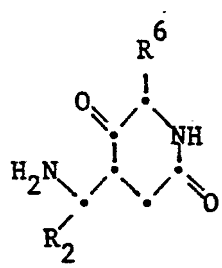

(XV)

worin die Aminogruppe und gegebenenfalls andere funktionelle Gruppen in geschützter Form vorliegen, durch Oxidation mit einer Peroxysäure nach Baeyer-Villiger, insbesondere mit Caroscher Säure, $H_2SO_5$, nach Ruzicka, erhalten werden.

Verfahren k)(Schutzgruppenabspaltung):

Schutzgruppen von gegebenenfalls vorhandenen funktionellen Gruppen sind z.B. die obengenannten Schutzgruppen.

Diese, z.B. geschützte Carboxyl-, Amino-, Hydroxy- und/oder Mercaptogruppen, werden in an sich bekannter Weise, mittels Solvolyse (auch enzymatisch), insbesondere Hydrolyse, Alkoholyse oder Acidolyse, oder mittels Reduktion, insbesondere Hydrogenolyse, oder chemische Reduktion, gegebenenfalls stufenweise oder gleichzeitig freigesetzt.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B.durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators,freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Jod-niederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumjodid, gespalten werden kann. Substituiertes 2-Silylethoxycarbonyl kann auch durch Behandeln mit einem, das Fluoridanion liefernden Salz der Fluorwasserstoffsäure, wie einem Alkalimetallfluorid, z.B. Natrium- oder Kaliumfluorid, in

Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden. Mit einer organischen Silylgruppe, wie Triniederalkylsilyl z.B. Trimethylsilyl, verestertes Carboxyl kann in üblicher Weise solvolytisch, z.B. durch Behandeln mit Wasser, einem Alkohol oder Säure, oder ausserdem mit einem Fluorid, wie oben beschrieben, freigesetzt werden. Verestertes Carboxyl kann auch enzymatisch gespalten werden, z.B. verestertes Arginin oder Lysin, wie Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfalls nach Umwandlung einer 2-Bromniederalkoxycarbonylaminogruppe in eine 2-Jod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silyläthoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure,

z.B. Chlorwasserstoffsäure, oder einer organischen Säure, z.B. Ameisen-, Essig- oder Trifluoressigsäure, gegebenenfalls in Gegenwart von Wasser, und eine mit einer organischen Silylgruppe geschützte Aminogruppe z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessend Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwasserstoffsäure, wie oben im Zusammenhang mit der Freisetzung einer entsprechend geschützten Carboxylgruppe angegeben, in die freie Aminogruppe überführt werden. Ebenso kann man direkt an ein Heteroatom, wie Stickstoff, gebundenes Silyl, wie Trimethylsilyl, mittels Fluorid abspalten.

Ein in Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Eine durch 2,2-Dichloracetyl geschützte

Hydroxy- bzw. Mercaptogruppe wird z.B. durch basische Hydrolyse, eine durch tert.-Niederalkyl oder durch einen 2-oxa- oder 2-thia-aliphatischen oder-cycloaliphatischen Kohlenwasserstoffrest verätherte Hydroxy- bzw. Mercaptogruppe durch Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden.

Wenn vorstehend nicht anders angegeben, werden die Verfahren a) bis k) in einem inerten Lösungsmittel oder Lösungsmittelgemisch bei einer Temperatur zwischen etwa -20°C und etwa +120°C, und, wenn nötig, unter Schutzgas durchgeführt.

Die zur Ausführung der vorstehend beschriebenen Verfahren a) bis k) benötigten Ausgangsstoffe sind bekannt oder können nach an sich bekannten Verfahren, z.B. nach oder analog den in dieser Anmeldung beschriebenen hergestellt werden.

Salze von Verbindungen der Formel (I) mit salzbildenden Gruppen können in an sich bekannter Weise hergestellt werden. So kann man Salze von Verbindungen der Formel (I) mit sauren Gruppen, z.B. durch Behandeln mit Metallverbindungen, wie Alkalimetallsalzen von geeigneten organischen Carbonsäuren, z.B. dem Natriumsalz der α-Ethyl-capronsäure, oder mit anorganischen Alkali- oder Erdalkalimetallsalzen, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel (I) mit mindestens einer basischen Gruppe, z.B. einer freien Aminogruppe, erhält man in üblicher Weise, z.B. durch Behandeln mit einer Säure oder einem

geeigneten Anionenaustauschreagens. Innere Salze von Verbindungen der Formel (I), welche z.B. eine freie Carboxylgruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.
Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Stereoisomerengemische, insbesondere Diastereomerengemische, können in an sich bekannter Weise, z.B. durch fraktionierte Kristallisation, Chromatographie etc., in die einzelnen Isomeren aufgetrennt werden.

Racemate können in an sich bekannter Weise, z.B. nach Ueberführung der optischen Antipoden in Diastereomere, beispielsweise durch Umsetzung mit optisch aktiven Säuren oder Basen, gespalten werden.

An einzelnen Asymmetriezentren kann die Konfiguration gezielt umgekehrt werden. Z.B. kann man die Konfiguration an einem eine Hydroxylgruppe tragenden C-Atom durch nucleophile Substitution zweiter Ordnung nach Ueberführung der Hydroxylgruppe in eine gute Abgangsgruppe epimerisieren.

Die Erfindung betrifft auch diejenigen Ausführungsformen des Verfahrens, bei denen man von einer auf irgendeiner Stufe als Zwischenprodukt erhältlichen Verbindung ausgeht und die fehlenden Schritte durchführt oder man das Verfahren auf irgendeiner Stufe abbricht oder man eine nach dem erfindungsgemässen Verfahren erhältliche Verbindung unter den Verfahrensbedingungen erzeugt und in situ weiterverarbeitet.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die pharmakologisch verwendbaren Verbindungen der vorliegenden Erfindung können z.B. zur Herstellung von pharmazeutischen Präparaten verwendet werden, welche eine wirksame Menge der Aktivsubstanz zusammen oder im Gemisch mit einer signifikanten Menge von anorganischen oder organischen, festen oder flüssigen pharmazeutisch verwendbaren Trägerstoffen enthalten.

Bei den erfindungsgemässen pharmazeutischen Präparaten handelt es sich um solche zur enteralen, wie nasalen, rektalen oder oralen, sowie vorzugsweise parenteralen, wie intramuskulären oder intravenösen, Verabreichung an Warmblüter, welche eine effektive Dosis des pharmakologischen Wirkstoffs allein oder zusammen mit einer signifikanten Menge eines pharmazeutisch anwendbaren Trägermaterials enthalten. Die Dosierung des Wirkstoffes hängt von der Warmblüter-Spezies, dem Körpergewicht, Alter und dem individuellen Zustand, der zu behandelnden Krankheit sowie von der Applikationsweise ab.

Die an Warmblüter, z.B. Menschen, von etwa 70 kg Körpergewicht zu verabreichenden Dosen liegen zwischen etwa 3 mg und etwa 3 g, vorzugsweise zwischen etwa 10 mg und etwa 1 g, z.B. bei ungefähr 300 mg, pro Warmblüter und Tag, verteilt auf vorzugsweise 1 bis 3 Einzeldosen, die z.B. gleich gross sein können. Ueblicherweise erhalten Kinder die halbe Erwachsendosis.

Die neuen pharmazeutischen Präparate enthalten von etwa 1% bis etwa 95%, vorzugsweise von etwa 20% bis etwa 90% des Wirkstoffes. Erfindungsgemässe pharmazeutische Präparate können z.B. in Dosiseinheitsform, wie Ampullen, Vials, Suppositorien, Dragées, Tabletten oder Kapseln, vorliegen.

Die pharmazeutischen Präparate der vorliegenden Erfindung werden in an sich bekannter Weise, z.B. mittels konventioneller Lösungs-, Lyophilisierungs-, Misch-, Granulier- oder Dragierverfahren, hergestellt.

Vorzugsweise verwendet man Lösungen des Wirkstoffes, daneben auch Suspensionen, und zwar insbesondere isotonische wässerige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz und/oder Emulgiermittel, Löslichkeitsvermittler, Salze zur Regulierung des osmotischen Druckes und/oder Puffer enthalten und werden in an sich bekannter Weise, z.B. mittels konventioneller Lösungs- oder Lyophilisierungsverfahren, hergestellt. Die genannten Lösungen oder Suspensionen können viskositätserhöhende Stoffe, wie Natrium-Carboxymethylcellulose, Carboxymethylcellulose, Dextran, Polyvinylpyrrolidon oder Gelatine, enthalten.

Suspensionen in Oel enthalten als ölige Komponente die für Injektionszwecke gebräuchlichen vegetabilen, synthetischen oder halbsynthetischen Oele. Als solche sind insbesondere flüssige Fettsäureester zu nennen, die als Säurekomponente eine langkettige Fettsäure mit 8-22, besonders 12-22 Kohlenstoffatomen, wie z.B. Laurinsäure, Tridecylsäure, Myristinsäure, Pentadecylsäure, Palmitinsäure, Margarinsäure, Stearinsäure, Arachinsäure, Behensäure oder entsprechende ungesättigte Säuren, wie z.B. Oelsäure, Elaidinsäure, Erucasäure, Brassidinsäure oder Linolsäure, enthalten. Die Alkoholkomponente hat maximal 6 Kohlenstoffatome und ist ein ein- oder mehrwertiger, z.B. ein-, zwei- oder dreiwertiger Alkohol, z.B. Methanol, Aethanol, Propanol, Butanol oder Pentanol oder deren Isomere, vor allem aber

Glycol oder Glyzerin. Als Fettsäureester sind daher beispielsweise zu nennen: Aethyloleat, Isopropylmyristat, Isopropylpalmitat, "Labrafil M 2735" (Polyoxyäthylenglyzerintrioleat der Firma Gattefossé, Paris), "Miglyol 812" (Triglyzerid gesättigter Fettsäuren der Kettenlänge $C_8$ bis $C_{12}$ der Firma Chemische Werke Witten/Ruhr, Deutschland), besonders aber vegetabile Oele wie Baumwollsaatöl, Mandelöl, Olivenöl, Ricinusöl, Sesamöl, Sojabohnenöl, vor allem Erdnussöl.

Die Herstellung der Injektionspräparate erfolgt in üblicher Weise unter antimikrobiellen Bedingungen, ebenso das Abfüllen in Ampullen oder Vials sowie das Verschliessen der Behälter.

Pharmazeutische Präparate zur oralen Anwendung können erhalten werden, indem man den Wirkstoff mit festen Trägerstoffen kombiniert, ein erhaltenes Gemisch gegebenenfalls granuliert und das Gemisch bzw. Granulat, wenn erwünscht oder notwendig nach Zugabe von geeigneten Hilfsstoffen, zu Tabletten oder Dragée-Kernen verarbeitet. Dabei kann man sie auch in Kunststoffträger einbauen, die die Wirkstoffe dosiert abgeben oder diffundieren lassen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister unter Verwendung z.B. von Mais-, Weizen-, Reis- oder Kartoffelstärke, Gelatine, Traganth, Methylcellulose, Hydroxypropyl-methylcellulose, Natriumcarboxymethylcellulose und/oder Polyvinylpyrrolidon, und/oder, wenn erwünscht, Sprengmittel, wie die obengenannten Stärken, ferner Carboxymethylstärke, quervernetztes Poylvinylpyyrolidon, Agar, Alginsäure oder ein Salz davon, wie Natriumalginat. Hilfsmittel sind in erster

Linie Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls magensaftresistenten Ueberzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemischen oder, zur Herstellung von magensaftresistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Die folgenden Beispiele dienen zur Illustration der Erfindung. Temperaturen werden in Celsiusgraden angegeben.

Die $R_f$-Werte werden wenn nicht anders angegeben, auf Kieselgeldünnschichtplatten in folgenden Lösungsmittelsystemen ermittelt:

A: n-BuOH-AcOH-$H_2O$ (67:10:23)
B: Ethylacetat-Pyridin-AcOH-$H_2O$ (62:21:6:11)
C: n-BuOH-Pyridin-AcOH-$H_2O$ (38:24:8:30)
D: Pyridin-n-BuOH-n-Amylalkohol-Methylethylketon-AcOH-Ameisensäure-$H_2O$ (25:20:15:10:3:3:25)
E: $CHCl_3$-MeOH-$H_2O$-AcOH (70:40:10:0,5)
F: $CHCl_3$-MeOH-$H_2O$-AcOH (90:10:1:0,5)
G: $CHCl_3$-MeOH-$H_2O$-AcOH (75:27:5:0,5)
H: Ethylacetat-AcOH-$H_2O$-MeOH (67:10:23:12)
I: $CHCl_3$-MeOH-AcOH-$H_2O$ (80:20:3:3)
K: n-BuOH-Pyridin-Ameisensäure-$H_2O$ (42:24:4:20)
L: $CHCl_3$-MeOH-AcOH-$H_2O$ (70:30:5:5)
M: n-Butanol-Pyridin-AcOH-$H_2O$ (42:24:4:30)
N: n-Butanol-Pyridin-Ameisensäure-$H_2O$ (44:24:2:20)

O: $CHCl_3$-MeOH-$H_2O$-AcOH (55:47:13:0,5)

Z.B. bedeutet im folgenden "$R_f$ (A)", dass der $R_f$-Wert im System A ermittelt wurde. Das Mengenverhältnis der Lösungsmittel zueinander ist in Volumenproportionen angegeben.

Abkürzungen

Ac = Acetyl
Boc = tert. Butyloxycarbonyl
Bu = Butyl
DC = Dünnschichtchromatographie, wenn nicht anders angegeben auf Kieselgel
DCCI = Dicylohexyl-carbodiimid
DCH = Dicyclohexyl-harnstoff
DMF = Dimethylformamid
Et = Ethyl
HOBt = 1-Hydroxy-benzotriazol
HONB = N-Hydroxy-5-norbornen-2,3-dicarbonsäureimid
H-Sta(Me)-OH = O-Methyl-statin = (4S)-Amino-(3S)-methoxy-6-methyl-heptancarbonsäure
HV = Hochvakuum
H-Sta(Ac)-OH = O-Acetyl-statin = (3S)-Acetoxy-(4S)-amino-6-methyl-heptancarbonsäure
Lac = -O-CH($CH_3$)-CO- (bivalenter Rest der Milchsäure H-Lac-OH), ohne anderslautende Angabe mit L-Konfiguration
Me = Methyl
Min. = Minute(n)
Smp. = Schmelzpunkt
Su = Succinimidyl
TFA = Trifluoressigsäure
Vol. = Volumen(anteile)
Z = Benzyloxycarbonyl
Ø = Durchmesser

Beispiel 1: 540 mg H-Pro-His-Pro-Phe-His-Sta-Val-Tyr-Lys-OMe x 4 TFA werden in 9 ml $H_2O$ gelöst (pH = 1,85) und der pH-Wert mit 0,3 N $NH_3$ auf 5,0 gestellt. Man gibt 55 µl 0,5%ige wässerige Trypsinlösung zu und rührt bei Raumtemperatur, wobei der pH-Wert durch Zugabe von 0,3 N $NH_3$ mittels pH-Stat bei 5,0 gehalten wird. Nach beendigter Basenaufnahme (ca. 1 Stunde) gibt man 0,5 ml Eisessig zu, erhitzt während 2 Minuten auf 95° und lyophilisiert. Zur Umwandlung ins Acetat wird in 0,05 N AcOH gelöst und langsam durch eine Säule (Ø = 1 cm, Länge = 12 cm) von schwach basischem Ionenaustauscher (z.B. Merck Nr. II) in der Acetatform filtriert, das Eluat eingeengt und wieder lyophilisiert. Man erhält H-Pro-His-Pro-Phe-His-Sta-Val-Tyr-Lys-OH x AcOH als amorphes, gut wasserlösliches Pulver; $R_f$ (C) = 0,28; $R_f$ (D) = 0,17; $R_f$ (K) = 0,20.

Der Ausgangsstoff H-Pro-His-Pro-Phe-His-Sta-Val-Tyr-Lys-OMe x 4 TFA ist folgendermassen erhältlich:

Stufe 1.1: 14,5 g Z-Lys(Boc)-OMe [A. Costopanagiotis et al., J. Org. Chem. 33, 1261 (1968)] werden in 145 ml 95%igem MeOH gelöst und nach Zugabe von 1,45 g Pd-Kohle (10% Pd) unter $CO_2$-Absorption bis zur Sättigung hydriert. Der Katalysator wird abfiltriert, das Filtrat zum Oel eingeengt, 30 ml DMF zugegeben und wieder eingeengt. Diese Lösung, die H-Lys(Boc)-OMe enthält, wird direkt weiterverwendet.

Stufe 1.2: 13,8 g Z-Val-Tyr-OH [R. Schwyzer et al. Helv. Chim. acta. 41, 1273 (1958)] und 5,1 g HOBt x $H_2O$ werden in 100 ml DMF gelöst und dazu die obige Lösung von H-Lys(Boc)-OMe in wenig DMF gegeben. Man kühlt auf 0°, gibt 7,6 g DCCI zu und rührt während 6 Stunden bei 0° und 10 Stunden bei Raumtemperatur. Der ausgeschiedene DCH wird abfiltriert und das Filtrat zur Trockne eingeengt. Man reinigt durch Zerreiben mit Diisopropylether und durch Umfällen aus Trifluorethanol-Essigester-Petrolether, worauf man Z-Val-Tyr-Lys(Boc)-OMe erhält; $R_f$ (F) = 0,39.

Stufe 1.3: 8 g Z-Val-Tyr-Lys(Boc)-OMe werden in 160 ml 95%igem MeOH suspendiert und nach Zugabe von 800 mg Pd-Kohle unter $CO_2$-Absorption hydriert, wobei die Substanz in Lösung geht. Nach beendigter $H_2$-Absorption wird filtriert, das Filtrat zur Trockne eingeengt und aus MeOH-$H_2O$ als Nadeln umkristallisiert, worauf man H-Val-Tyr-Lys(Boc)-OMe erhält; Smp. = 165-166°; Rf (G) = 0,54; $R_f$ (I) = 0,29.

Stufe 1.4: 1 g Z-Sta-OH (Herstellung siehe Stufe 1.12), 1,49 g H-Val-Tyr-Lys(Boc)-OMe und 650 mg HOBt x $H_2O$ werden in 15 ml DMF gelöst, 704 mg DCCI zugegeben und während 20 Stunden bei Raumtemperatur gerührt. Der auskristallisierte DCH wird bei 0° abfiltriert, das Filtrat bis zur öligen Beschaffenheit eingeengt, nach Zugabe von 30 ml Diisopropylether das Kondensationsprodukt als Pulver ausgefällt und nach 10minütigem Rühren bei 0° abfiltriert. Es wird durch Craig-Verteilung im System Hexan-Isopropanol-0,1 N Essigsäure (10:10:3) über 900 Stufen gereinigt, K-Wert (Verteilungskoeffizient) = 0,29, worauf man Z-Sta-Val-Tyr-Lys(Boc)-OMe erhält; $R_f$ (F) = 0,31.
Stufe 1.5: 850mg Z-Sta-Val-Tyr-Lys(Boc)-OMe werden in 17 ml 95%igem MeOH gelöst und mit 85 mg Pd-Kohle unter $CO_2$-Absorption bis zur Sättigung (ca. 1 Std.) hydriert. Der Katalysator wird abfiltriert, das Filtrat zur Trockne eingeengt und im HV bei 40° getrocknet, worauf man H-Sta-Val-Tyr-Lys(Boc)-OMe als amorphes Pulver erhält; $R_f$ (E) = 0,58; $R_f$ (H) = 0,50. Das erhaltene Produkt wird in Stufe 1.10 weiterumgesetzt.

Stufe 1.6: 7 g Z-Pro-Phe-His-OMe [H. DeWald et al., J. Med. Pharm. Chem. 7, 50 (1964)] werden in 200 ml 95%igem MeOH suspendiert, 0,7 g Pd-Kohle zugegeben und unter $CO_2$-Absorption bis zur Sättigung hydriert. Der Katalysator wird abfiltriert, das Filtrat zur Trockne eingeengt, der amorphe, schaumige Rückstand pulverisiert und im HV bei 40° getrocknet, worauf man H-Pro-Phe-His-OMe erhält; $R_f$ (E) = 0,21.

Stufe 1.7: 1,25 g Boc-Pro-OSu [G.W. Anderson et al., J. Amer. Chem. Soc. 86, 1839 (1964)] und 0,68 g H-His-OH werden in 4 ml $H_2O$ und 2 ml

Dioxan suspendiert. Unter starkem Rühren wird 2 N NaOH mit Hilfe eines pH-Stats bei pH 9,0 zugegeben. Nach 45 Min. kommt die NaOH-Aufnahme zum Stillstand. Man rührt die jetzt klare Lösung noch während 10 Minuten, stellt dann mit 4 N HCl einen pH-Wert von 5,0 ein und engt auf ca. 5 ml ein. Nach Zugabe von 20 ml $H_2O$ wird nochmals auf ca. 5 ml konzentriert und dann durch Chromatographie an einer Säule von Absorberharz, Diaion HP-20 (Ø = 1,6 cm; Länge = 20 cm) gereinigt. Die Hauptmenge dieses Produktes wird mit 20% Isopropanol in $H_2O$ eluiert. Die nach Dünnschichtkontrolle reinen Fraktionen werden vereinigt, nahezu zur Trockne eingeengt und nach Zugabe von $H_2O$ lyophilisiert, worauf man Boc-Pro-His-OH als amorphes Pulver erhält; $R_f$(E) = 0,45; $R_f$ (A) = 0,21.

Stufe 1.8: 6,59 g Boc-Pro-His-OH, 7 g H-Pro-Phe-His-OMe (aus Stufe 1.6) und 5,65 g HONB werden in 35 ml DMF gelöst. Nach Abkühlen auf 0° gibt man 4,82 g DCCI zu und rührt während 6 Stunden im Eisbad und über Nacht bei Raumtemperatur. Nach weiteren 30 Minuten unter Rühren bei 0° wird DCH abfiltriert, das Filtrat zur Trockne eingeengt und durch Zugabe von 200 ml Diisopropylether als klebrige Masse ausgefällt und getrocknet. Man löst dann in 188 ml MeOH, 6 ml Eisessig und 6 ml $H_2O$, erwärmt während 1 Stunde auf 60°, engt auf 40 ml ein und fällt mit 400 ml Diisopropylether wieder als Oel aus. Nach 1 Stunde bei 0° wird die überstehende Lösung abdekantiert und der Rückstand getrocknet, worauf man Boc-Pro-His-Pro-Phe-His-OMe erhält; $R_f$ (A) = 0,17; $R_f$ (E) = 0,51.

Stufe 1.9: 17,2 g rohes Boc-Pro-His-Pro-Phe-His-OMe werden in 54 ml MeOH gelöst, mit 650 ml 0,1 N NaOH versetzt, 15 Minuten bei 25° stehen gelassen, durch Zugabe von 650 ml 0,1 N HCl neutralisiert und zur Trockne eingeengt. Der Rückstand wird in 120 ml $H_2O$ gelöst, der pH-Wert genau auf 6,8 gestellt und diese Lösung im System n-Butanol-$H_2O$ einer Craig-Verteilung über 500 Stufen unterworfen; (K = 2,8). Die nach Dünnschichtanalytik reinen Fraktionen werden vereinigt, zur

Trockne eingeengt, in $H_2O$ gelöst und lyophilisiert, worauf man Boc-Pro-His-Pro-Phe-His-OH in amorpher Form erhält; $R_f$ (A) = 0,15; $R_f$ (C) = 0,42; $R_f$ (E) = 0,25.

Stufe 1.10: 840 mg Boc-Pro-His-Pro-Phe-His-OH, 600 mg H-Sta-Val-Tyr-Lys(Boc)-OMe (aus Stufe 1.5) und 250 mg HONB werden in 6 ml DMF gelöst, auf 0° gekühlt und mit 290 mg DCCI versetzt. Man rührt während 6 Stunden bei 0° und 24 Stunden bei 25°, filtriert den DCH ab, und dampft das Filtrat zur Trockne ein. Der Rückstand wird in 18,8 ml MeOH, 0,6 ml Eisessig und 0,6 ml $H_2O$ gelöst, 1 Stunde auf 60° erhitzt, auf ca. 3 ml eingeengt und das Peptid mit 20 ml Diisopropylether als schmieriges Material ausgefällt. Es wird in einer Craig-Verteilung im System MeOH-0,1 N AcOH-Ethylenchlorid-Chloroform (10:3:8:4) über 750 Stufen gereinigt; K = 0,75. Die chromatographisch reine Mittelfraktion wird durch starkes Einengen und Lyophilisieren isoliert, worauf man Boc-Pro-His-Pro-Phe-His-Sta-Val-Tyr-Lys(Boc)-OMe als amorphes Pulver erhält; $R_f$ (B) = 0,38; $R_f$ (I) = 0,46, $R_f$ (C) = 0,64.

Stufe 1.11: 470 mg Boc-Pro-His-Pro-Phe-His-Sta-Val-Tyr-Lys(Boc)-OMe werden bei 25° in 2,3 ml 95%iger TFA gelöst und während 30 Minuten stehen gelassen. Das gebildete H-Pro-His-Pro-Phe-His-Sta-Val-Tyr-Lys-OMe wird bei 0° durch Zugabe von 20 ml Diisopropylether in pulveriger Form als Trifluoracetat ausgefällt, abfiltriert und getrocknet; $R_f$ (C) = 0,36; $R_f$ (D) = 0,24.

Stufe 1.12:
Das in Stufe 1.4 eingesetzte Z-Sta-OH erhält man folgendermassen: 6,0 g (3S,4S)-N-Z-Statinethylester werden in 220 ml Dioxan und 220 ml Wasser aufgenommen. Die trübe Lösung wird bei 0° mit 1N NaOH auf pH 11.90 gestellt. Das Gemisch wird 30 Minuten bei 0° nachgerührt, wobei der pH-Wert auf 12.14 steigt. Die klare Reaktionslösung wird bei 0° mit 1N HCl auf pH 6.5 gestellt, das Dioxan im Vakuum abgedampft

und der Rückstand zwischen Ether und gesättigter Natriumhydrogencarbonatlösung ausgeschüttelt. Die Wasserphase wird mit 10%iger Zitronensäure auf pH 2.5 gestellt und mit Ether extrahiert. Die organische Phase wird mit Sole gewaschen und getrocknet. Durch Kristallisieren der Rückstandes aus Ether/Petrolether (1:1) erhält man (3S, 4S)-N-Z-Statin [Z-Sta-OH, (4S)-Benzyloxycarbonylamino-(3S)-hydroxy-6-methylheptancarbonsäure, weisse Kristalle, Smp. 116-117°, $[\alpha]_D^{20}$ = -33° ($CH_3OH$, c = 1.23)]. Auf analoge Weise erhält man, ausgehend von (3R, 4S)-N-Z-Statinethylester, 3R,4S-N-Z-Statin [weisse Kristalle, Smp. 135-136°, $[\alpha]_D^{20}$ = -21° ($CH_3OH$, c = 1.13)].

Stufe 1.13: Das in Stufe 1.12 verwendete Ausgangsmaterial erhält man folgendermassen:

Zu einer Lösung von 33 ml (0.233 Mol) Diisopropylamin in 75 ml Tetrahydrofuran unter Argon werden bei -20° 136.9 ml (0.233 Mol) einer 1.7molaren Lösung von Butyllithium in Hexan zugetropft. Nach 15 Minuten wird auf -76° abgekühlt, tropfenweise mit 22.78 ml (0.233 Mol) Ethylacetat versetzt und 15 Minuten nachgerührt. Während 20 Minuten wird nun eine auf -76° vorgekühlte Lösung von 34.1 g (0.137 Mol) N-Z-Leucinal [(2S)-Benzyloxycarbonylamino-4-methylpentanal; Ito et al., Chem. Pharm. Bull. 23, 3081 (1975)] in 100 ml Tetrahydrofuran zugetropft. Das Reaktionsgemisch wird während 15 Minuten bei -76° nachgerührt und anschliessend im Laufe von 30 Minuten mit 118 ml 2N HCl versetzt. Diese Suspension wird bei 0° mit 2N HCl auf pH 2.5 gestellt, nach Erwärmen auf Raumtemperatur mit Ether extrahiert, die organische Phase mit Sole (gesättigter Natriumchloridlösung) gewaschen und getrocknet. Das Rohprodukt wird mittels Mitteldruckchromatographie [LiChroprep Si 60, 25-40 µm, Ethylacetat-Hexan (1:5)] aufgetrennt. Man erhält bei der Elution zuerst (3S, 4S)-N-Z-Statinethylester [wachsartige Kristalle, Smp. 51-54°, $[\alpha]_D^{20}$ = -26° ($CH_3OH$, c = 0.69)] und dann den polareren (3R, 4S)-N-Z-Statinethylester [weisse Kristalle, Smp. 103-104°, $[\alpha]_D^{20}$ = -17° ($CH_3OH$, c = 1.00)].

Beispiel 2: 370 mg H-Pro-His-Pro-Phe-His-Sta-Ile-His-Lys-OMe x 5 TFA werden in 8 ml $H_2O$ gelöst. Der pH-Wert wird mit 0,3 N $NH_3$ auf 5,0 erhöht, worauf man 40 µl einer 1%igen wässrigen Lösung von Trypsin zugibt und mittels pH-Stat unter Zugabe von 0,3 N $NH_3$ den pH-Wert konstant hält. Die Basenaufnahme ist nach ca. 40 Min. beendet. Man gibt 1 ml Eisessig zu, erhitzt während 2 Min. im siedenden Wasserbad, engt auf ca. 3 ml ein und lyophilisiert. Der Rückstand wird in 3 ml 0,05 N AcOH gelöst, durch langsames Filtrieren durch eine Säule (Ø = 1 cm, Länge = 10 cm) von schwach basischem Ionenaustauscher (Merck Nr. II) in der Acetatform ins Acetat übergeführt und das Eluat wiederum lyophilisiert, worauf man H-Pro-His-Pro-Phe-His-Sta-Ile-His-Lys-OH x AcOH als amorphes, weisses Pulver erhält; $R_f$ (D) = 0,12; $R_f$ (K) = 0,14.

Der Ausgangsstoff H-Pro-His-Pro-Phe-His-Sta-Ile-His-Lys-OMe x 5 TFA ist folgendermassen erhältlich:

Stufe 2.1: 1,6 g Z-His-Lys(Boc)-OMe [S.Guttman et al., Helv. Chim. Acta 52, 1789 (1969)] werden in 33 ml MeOH gelöst, dazu 3 ml 1 N HCl und 160 mg Pd-Kohle (10% Pd) gegeben und unter $CO_2$-Absorption bis zur Sättigung hydriert. Nach dem Abfiltrieren des Katalysators wird eingeengt, der ölige Rückstand in 20 ml DMF gelöst und erneut bis auf ca. 10 ml eingeengt, worauf man H-His-Lys(Boc)-OMe in Form einer Lösung erhält, die sofort in Stufe 2.2 weiterverarbeitet wird.

Stufe 2.2: 1,32 g Z-Ile-OSu [G.W. Anderson et al., J.Amer.Chem.Soc. 86, 1839 (1964)] werden in der obigen Lösung von H-His-Lys(Boc)-OMe in ca. 10 ml DMF gelöst und mit 0,41 ml $NEt_3$ vermischt. Man lässt während 8 Stunden bei Raumtemperatur stehen, gibt 15 ml Diisopropylether zu, homogenisiert und filtriert ab. Der Nutschenrückstand wird in 15 ml Methanol gelöst und bei 0° zu 23 ml wässeriger $NaHCO_3$-Lösung gegeben. Der ausgefallene Niederschlag wird abfiltriert, mit $H_2O$ gewaschen, getrocknet und aus MeOH-Essigester-Petrolether umgefällt, worauf man Z-Ile-His-Lys(Boc)-OMe als amorphes Pulver erhält; $R_f$ (B) = 0,65; $R_f$ (I) = 0,48.

Stufe 2.3: 8 g Z-Ile-His-Lys(Boc)-OMe werden in einer Mischung von 80 ml MeOH, 8 ml $H_2O$ und 80 ml Trifluorethanol suspendiert und mit 800 mg Pd-Kohle (10% Pd) unter $CO_2$-Absorption bis zur Sättigung hydriert, wobei das anfänglich nicht gelöste Material in Lösung geht. Der Katalysator wird abfiltriert, das Filtrat zur Trockne eingeengt und der harzige Rückstand aus Acetonitril kristallisiert, worauf man H-Ile-His-Lys(Boc)-OMe in Form von Nadeln mit Smp. 129 - 130° erhält; $R_f$ (I) = 0,12; $R_f$ (B) = 0,14.

Stufe 2.4: 900 mg Z-Sta-OH (Herstellung siehe Stufe 1.12), 1,24 g H-Ile-His-Lys(Boc)-OMe und 560 mg HOBt x $H_2O$ werden in 13 ml DMF gelöst. Nach Zugabe von 600 mg DCCI wird während 20 Stunden bei Raumtemperatur stehen gelassen, dann das auskristallisierte DCH abfiltriert und das Filtrat zur Trockne eingeengt. Der schmierige Rückstand wird mit 30 ml Diisopropylether bei 0° zerrieben, das Lösungsmittel dekantiert und der Rückstand getrocknet. Man reinigt durch Craig-Verteilung im System Methanol-Puffer-Ethylenchlorid-Chloroform (10:3:8:4; Puffer = 14,3 ml Eisessig und 9,6 g Ammoniumacetat in 1000 ml $H_2O$) über 700 Stufen. Die dünnschichtchromatografisch reinen Fraktionen (K = 0,5) werden vereinigt, stark eingeengt und lyophilisiert. Zur Entfernung von Essigsäure wird aus 5%iger methanolischer $NaHCO_3$-Lösung umgefällt, worauf man Z-Sta-Ile-His-Lys(Boc)-OMe als amorphes Pulver erhält: $R_f$ (B) = 0,8; $R_f$ (I) = 0,56.

Stufe 2.5: 1 g Z-Sta-Ile-His-Lys(Boc)-OMe wird in 10 ml 95%igem MeOH gelöst und mit 100 mg Pd-Kohle (10% Pd) unter $CO_2$-Absorption bis zur Sättigung hydriert. Nach Abfiltrieren des Katalysators und Einengen zur Trockne erhält man H-Sta-Ile-His-Lys(Boc)-OMe als amorphes Pulver, das direkt weiterverarbeitet wird; $R_f$ (B) = 0,32; $R_f$ (I) = 0,17.

Stufe 2.6: 480 mg Boc-Pro-His-Pro-Phe-His-OH (Herstellung siehe Beispiel 1, Stufe 1.9), 340 mg H-Sta-Ile-His-Lys(Boc)-OMe und 150 mg HONB werden unter Erwärmen in 3,5 ml DMF gelöst. Nach Abkühlen auf 0° werden 170 mg DCCI zugegeben, worauf man während 5 Stunden bei

0° rührt und 20 Stunden bei Raumtemperatur stehen lässt. Das Kondensationsprodukt wird durch Zugabe von 35 ml Diisopropylether ausgefällt und abfiltriert. Es wird durch Craig-Verteilung über 1000 Stufen im gleichen Lösungsmittelsystem wie in Stufe 2.4 beschrieben, gereinigt (K = 0,6), worauf man Boc-Pro-His-Pro-Phe-His-Sta-Ile-His-Lys(Boc)-OMe erhält; $R_f$ (A) = 0,22; $R_f$ (B) = 0,21; $R_f$ (E) = 0,54.

Stufe 2.7: 330 mg Boc-Pro-His-Pro-Phe-His-Sta-Ile-His-Lys(Boc)-OMe werden in 1,7 ml 95%iger Trifluoressigsäure gelöst und während 30 Minuten bei 25° stehen gelassen. Man gibt sodann 14 ml Diisopropylether zu, rührt während 30 Minuten bei 0°, filtriert ab und trocknet im HV über KOH, worauf man H-Pro-His-Pro-Phe-His-Sta-Ile-His-Lys-OMe x 5 TFA erhält; $R_f$ (D) = 0,17; $R_f$ (K) = 0,20.

<u>Beispiel 3:</u> Analog den in dieser Anmeldung beschriebenen Verfahren erhält man:

H-Pro-His-Pro-Phe-His-Sta-$NH_2$,
H-Pro-His-Pro-Phe-His-Sta-NH-$CH_2$-CH-$CH_2$-$CH_3$,
$CH_3$

H-Pro-His-Pro-Phe-His-Sta-OH,
H-Pro-His-Pro-Phe-His-(3R, 4S)-Sta-Val-Tyr-Lys-OH,
H-Pro-His-Pro-Phe-His-Sta(Me)-Ile-His-Lys-OH,
H-Pro-His-Pro-Phe-His-Sta(Ac)-Ile-His-Lys-OH,

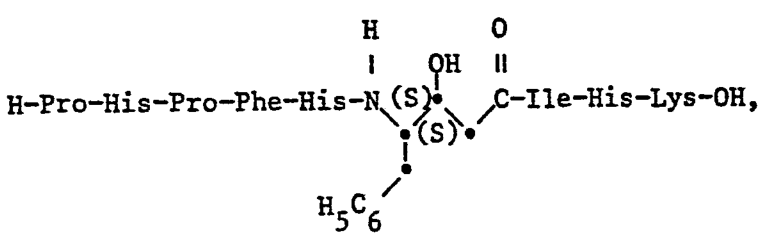

H-Pro-Phe-His-Sta-Ile-His-Lys-OH,
H-Pro-His-Pro-Phe-His-Sta-$OC_2H_5$,
H-Pro-His-Pro-Phe-His-(3R, 4S)-Sta-Ile-His-Lys-OH,
H-Pro-His-Pro-Phe-His-Sta(Me)-Val-Tyr-Lys-OH,
H-Pro-His-Pro-Phe-His-Sta(Ac)-Val-Tyr-Lys-OH,

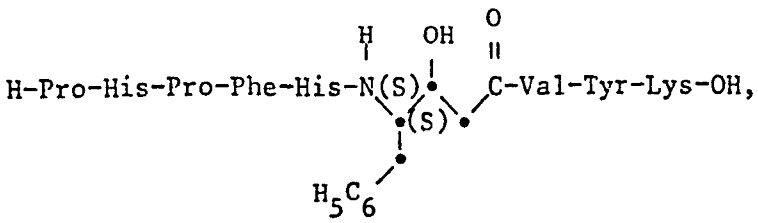

H-Pro-His-Pro-Phe-His-Sta-Val-Tyr-$NH_2$,
H-Pro-His-Pro-Phe-His-Sta-Ile-His-$NH_2$,
H-Pro-His-Pro-Phe-His-Sta-Ile-D-His-$NH_2$,

H-Pro-His-Pro-Phe-His-Sta-Val-NH-$CH_2$-$CH_2$- 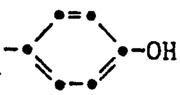 ,

H-Pro-His-Pro-Phe-His-Sta-Ile-NH-$CH_2$-$CH_2$- 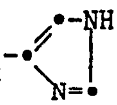 ,

H-Pro-His-Pro-Phe-His-Sta-Phe-Val-Tyr-Lys-OH,
H-His-Pro-Phe-His-Sta-Ile-His-Lys-OH,
H—D—His-Pro-Phe-His-Sta-Ile-His-Lys-OH,
H-Arg-Arg-Pro-Phe-His-Sta-Ile-His-Lys-OH und
H-Arg—D—Arg-Pro-Phe-His-Sta-Ile-His-Lys-OH.

Beispiel 4: 595 mg Z-Arg-Arg-Pro-Phe-His-OH (als Trihydrochlorid, enthaltend 24 Gew.-% NaCl), 250 mg H-Sta-Ile-His-Lys(Boc)-OMe (Stufe 2.5) und 74 mg HOBt x $H_2O$ werden in 2,5 ml DMF gelöst bzw. suspendiert, auf $0^o$ gekühlt und mit 110 mg DCCI zersetzt. Man rührt während 10 Stunden bei $0^o$, lässt noch 1 Tag bei Raumtemperatur stehen und filtriert NaCl und DCH ab. Das Filtrat wird zum Oel eingeengt und daraus durch Zugabe von 50 ml Diisopropylether das Kondensationsprodukt ausgefällt und getrocknet. Es wird in 8 ml Methanol-Eisessig-$H_2O$ (94:3:3) während 1 Stunde auf $60^o$ erwärmt, durch Zugabe von 80 ml Diisopropylether bei $0^o$ gefällt, abfiltriert und getrocknet. Man löst den Rückstand in 20 ml 0,1 N Essigsäure, filtriert von wenig unlöslichem Material ab und lässt das Filtrat langsam durch eine Säule (Ø = 1,2 cm; Länge = 15 cm) von schwach basischem Ionenaustauscher (z.B. Merck Nr. II) in der Acetatform laufen. Das Eluat wird auf 10-15 ml eingeengt und lyophilisiert. Zur Reinigung wird es einer Craig-Verteilung im System n-Butanol-

Eisessig-$H_2O$ (4:1:5) über 400 Stufen unterworfen; K-Wert ca. 0,5. Die chromatographisch reinen Fraktionen werden vereinigt, zum Oel eingeengt, in Wasser gelöst und lyophilisiert, wobei man Z-Arg-Arg -Pro-Phe-His-Sta-Ile-His-Lys(Boc)-OMe als amorphes Pulver (Acetatform) erhält; $R_f$ (M) = 0,55; $R_f$ (N) = 0,35; $R_f$ (O) = 0,5, $R_f$ (L) = 0,2.

Der Ausgangsstoff Z-Arg-Arg-Pro-Phe-His-OH ist folgendermassen erhältlich:

Stufe 4.1: 486 mg Z-Arg-OH werden in 5 ml DMF suspendiert und durch Zugabe von 315 µl einer 5 N Lösung von HCl in Dioxan in Lösung gebracht. Man fügt 500 mg H-Pro-Phe-His-OMe (Stufe 1.6) und 185 mg HOBt x $H_2O$ in fester Form zu, wobei eine klare Lösung entsteht. Nach Abkühlen auf $0^o$ werden 325 mg DCCI zugegeben und das Ganze während 10 Stunden im Eisbad und 1 Tag bei Raumtemperatur stehengelassen. Der ausgeschiedene DCH wird abfiltriert, das Filtrat auf 2-3 ml eingeengt und daraus das Rohprodukt durch Zugabe von Diisopropylether ausgefällt. Es wird in 14 ml Methanol-Eisessig-$H_2O$ (94:3:3) gelöst, 1 Stunde auf $60^o$ erwärmt, wiederum auf 2-3 ml konzentriert und mit Diisopropylether gefällt. Man löst in 10 ml $H_2O$, stellt mit 2 N HCl auf pH 3,0 und reinigt mittels Craig-Verteilung im System n-Butanol-$H_2O$. Beim Einengen der reinen Fraktion und Lyophilisieren erhält man Z-Arg-Pro-Phe-His-OMe in der Form des Dihydrochlorides; $R_f$ (E) = 0,45; $R_f$ (C) = 0,55.

Stufe 4.2: 2,5 g Z-Arg-Pro-Phe-His-OMe werden in 25 ml 95%igem Methanol gelöst und nach Zugabe von 250 mg Pd-Kohle unter $CO_2$-Absorption bis zur Sättigung hydriert. Der Katalysator wird abfiltriert, und das Filtrat zur Trockne eingeengt, worauf man H-Arg-Pro-Phe-His-OMe in Form eines amorphen Pulvers erhält; $R_f$ (E) = 0,05; $R_f$ (C) = 0,3.

Stufe 4.3: 1,27 g Z-Arg-OH werden in 20 ml DMF und 240 µl einer 5 N Lösung von HCl in Dioxan unter leichtem Erwärmen gelöst. Nach Zugabe von 1,9 g H-Arg-Pro-Phe-His-OMe (als Dihydrochlorid) und 0,45 g HOBt x $H_2O$ wird auf 0° gekühlt und dann 0,85 g DCCI in fester Form zugefügt. Nach dessen Auflösung lässt man während 8 Stunden im Eisbad und 1 Tag bei Raumtemperatur stehen und filtriert den ausgeschiedenen DCH ab. Das Filtrat wird auf ca. 5 ml eingeengt und daraus das Peptid durch Zugabe von 50 ml Diisopropylether ausgefällt und getrocknet. Es wird in 45 ml Methanol-Eisessig-$H_2O$ (94:3:3) gelöst, 1 Stunde auf 60° erwärmt, wiederum auf ca. 5 ml eingeengt und mit Diisopropylether ausgefällt und getrocknet. Das Rohprodukt wird durch Chromatographie an 120g Kieselgel (60, Merck, 230-400 mesh) unter Elution mit Chloroform-Methanol-$H_2O$-Eisessig (140:80:20:1) gereinigt. Die reinen Fraktionen werden vereinigt, bis fast zur Trockne eingeengt, in $H_2O$ gelöst und lyophilisiert, worauf man Z-Arg-Arg-Pro-Phe-His-OMe in der Form des Dihydrochlorid-monoacetats erhält; $R_f$ (C) = 0,4; $R_f$ (E) = 0,3.

Stufe 4.4: 1,3 g Z-Arg-Arg-Pro-Phe-His-OMe werden in 26 ml $H_2O$ gelöst, nach Zugabe von 6,5 ml 1 N NaOH während 5 Min. bei 25° stehengelassen und dann mit 2 N HCl auf pH 2,5 gestellt und lyophilisiert. Der Trockenrückstand wird in 30 ml $H_2O$ gelöst und nochmals lyophilisiert, worauf er 1,6 g wiegt und 0,38g NaCl enthält. Das erhaltende Z-Arg-Arg-Pro-Phe-His-OH liegt als Trihydrochlorid vor und wird in dieser Form zur Kondensation mit H-Sta-Ile-His-Lys(Boc)-OMe (Stufe 2.5) verwendet; $R_f$ (C) = 0,3.

<u>Beispiel 5:</u> 100 mg Z-Arg-Arg-Pro-Phe-His-Sta-Ile-His-Lys(Boc)-OMe(Bsp.4) werden in 500 µl 95%iger TFA gelöst, während 25 Min. stehengelassen und durch Zugabe von 5 ml Diisopropylether gefällt. Die Fällung wird abfiltriert, getrocknet, in 2 ml $H_2O$ gelöst und zur Umwandlung in das Acetat langsam durch eine Säule (Ø = 1 cm, Länge = 8 cm) von schwach basischem Ionenaustauscher in der Acetatform filtriert. Das Eluat wird

eingeengt und lyophilisiert, worauf man Z-Arg-Arg-Pro-Phe-His-Sta-Ile-His-Lys-OMe erhält; $R_f$(D) = 0,35; $R_f$ (M) = 0,3; $R_f$ (N) = 0,3.

Beispiel 6: 145 mg Z-Arg-Arg-Pro-Phe-His-Sta-Ile-His-Lys(Boc)-OMe (Bsp. 4) werden in 2,5 ml 95%igem MeOH gelöst und nach Zugabe von 15 mg Pd-Kohle unter Durchleiten von Wasserstoff und Rühren mit dem Magnetstäbchen hydriert bis zum vollständigen Verschwinden (gemäss Dünnschichtkontrolle) des Ausgangsproduktes. Man filtriert, engt das Filtrat zur Trockne ein, Löst in 3 ml $H_2O$ und lyophilisiert, worauf man H-Arg-Arg-Pro-Phe-His-Sta-Ile-His-Lys(Boc)-OMe, erhält; $R_f$ (D) = 0,32; $R_f$ (M) = 0,25; $R_f$ (N) = 0,3.

<u>Beispiel 7:</u> 200 mg H-Arg-Arg-Pro-Phe-His-Sta-Ile-His-Lys(Boc)-OMe (Bsp. 6) werden in 1 ml 95%iger TFA gelöst, während 25 Min. stehengelassen und dann durch Zugabe von 10 ml Diisopropylether ausgefällt. Man filtriert die Fällung ab, trocknet sie, löst in 4 ml $H_2O$ und filtriert die Lösung zwecks Umwandlung in das Acetat langsam durch eine Säule (Ø = 1 cm, Länge = 12 cm) von schwach basischem Ionenaustauscher in der Acetatform. Das Eluat wird auf ein kleines Volumen konzentriert und lyophilisiert, worauf man H-Arg-Arg-Pro-Phe-His-Sta-Ile-His-Lys-OMe erhält; $R_f$ (D) = 0,15; $R_f$ (M) = 0,12; $R_f$ (N) = 0,09.

<u>Beispiel 8:</u> 80 mg H-Arg-Arg-Pro-Phe-His-Sta-Ile-His-Lys-OMe (Bsp. 7) werden in 1,5 ml $H_2O$ gelöst und durch Zugabe von 0,1 N $NH_3$ stellt man einen pH-Wert von 5,5 ein. Man gibt 8 µl einer 1%igen wässrigen Trypsinlösung zu und hält den pH-Wert durch Zugabe von 0,1 N $NH_3$ mittels pH-Stat bei 5,5. Nach ca. 20 Min. ist die Basenaufnahme beendet, worauf man 350 µl Eisessig zugibt und während 2 Min. im siedenden Wasserbad erhitzt. Die Lösung wird zur Trockne eingeengt, der Rückstand in 2 ml 0,05 N AcOH gelöst und zur Abtrennung des inaktivierten Enzyms durch eine Säule (Ø = 1,2 cm; Länge = 25 cm) von Biogel P-10 (Polyacrylamidgel, Ausschlussgrenze bei einem Molekulargewicht von 20 000; Hersteller: Bio-Rad, Richmond, Californien, USA) gepumpt. Die durch UV-Detektion ermittelte Fraktion mit einem auf das untengenannte Peptid passenden Molekulargewicht wird auf ein kleines Volumen konzentriert und lyophilisiert, worauf man H-Arg-Arg-Pro-Phe-His-Sta-Ile-His-Lys-OH erhält; $R_f$ (D) = 0,12; $R_f$ (M) = 0,07; $R_f$ (N) = 0,05.

Beispiel 9: Analog den in dieser Anmeldung beschriebenen Verfahren erhält man:

H-Pro-Phe-His-Sta-Ala-Sta-OH,
H-Pro-Phe-His-Sta-Ala-Sta-OMe,
H-Pro-Phe-His-Sta-Ala-Sta-$NH_2$,
Z-Pro-Phe-His-Sta-Ala-Sta-OH,
Z-Pro-Phe-His-Sta-Ala-Sta-OMe,
Z-Pro-Phe-His-Sta-Ala-Sta-$NH_2$,
Boc-Pro-Phe-His-Sta-Ala-Sta-OH,
Boc-Pro-Phe-His-Sta-Ala-Sta-OMe,
Boc-Pro-Phe-His-Sta-Ala-Sta-$NH_2$,
H-Pro-Phe-His-Sta-Ile-Sta-OH,
H-Pro-Phe-His-Sta-Ile-Sta-OMe,
H-Pro-Phe-His-Sta-Ile-Sta-$NH_2$,
Z-Pro-Phe-His-Sta-Ile-Sta-OH,
Z-Pro-Phe-His-Sta-Ile-Sta-OMe,
Z-Pro-Phe-His-Sta-Ile-Sta-$NH_2$,
Boc-Pro-Phe-His-Sta-Ile-Sta-OH,
Boc-Pro-Phe-His-Sta-Ile-Sta-OMe,
Boc-Pro-Phe-His-Sta-Ile-Sta-$NH_2$,
(Boc, Z oder H)-Pro-Phe-His-Sta-Gly-Sta-(OH, OMe oder $NH_2$,
(Boc, Z oder H)-Pro-Phe-His-Sta-Sar-Sta-(OH, OMe oder $NH_2$ ),
(Boc, Z oder H)-Pro-Phe-His-Sta-β-Ala-His-(OH, OMe oder $NH_2$),
(Boc, Z oder H)-Pro-Phe-His-Sta-NH 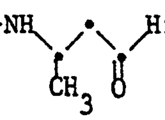 His-(OH, OMe oder $NH_2$),

(Boc, Z oder H)-Pro-Phe-His-Sta-O 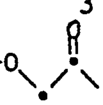 Sta-His-(OH, OMe oder $NH_2$),

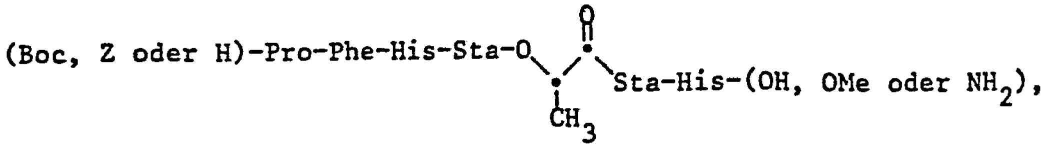,

(Boc, Z oder H)-Pro-Phe-His-Sta-Sta- His-(OH, OMe oder $NH_2$),

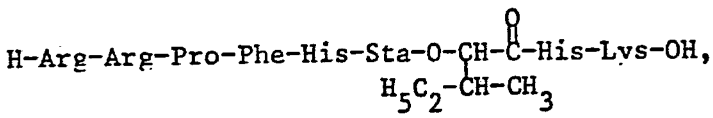,

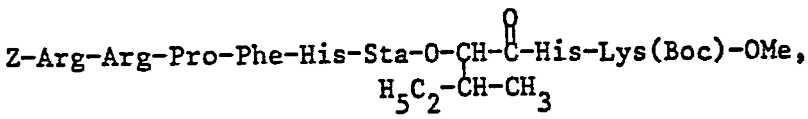,

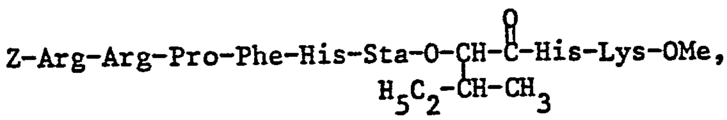,

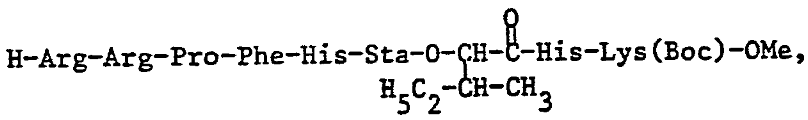,

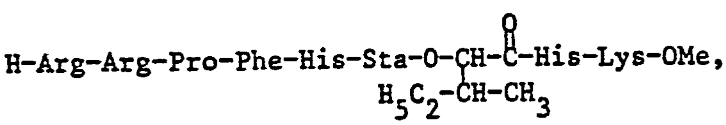,

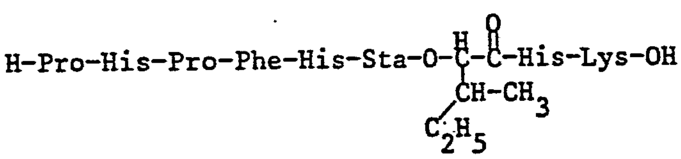 und

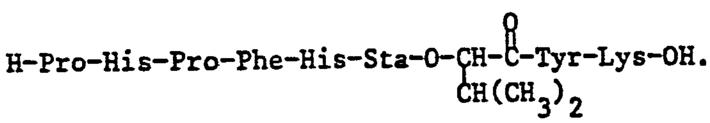.

Beispiel 10: Gelatine Lösung

Eine sterilfiltrierte wässrige Lösung von H-Arg-Arg-Pro-Phe-His-Sta-Ile-His-Lys-OH wird unter Erwärmen mit einer sterilen Gelatinelösung, welche als Konservierungsmittel Phenol enthält, unter aseptischen Bedingungen vermischt, so dass 1,0 ml Lösung folgende Zusammensetzung hat:

| | |
|---|---|
| H-Arg-Arg-Pro-Phe-His-Sta-Ile-His-Lys-OH | 3 mg |
| Gelatine | 150,0 mg |
| Phenol | 4,7 mg |
| dest. Wasser bis zu | 1,0 ml |

Die Mischung wird unter aseptischen Bedingungen in Vials zu 1,0 ml abgefüllt.

Beispiel 11: Sterile Trockensubstanz zur Injektion

Man löst 5 mg H-Arg-Arg-Pro-Phe-His-Sta-Ile-His-Lys-OH in 1 ml einer wässrigen Lösung mit 20 mg Mannit. Die Lösung wird sterilfiltriert und unter aseptischen Bedingungen in eine 2 ml Ampulle gefüllt, tiefgekühlt und lyophilisiert. Vor dem Gebrauch wird das Lyophilisat in 1 ml destilliertem Wasser oder 1 ml physiologischer Salzlösung gelöst. Die Lösung wird intramuskulär oder intravenös angewendet. Diese Formulierung kann auch in Doppelkammerspritzampullen abgefüllt werden.

Beispiel 12: Nasenspray

In einer Mischung von 3,5 ml "Miglyol 812" und 0,08 g Benzylalkohol werden 500 mg fein gemahlenes ($<$5,0 μ) H-Arg-Arg-Pro-Phe-His-Sta-Ile-His-Lys-OH suspendiert. Diese Suspension wird in einen Behälter mit Dosierventil eingefüllt. Es werden nun 5,0 mg "Freon 12" unter Druck durch das Ventil in den Behälter abgefüllt. Durch Schütteln wird das "Freon" in der Miglyol-Benzylalkoholmischung gelöst. Dieser Spraybehälter enthält ca. 100 Einzeldosen, die einzeln appliziert werden können.

Beispiel 13: Eine Lösung von 214 mg Z-Pro-Phe-His-OH, 152 mg H-Sta-Ala-Sta-OMe und 56 mg HOBt x $H_2O$ in 6 ml DMF wird im Eisbad gerührt und mit 97 mg DCCI versetzt. Es wird 1 Stunde bei 0° und 16 Stunden bei 25° gerührt, vom gebildeten Dicyclohexylharnstoff abfiltriert und das Filtrat im Hochvakuum eingedampft. Der Rückstand wird in 10 ml eines Methanol-Eisessig-Wasser-Gemisches (94:3:3) aufgenommen und 60 Minuten bei 60° gerührt. Das Lösungsmittel wird anschliessend am Rotationsverdampfer entfernt, der Rückstand mit wenig Essigester bei 0° verrührt, die resultierende Suspension filtriert und das Filtrat vom Lösungsmittel befreit. Nach erneutem Lösen in Essigester extrahiert man mit gesättigter Natriumbicarbonatlösung und mit Kochsalzlösung, trocknet die organischen Phasen und dampft ein. Dieser Rückstand wird an 80 g Kieselgel (Korngrösse 0,04-0,063 mm) unter Verwendung von Chloroform-Methanol (9:1) als Laufmittel chromatographiert (Fraktionen à ca. 20 ml). Die Fraktionen 16-23 werden vereinigt, vom Lösungsmittel befreit, der Rückstand in wenig Methanol aufgenommen, filtriert und das Filtrat erneut eingedampft. Nach Trocknung des Rückstands im Hochvakuum erhält man Z-Pro-Phe-His-Sta-Ala-Sta-OMe (vgl. Beispiel 9) als gelbliches Pulver; $R_f$ (G) = 0,72; $R_f$ ($CHCl_3$-MeOH [9:1]) = 0,26.

Der Ausgangsstoff H-Sta-Ala-Sta-OMe ist folgendermassen erhältlich:

Stufe 13.1: 371 mg Z-Sta-OH (vgl. Stufe 1.12), 240 mg H-Ala-$OC(CH_3)_3$ und 184 mg HOBt x $H_2O$ werden in 10 ml DMF gelöst. Nach Kühlung der Lösung auf 0° werden 133 mg N-Methylmorpholin und 321 mg DCCI zugegeben. Man rührt 1 Stunde bei 0° und 14 Stunden bei 25°, filtriert anschliessend vom gebildeten DCH ab und dampft das Filtrat zur Trockne ein. Der Rückstand wird in Essigester aufgenommen und die Lösung mit gesättigter Natriumhydrogencarbonatlösung extrahiert. Die organischen Phasen werden getrocknet, eingedampft und der Rückstand wird an 80 g Kieselgel (Korngrösse 0,04-0,063 mm) flashchromatographiert (Laufmittel: Methylenchlorid-Diethylether [4:1], Fraktionen à ca. 25 ml). Die Fraktionen 24-40 werden vereinigt, eingedampft, der Rückstand wird in wenig Methylenchlorid

gelöst, die Lösung filtriert und erneut eingedampft. Nach Trocknung des Rückstands im Hochvakuum erhält man Z-Sta-Ala-$OC(CH_3)_3$ als leicht gelbliches Oel; $R_f$ (Methylenchlorid-Diethylether [4:1]) = 0,10.

Stufe 13.2: 470 mg Z-Sta-Ala-$OC(CH_3)_3$ (aus Stufe 13.1) werden in 10 ml Trifluoressigsäure gelöst und die Lösung wird 10 Minuten bei 25°C gerührt. Anschliessend wird das Lösungsmittel am Rotationsverdampfer entfernt und der Rückstand an 200 g Kieselgel flashchromatographiert (Laufmittel zuerst: Chloroform-Methanol-konz. wässerige Ammoniaklösung [40:10:1], dann Chloroform-Methanol-konz. wässerige Ammoniaklösung [5:3:1]). Die produkthaltigen Fraktionen werden vereinigt und eingedampft, der Rückstand wird in Chloroform aufgenommen und die Chloroformlösung mit verdünnter Salzsäure und gesättigter Kochsalzlösung extrahiert, getrocknet und eingedampft. Nach Trocknung des Rückstands im Hochvakuum erhält man Z-Sta-Ala-OH als farblosen Schaum; $R_f$ (G) = 0,43.

Stufe 13.3: 250 mg Z-Sta-OH (vgl.Stufe 1.12) werden in 4 ml 90%igem Methanol gelöst. Die Lösung wird mit 1 ml einer 20%igen wässerigen Cäsiumcarbonatlösung versetzt und das Lösungsmittel anschliessend am Rotationsverdampfer entfernt. Der Rückstand wird 2mal in 2,5 ml DMF gelöst und das DMF jeweils wieder am Rotationsverdampfer entfernt. Der Rückstand wird im Hochvakuum getrocknet, dann in 2,5 ml DMF gelöst und die Lösung mit 55 µl Methyljodid versetzt. Es wird 18 Stunden bei 25°C gerührt, das Lösungsmittel anschliessend entfernt und der Rückstand in Essigester aufgenommen. Nach zweimaliger Extraktion mit Kochsalzlösung wird die organische Phase über Natriumsulfat getrocknet und eingedampft. Der Rückstand wird an 55 g Kieselgel flashchromatographiert (Laufmittel: Chloroform-Methanol [95:5]). Die produkthaltigen Fraktionen werden vereinigt und eingedampft, wobei man Z-Sta-OMe als gelbliches Oel erhält; $R_f$ (Chloroform-Methanol [95:5]) = 0,49.

Stufe 13.4: 818 mg Z-Sta-OMe gelöst in 40 ml Methanol werden in Gegenwart von 190 mg Palladium-auf-Kohle-Katalysator (10% Pd) bei Raumtemperatur und Normaldruck bis zur Sättigung hydriert. Während

der Hydrierung wird der pH-Wert durch kontinuierliche Zugabe von 0,5 N HCl bei 5 konstant gehalten. Nach der Hydrierung wird der Katalysator abfiltriert und das Filtrat eingedampft. Man erhält H-Sta-OMe x HCl als farblosen Schaum; $R_f$ (G) = 0,21. Dieses Rohprodukt wird direkt in Stufe 13.5 eingesetzt.

Stufe 13.5: Analog Stufe 13.1 erhält man, ausgehend von 150 mg H-Sta-OMe x HCl (aus Stufe 13.4), 253 mg Z-Sta-Ala-OH (aus Stufe 13.2), 102 mg HOBt x $H_2O$, 67 mg N-Methyl-morpholin und 178 mg DCCI, Z-Sta-Ala-Sta-OMe; $R_f$ (Chloroform-Methanol [9:1]) = 0,37.

Stufe 13.6: 259 mg Z-Sta-Ala-Sta-OMe (aus Stufe 13.5) gelöst in 10 ml 90%igem Methanol werden in Gegenwart von 30 mg Palladium-auf-Kohle-Katalysator (10% Pd) bei 25°, Normaldruck und unter $CO_2$-Absorption ($CO_2$ aus Abspaltung der Z-Schutzgruppe) bis zur Sättigung hydriert. Der Katalysator wird abfiltriert, das Filtrat eingedampft und der Rückstand im Hochvakuum getrocknet. Man erhält H-Sta-Ala-Sta-OMe als farblosen Schaum; $R_f$ (Chloroform-Methanol-Ammoniak [40:10:1]) = 0,41, $R_f$ (C) = 0,29.

Der Ausgangsstoff Z-Pro-Phe-His-OH ist folgendermassen erhältlich:

Stufe 13.7: 1,92 g Z-Pro-Phe-His-OMe (H. Derwald et al., J. Med. Chem. _7_, 50 [1964]), gelöst in 50 ml Methanol, werden mit 35 ml Wasser und 5,0 ml 1 normaler Natronlauge versetzt. Die Lösung wird 45 Minuten bei 25° gerührt und anschliessend mit 5,0 ml 1normaler Salzsäure neutralisiert. Das Lösungsmittel wird entfernt und der Rückstand an 10 g Kieselgel flashchromatographiert (Laufmittel: Chloroform-Methanol-konz. wässerige Ammoniaklösung [5:3:1]; Fraktionen à ca 15 ml). Die Fraktionen 6-13 werden vereinigt, eingedampft und der Rückstand in 70 ml Wasser gelöst. Nach Neutralisation durch Zugabe verdünnter Salzsäure bis pH 5,0 wird 2mal mit n-Butanol extrahiert und die vereinigten Butanolphasen werden mit Wasser gewaschen und eingedampft. Nach Trocknung des Rückstandes im Hochvakuum erhält man Z-Pro-Phe-His-OH als farbloses Pulver, $R_f$ (I) = 0,23.

Beispiel 14: 125 mg Z-Pro-Phe-His-Sta-Ala-Sta-OMe (aus Beispiel 13), gelöst in 10 ml 90%igem Methanol, werden in Gegenwart von 30 mg Palladium-auf-Kohle-Katalysator (10% Pd) bei 25°, Normaldruck und unter $CO_2$-Absorption ($CO_2$ aus Abspaltung der Z-Schutzgruppe) bis zur Sättigung hydriert. Anschliessend wird vom Katalysator abfiltriert, das Filtrat eingedampft und der Rückstand im Hochvakuum getrocknet. Man erhält H-Pro-Phe-His-Sta-Ala-Sta-OMe (vgl. Beispiel 9) als farblosen Schaum; $R_f$ (G) = 0,12.

Beispiel 15: 40 mg H-Pro-Phe-His-Sta-Ala-Sta-OMe werden in 10 ml gesättiger methanolischer Ammoniaklösung gelöst. Die Lösung lässt man 48 Stunden bei Raumtemperatur stehen, entfernt dann das Lösungsmittel am Rotationsverdampfer und chromatographiert den Rückstand an Kieselgel im System-Chloroform-Methanol-konz. wässrige Ammoniaklösung (40:10:1). Die Hauptfraktionen werden vereinigt und eingedampft. Nach Trocknung des Rückstands im Hochvakuum erhält man H-Pro-Phe-His-Sta-Ala-Sta-$NH_2$ (vgl. Beispiel 9) als farbloses Pulver; $R_f$ (Chloroform-Methanol-konz. Ammoniaklösung [40:10:1]) = 0,27.

Beispiel 16: 677 mg Z-Arg-Arg-Pro-Phe-His-OH x 3HCl (siehe Stufe 4.4) (enthaltend 24 Gew.-% NaCl), 272 mg H-Sta-Sar-His-Lys(Boc)-OMe, 85 mg HOBt x $H_2O$ und 48 µl N-Methyl-morpholin werden in 6 ml DMF gelöst. Nach Abkühlen auf 0° gibt man 124 mg DCCI zu, rührt einige Minuten und lässt dann über Nacht bei 0° und während 30 Std. bei Raumtemperatur stehen. Der ausgeschiedene DCH wird abfiltriert, das Filtrat zur Trockne eingeengt, der Rückstand in 12 ml Methanol-Eisessig-$H_2O$ (94:3:3) während 1 Std. auf 60° erwärmt, die Lösung auf ca. 3 ml konzentriert und das Rohprodukt durch Zugabe von 30 ml Diisoproylether ausgefällt. Zur Umwandlung ins Acetat wird in 5 ml 0,05 N Essigsäure gelöst, von wenig Unlöslichem abfiltriert und das Filtrat langsam durch eine Säule von schwach basischem Anionenaustauscher (Merck Nr. II; Länge = 11 cm; ∅ = 1,2 cm) filtriert. Das Eluat wird lyophilisiert und mittels Craig-Verteilung im System n-Butanol-Eisessig-$H_2O$ (4:1:5) über 510 Stufen gereinigt (K = 0,4).

Die chromatographisch einheitlichen Fraktionen werden vereinigt, auf ein kleines Volumen konzentriert und lyophilisiert. Z-Arg-Arg-Pro-Phe-His-Sta-Sar-His-Lys(Boc)-OMe wird so in der Acetatform als amorphes, gut wasserlösliches Pulver erhalten; $R_f$ (D) = 0,31; $R_f$ (M) = 0,44: $R_f$ (O) = 0,32.

Das als Ausgangsmaterial verwendete H-Sta-Sar-His-Lys(Boc)-OMe ist folgendermassen erhältlich:

Stufe 16.1: 500 mg Z-His-Lys(Boc)-OMe werden in 10 ml MeOH und 0,94 ml 1 N HCl gelöst. Nach Zugabe von 50 mg Pd-Kohle (10% Pd) wird unter $CO_2$-Absorption bis zur Sättigung hydriert, dann der Katalysator abfiltriert und das Filtrat auf ca. 2 ml konzentriert. Man gibt 10 ml DMF zu und engt wieder auf 5 ml ein. Diese Lösung enthält 401 mg HCl x H-His-Lys(Boc)-OMe. Sie wird mit 252 mg Z-Sar-OH, 53 µl N-Methyl-morpholin, 144 mg HOBt x $H_2O$ und 252 mg DCCI versetzt, bis zur Auflösung aller Komponenten kurz gerührt und dann während 20 Stunden bei Raumtemperatur stehen gelassen. Der auskristallisierte DCH wird bei 0° abfiltriert, das Filtrat im Hochvakuum zum Honig eingeengt, in 11 ml Methanol-Eisessig-$H_2O$ (94:3:3) während einer Stunde auf 60° erwärmt und wieder auf 5 ml eingeengt. Man fällt durch Zugabe von 50 ml Diisopropylether aus, lässt 1 Stunde im Eisbad stehen und dekantiert die überstehende Mutterlauge. Das Rohprodukt wird durch Chromatographie an 25 g Kieselgel (Merck Nr. 60, 230-400 mesh) durch Elution mit Chloroform-Methanol-$H_2O$-Eisessig (180:20:2:1) gereinigt. Die reinen Fraktionen werden zur Entfernung der Essigsäure in Essigester gelöst, nacheinander mit 5%iger wässeriger Natriumhydrogencarbonatlösung und mit $H_2O$ gewaschen und durch Einengen der Essigesterphase erhält man daraus Z-Sar-His-Lys(Boc)-OMe als amorphes Pulver; $R_f$ (I) = 0,45; $R_f$ (G) = 0,6.

Stufe 16.2: 390 mg Z-Sar-His-Lys(Boc)-OMe werden in 10 ml 95%igem MeOH gelöst und nach Zugabe von 40 mg Pd-Kohle unter $CO_2$-Absorption bis zur Sättigung hydriert. Der Katalysator wird abfiltriert, das

Filtrat zur Trockne eingeengt und der amorphe Rückstand im Hochvakuum bei 40° zur Gewichtskonstanz getrocknet, worauf man H-Sar-His-Lys(Boc)-OMe erhält; $R_f$ (G) = 0,1; $R_f$ (E) = 0,25.

Stufe 16.3: 254 mg Z-Sta-OH (siehe Stufe 1.12), 297 mg H-Sar-His-Lys(Boc)-OMe, 97 mg HOBt x $H_2O$ und 196 mg DCCI werden in dieser Reihenfolge in 3 ml DMF gelöst und während 20 Stunden bei Raumtemperatur stehengelassen. Der auskristallisierte DCH wird abfiltriert, das Filtrat zur Trockne eingeengt, in 7 ml Methanol-Eisessig-$H_2O$ (94:3:3) während 1 Stunde auf 60° erwärmt und diese Lösung wiederum zur Trockne eingeengt. Der ölige Rückstand wird durch Chromatographie an 75 g Kieselgel (Merck Nr. 60, 230-400 mesh) durch Elution mit $CHCl_3$-MeOH (9:1) gereinigt. Die reinen Fraktionen werden in Essigester gelöst, mit $NaHCO_3$-Lösung und mit $H_2O$ gewaschen und durch Einengen der organ. Phase wird Z-Sta-Sar-His-Lys(Boc)-OMe in amorpher Form (Honig) erhalten; $R_f$ (F) = 0,15; $R_f$ (I) = 0,5.

Stufe 16.4: 380 mg Z-Sta-Sar-His-Lys(Boc)-OMe werden in 10 ml 95%igem MeOH nach Zugabe von 100 mg Pd-Kohle (10% Pd) unter $CO_2$-Absorption bis zur Sättigung hydriert, die Lösung filtriert und zur Trockne eingeengt. Nach Trocknen im Hochvakuum erhält man H-Sta-Sar-His-Lys(Boc)-OMe als amorphes Pulver; $R_f$ (G) = 0,18; $R_f$ (E) = 0,53.

Beispiel 17: 100 mg Z-Arg-Arg-Pro-Phe-His-Sta-Sar-His-Lys(Boc)-OMe (siehe Beispiel 16) werden in 2ml 95%igem MeOH gelöst und nach Zugabe von 10 mg Pd-Kohle (10% Pd) unter Durchleiten von Wasserstoff bis zum vollständigen Verschwinden des Ausgangsmaterials (Dünnschicht-Kontrolle) hydriert. Der Katalysator wird abfiltriert, das Filtrat zur Trockne eingeengt, der Rückstand in 2 ml $H_2O$ gelöst und lyophilisiert, worauf man H-Arg-Arg-Pro-Phe-His-Sta-Sar-His-Lys-(Boc)-OMe erhält; $R_f$ (D) = 0,15; $R_f$ (M) = 0,25; $R_f$ (O) = 0,22.

Beispiel 18: 77 mg H-Arg-Arg-Pro-Phe-His-Sta-Sar-His-Lys(Boc)-OMe (aus Beispiel 17) werden in 0,4 ml 95%iger TFA gelöst und während 25 Minuten stehengelassen. Durch Zugabe von 5 ml Diisopropylether und

Abfiltrieren des Niederschlags erhält man H-Arg-Arg-Pro-Phe-His-Sta-Sar-His-Lys-OMe als amorphes, hygroskopisches Pulver; $R_f$ (D) = 0,04; $R_f$ (M) = 0,04; $R_f$ (O) = 0,05.

Zur Herstellung des Acetats filtriert man die erhaltene Verbindung durch eine Säule mit schwach basischem Ionentauscher in der Acetatform wie üblich.

Beispiel 19: 110 mg H-Arg-Arg-Pro-Phe-His-Sta-Sar-His-Lys-OMe (als Trifluoracetat; siehe Beispiel 18) werden in 1 ml $H_2O$ gelöst und der pH-Wert wird mit 0,5 N $NH_3$ auf 5,0 gestellt. Nach Zufügen von 20 µl einer 1%igen wässerigen Trypsinlösung wird der pH-Wert mittels pH-Stat unter Rühren bei Raumtemperatur und Zufügen von 0,1 N $NH_3$ bei 5,0 gehalten. Nach ca. 10-15 Min. ist die Basenaufnahme beendet. Die Lösung wird mit 200 µl Eisessig angesäuert, während 2 Minuten im siedenden Wasserbad erhitzt und lyophilisiert. Zur Umwandlung ins Acetat löst man den Rückstand in $H_2O$ und filtriert langsam durch eine in 0,05 N Essigsäure äquilibrierte Säule (∅ = 0,7 cm; Länge = 8 cm) von schwach basischem Ionentauscher (z.B. Merck Nr. II). Das Eluat wird auf ein kleines Volumen konzentriert und lyophilisiert, wobei man H-Arg-Arg-Pro-Phe-His-Sta-Sar-His-Lys-OH (als Acetat) als amorphes, gut wasserlösliches Pulver erhält; $R_f$ (D) = 0,03; $R_f$ (M) = 0,03; $R_f$ (O) = 0,03.

Beispiel 20: 721 mg Z-Arg-Arg-Pro-Phe-His-OH x 3 HCl (siehe Stufe 4.4) (enthaltend 24 Gew.-% NaCl), 176 mg H-Sta-ß-Ala-His-$NH_2$, 91 mg HOBt x $H_2O$ und 50 µl N-Methyl-morpholin werden in 5 ml DMF gelöst. Nach Abkühlen auf 0° werden 132 mg DCCI zugegeben und dann wird während 6 Stunden bei 0° gerührt und über Nacht bei Raumtemperatur stehengelassen. Der auskristallisierte DCH wird abfiltriert, das Filtrat zur Trockne eingeengt und der Rückstand in 14 ml Methanol-Eisessig-$H_2O$ (94:3:3) während 1 Std. auf 60° erwärmt. Die Lösung wird auf 3 ml konzentriert und das Rohprodukt durch Zugabe von 30 ml Diisopropylether ausgefällt. Zur Umwandlung ins Acetat löst man in 5 ml 0,05 N Essigsäure, filtriert ein wenig Unlösliches ab und filtriert die Lösung langsam durch eine in 0,05 N Essigsäure

äquilibrierte Säule (Ø = 1,2 cm, Länge = 10 cm) von schwach basischem Ionentauscher in der Acetatform (Merck II). Das Eluat wird auf ca. 3 ml eingeengt und lyophilisiert. Die Reinigung erfolgt mittels Craig-Verteilung über 750 Stufen im System n-Pentanol-Eisessig-$H_2O$ (4:1:5); K = 0,13. Die chromatoraphisch reinen Fraktionen werden vereinigt, zur Trockne eingeengt und der Rückstand in 3 ml $H_2O$ gelöst und lyophilisiert. Man erhält Z-Arg-Arg-Pro-Phe-His-Sta-ß-Ala-His-$NH_2$ (Acetatform) als amorphes, wasserlösliches Pulver; $R_f$ (D) = 0,17; $R_f$ (M) = 0,30; $R_f$ (O) = 0,25.

Das als Ausgangsmaterial verwendete H-Sta-ß-Ala-His-$NH_2$ ist folgendermassen erhältlich:

Stufe 20.1: 6,0 g ß-Alanin werden in 33,7 ml 2N NaOH aufgenommen und auf +5° gekühlt. Bei dieser Temperatur werden nun gleichzeitig 22,5 ml einer 50proz. Lösung von Chlorameisensäurebenzylester in Toluol und 16,8 ml 4N NaOH zugetropft. Die Emulsion wird 3 Stunden im Eisbad nachgerührt. Nach Abtrennen der organischen Phase wird die wässrige Phase noch einmal mit Ether extrahiert, und anschliessend unter Eisbadkühlung mit 44 ml 2 N HCl auf pH 1 gestellt. Durch Abfiltrieren des Niederschlages und Trocknen im Hochvakuum bei 50° erhält man Z-ß-Ala-OH als weisses Pulver; $R_f$ ($CH_2Cl_2$-MeOH-konz. wässerige Ammoniaklösung [5:3:1]) = 0,37.

Stufe 20.2: Eine Lösung von 12,3 g Z-ß-Ala-OH und 16,0 g H-His-$OCH_3$ x 2 HCl in 300 ml DMF wird auf 0° abgekühlt und tropfenweise zuerst mit einer Lösung von 22,75 g Phosphorsäurediphenylesterazid in 110 ml DMF und dann mit einer Lösung von 30,7 ml Triethylamin in 110 ml DMF versetzt. Die so erhaltene Suspension wird während 2 Tagen bei Raumtemperatur gerührt und anschliessend im Hochvakuum zu einer öligen Suspension eingeengt. Dieser Rückstand wird in Essigester aufgenommen, mit Natriumhydrogencarbonat und Sole gewaschen, getrocknet und eingedampft. Das Rohprodukt wird durch Flash-Chromatographie (1,6 kg Kieselgel [ Typ 60, Merck], 40-63 µm,

0,4 bar, Fraktionen à 400 ml, Laufmittel: $CH_2Cl_2$-MeOH [13:2]) aufgetrennt. Durch Eindampfen der entsprechenden Fraktionen erhält man Z-ß-Ala-His-$OCH_3$ als leicht gelblichen Schaum; $R_f$ (L) = 0,69.

Stufe 20.3: 9,5 g Z-ß-Ala-His-$OCH_3$ werden in 95 ml 7normaler methanolischer Ammoniaklösung bei Raumtemperatur über Nacht gerührt. Die Suspension wird mit 30 ml Methanol verdünnt und während weiteren 2,5 Stunden bei Raumtemperatur gerührt. Die Kristalle werden abfiltriert und bei 30° im Hochvakuum getrocknet. Die Mutterlauge wird eingedampft und der Rückstand in 80 ml Isopropanol gelöst. Man versetzt mit 200 ml Ether und lässt über Nacht unter Rühren bei Raumtemperatur nochmals auskristallisieren.Die abfiltrierten Kristalle werden ebenfalls getrocknet. Die Kristalle aus beiden Kristallisationen bestehen aus Z-ß-Ala-His-$NH_2$ (leicht beige, Smp. 181-82°); $R_f$ (C) = 0.57.

Stufe 20.4: 8,19 g Z-ß-Ala-His-$NH_2$ werden in 200 ml Methanol-Wasser (95:5) mit 0,82 g Palladium-Kohle (5% Pd) bei Raumtemperatur unter Normaldruck während 15 Stunden hydriert. Nach Abfiltrieren des Katalysators, Abdampfen des Lösungsmittels und Trocknen erhält man H-ß-Ala-His-$NH_2$ als farbloses Pulver; $R_f$ (L) = 0,01.

Stufe 20.5: 370 mg Z-Sta-OH, 225 mg H-ß-Ala-His-$NH_2$, 153 mg HOBt x $H_2O$ und 247 mg DCCI werden in 4,5 ml DMF suspendiert und bei Raumtemperatur gerührt. Nach ca. 10 Minuten ist alles klar gelöst, und nach ca. 20 Minuten beginnt DCH auszukristallisieren. Die Mischung wird über Nacht stehengelassen, dann während 30 Minuten bei 0° gerührt, DCH abfiltriert und das Filtrat bis zum Oel eingeengt. Dieses wird in 14 ml Methanol-Eisessig-$H_2O$ (94:3:3) während 1 Stunde auf 60° erwärmt, die Lösung auf 3 ml eingeengt und das Rohprodukt durch 30 ml Diisopropylether ausgefällt und getrocknet. Zur Entfernung der Essigsäure wird in n-Butanol gelöst, nacheinander mit 5%iger $NaHCO_3$-Lösung und mit $H_2O$ gewaschen und die Butanolphase wieder zur Trockne eingeengt. Nach Reinigung durch Chromatographie

an 30 g Kieselgel (Merck Nr. 60, 230-400 mesh) unter Eluieren mit Chloroform-Methanol (75:25) erhält man Z-Sta-ß-Ala-His-$NH_2$; $R_f$ (G) = 0,28.

Stufe 20.6: 240 mg Z-Sta-ß-Ala-His-$NH_2$ werden in 10 ml 95%igem Methanol mit 50 mg Pd-Kohle (10% Pd) unter $CO_2$-Absorption bis zur Sättigung hydriert. Der Katalysator wird abfiltriert, das Filtrat zur Trockne eingeengt und der feste, amorphe Rückstand pulverisiert und im Hochvakuum nachgetrocknet, worauf man H-Sta-ß-Ala-His-$NH_2$ erhält; $R_f$ (E) = 0,11; $R_f$ (M) = 0,26.

Beispiel 21: 98 mg Z-Arg-Arg-Pro-Phe-His-Sta-ß-Ala-His-$NH_2$ (aus Beispiel 20) werden in 2 ml 95%igem MeOH gelöst und mit 20 mg Pd-Kohle (10% Pd) unter Rühren mit Magnet und Durchleiten von Wasserstoff bis zum Verschwinden des Ausgangsmaterials hydriert. Der Katalysator wird sodann abfiltriert, das Filtrat zur Trockne eingeengt und der glasige Rückstand in 2 ml $H_2O$ gelöst und lyophilisiert, worauf man H-Arg-Arg-Pro-Phe-His-Sta-ß-Ala-His-$NH_2$ als amorphes Pulver erhält; $R_f$ (D) = 0,05; $R_f$ (M) = 0,06; $R_f$ (O) = 0,06.

Beispiel 22: 333 mg Z-Arg-Arg-Pro-Phe-His-OH x 3 HCl (siehe Stufe 4.4; enthaltend 24 Gew.-% NaCl), 106 mg H-Sta-Lac-His-$NH_2$ x 2 HCl, 36 mg HOBt x $H_2O$ und 64 µl N-Methyl-morpholin werden in 1 ml DMF gelöst. Bei 0° gibt man 86 mg DCCI zu, rührt während 10 Minuten und lässt dann 15 Stunden bei 0° und 2 Tage bei Raumtemperatur stehen. Die unlöslichen Anteile (NaCl und DCH) werden abfiltriert und mit DMF gewaschen, das Filtrat zum Oel eingeengt, in 10 ml Methanol-Eisessig-$H_2O$ (94:3:3) während 1 Stunde auf 60° erwärmt und erneut eingeengt. Durch Zerreiben mit 40 ml Diisopropylether, Dekantieren und Trocknen des Rückstandes erhält man ein Rohprodukt, das in 4 ml 0,05 N Essigsäure gelöst und durch langsames Filtrieren durch eine Säule (Ø = 1 cm; Länge 10 cm) von schwach-basischem Ionentauscher (Acetatform, Merck II) ins Acetat umgewandelt wird. Das Eluat wird auf ca. 3 ml konzentriert und lyophilisiert und der Rückstand in einer Craig-Verteilung über 920 Stufen im System n-Butanol-Eisessig-$H_2O$ (4:1:5) gereinigt (K = 0,14). Die reinen Fraktionen werden vereinigt, zur Trockne eingeengt, der Rückstand in 2 ml $H_2O$ gelöst und lyophilisiert. Das so erhaltene Acetat von Z-Arg-Arg-Pro-Phe-His-Sta-Lac-His-$NH_2$ ist amorph und gut wasserlöslich; $R_f$ (D) = 0,23; $R_f$ (M) = 0,36; $R_f$ (O) = 0,29.

Das als Ausgangsmaterial verwendete H-Sta-Lac-His-$NH_2$ ist folgendermassen erhältlich:

Stufe 22.1: Eine Lösung von 138 mg (3S,4S)-N-Boc-Statin (Herstellung vgl. z.B. D.H. Rich et al., J. Org. Chem. 43, 3624 [1978]), 90 mg L-Milchsäurebenzylester und 62 mg 4-Dimethylaminopyridin, gelöst in 9 ml absolutem Methylenchlorid (filtriert durch Aluminiumoxid mit der Aktivitätsstufe I), wird bei 25° mit einer Lösung von 124 mg DCCI in 3 ml absolutem Methylenchlorid versetzt. Aus der resultierenden Lösung fällt nach wenigen Minuten DCH aus. Nach 2 Stunden wird filtriert und das Filtrat bis zu einem kleinen Volumen eingedampft. Die zurückbleibende Lösung wird an 90 g Kieselgel (Korngrösse 0,04-0,063 mm) bei einem Druck von ca. 0,3 bar flash-chromatographiert (Laufmittel: $CH_2Cl_2$-Diethylether [4:1], Fraktionen à ca. 20 ml). Die Fraktionen 6 und 7 werden vereinigt und das Lösungsmittel wird am Rotationsverdampfer entfernt. Man erhält Boc-Sta-Lac-$OCH_2$-$C_6H_5$ als gelbliches Oel, welches direkt in Stufe 22.2 eingesetzt wird; $R_f$ ($CH_2Cl_2$-Diethylether [4:1]) = 0,44.

Stufe 22.2: Eine Lösung von 79 mg Boc-Sta-Lac-$OCH_2$-$C_6H_5$ in 10 ml absolutem Dioxan wird in Gegenwart von 20 mg Palladium-auf-Kohle-Katalysator (10% Pd) bei 25° während 4 Stunden hydriert. Der Katalysator wird abfiltriert, das Filtrat eingedampft und der Rückstand im Hochvakuum getrocknet. Man erhält Boc-Sta-Lac-OH als farbloses Oel welches direkt in Stufe 22.3 eingesetzt wird; $R_f$ (G) = 0,60.

Stufe 22.3: 63 mg Boc-Sta-Lac-OH, 28 mg H-His-$NH_2$ und 28 mg HOBt x $H_2O$ werden in 2 ml DMF gelöst. Die Lösung wird im Eisbad gekühlt und mit 49 mg DCCI versetzt. Es wird während 4 Stunden bei 0° und während 16 Stunden bei 25° gerührt, wobei DCH ausfällt.
Die Suspension wird filtriert, das Filtrat im Hochvakuum eingedampft und der Rückstand in 3 ml eines Methanol-Eisessig-Wasser-Gemisches

(94:3:3) aufgenommen und 1 Stunde bei 60° gerührt. Die Suspension wird erneut eingedampft, der Rückstand in wenig Methanol-Essigester (1:1) aufgeschlämmt, filtriert und das Filtrat zur Trockne eingeengt. Der Rückstand wird an 35 g Kieselgel bei ca. 0,3 bar flashchromatographiert (Laufmittel: Methylenchlorid-Methanol-konz. wässerige Ammoniaklösung [80:10:1], Fraktionen à ca. 10 ml). Die Fraktionen 24-35 werden vereinigt, das Lösungsmittel entfernt, der Rückstand wird in wenig Methanol aufgenommen, die Lösung filtriert und das Filtrat erneut eingedampft. Nach Trocknung des Rückstands im Hochvakuum erhält man Boc-Sta-Lac-His-$NH_2$ als farbloses Pulver, welches direkt in Stufe 22.4 eingesetzt wird; $R_f$ ($CH_2Cl_2$-MeOH-konz. wässerige Ammoniaklösung [40:10:1]) = 0,62.

Stufe 22.4: 118 mg Boc-Sta-Lac-His-$NH_2$ werden in 1,18 ml Methanol gelöst, mit 2,36 ml 5 N HCl in Dioxan versetzt und während 2 Min. stehengelassen. Man fällt durch Zugabe von 30 ml Diisopropylether-Petrolether (1:1) ein Oel aus, lässt während 20 Min. bei 0° stehen, dekantiert die überstehende Mutterlauge und trocknet das Oel im Hochvakuum. Der Rückstand wird in 4 ml $H_2O$ gelöst und lyophilisiert, worauf man H-Sta-Lac-His-$NH_2$ (als Hydrochlorid) in Form eines amorphen, hygroskopischen Pulvers erhält; $R_f$ (B) = 0,05; $R_f$ (M) = 0,38; $R_f$ (O) = 0,26.

Beispiel 23: 30 mg Z-Arg-Arg-Pro-Phe-His-Sta-Lac-His-$NH_2$ (s. Beispiel 22) werden in 1 ml 95%igem Methanol gelöst und nach Zugabe von 10 mg Pd-Kohle (10% Pd) unter Rühren und Durchleiten von Wasserstoff hydriert. Nach beendigter Reaktion (Dünnschicht-Kontrolle) wird der Katalysator abfiltriert, das Filtrat zur Trockne eingeengt, der Rückstand in 0,6 ml $H_2O$ gelöst und lyophilsiert, worauf man H-Arg-Arg-Pro-Phe-His-Sta-Lac-His-$NH_2$ als Acetat in Form eines amorphen, wasserlöslichen Pulvers erhält; $R_f$ (D) = 0,07; $R_f$ (M) = 0,09; $R_f$ (O) = 0,09.

Beispiel 24: In analoger Weise wie in dieser Anmeldung beschrieben erhält man
Z-Arg-Arg-Pro-Phe-His-Sta(Ac)-Ile-His-Lys(Boc)-$OCH_3$,

H-Pro-His-Pro-Phe-His-Sta-Phe-Ile-His-Lys-OH mit $R_f$ (M) = 0,14 und $R_f$ (N) = 0,14,

H-Arg-D-Arg-Pro-Phe-His-Sta-Ile-His-Lys-OH (vgl. Beispiel 3) mit $R_f$ (M) = 0,06, $R_f$ (N) = 0,08 und $R_f$ (D) = 0,12,

H-Pro-His-Pro-Phe-D-His-Sta-Phe-Ile-His-Lys-OH mit $R_f$ (M) = 0,15, $R_f$ (K) = 0,23 und $R_f$ (D) = 0,17,

H-Pro-His-Pro-Phe-His-(3R,4S)-Sta-Ile-His-Lys-OH mit $R_f$ (M) = 0,125, $R_f$ (K) = 0,14 und $R_f$ (D) = 0,125,

H-Pro-His-Pro-Phe-D-His-Sta-Ile-His-Lys-OH mit $R_f$ (M) = 0,10, $R_f$ (K) = 0,21 und $R_f$ (D) = 0,125,

Z-Ile-His-Pro-Phe-His-Sta-Ile-His-Lys(Boc)-OMe mit $R_f$ (B) = 0,30 und $R_f$ (E) = 0,71,

H-Ile-His-Pro-Phe-His-Sta-Ile-His-Lys-OH mit $R_f$ (M) = 0,21, $R_f$ (N) = 0,14 und $R_f$ (D) = 0,20,

Z-Arg-His-Pro-Phe-His-Sta-Ile-His-Lys(Boc)-OMe mit $R_f$ (B) = 0,58, $R_f$ (M) = 0,52 und $R_f$ (E) = 0,19,

H-Arg-His-Pro-Phe-His-Sta-Ile-His-Lys-OH mit $R_f$ (M) = 0,04, $R_f$ (N) = 0,13 und $R_f$ (D) = 0,08.

Patentansprüche für die Vertragsstaaten BE CH DE GB FR IT LI LU NL und SE

1. Substituierte Tetrapeptide der Formel I,

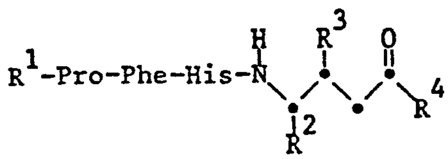

(I)

worin $R^1$ Wasserstoff oder Acyl, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, und Salze solcher Verbindungen mit salzbildenden Gruppen.

2. Verbindungen der Formel I nach Anspruch 1, worin $R^1$ den Acylrest eines Dipeptids, bestehend aus zwei in der Natur vorkommenden (L)-Aminosäuren oder deren D-Isomeren, den Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder einen anderen aliphatischen, aromatischen oder aromatisch-aliphatischen Acylrest mit bis zu 18 C-Atomen, der verschieden ist von dem Acylrest von N-unsubstituiertem oder N-substituiertem (D)- oder (L)-Histidin oder Sarkosin, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, mit Ausnahme der Verbindungen (H, Boc oder N-Isovaleryl)-Pro-His-Pro-Phe-His-Sta-Leu-Phe-$NH_2$ und (H, Boc, N-Acetyl oder N-Phenoxyacetyl)-Pro-Phe-His-Sta-Leu-Phe-$NH_2$, und Salze von solchen Verbindungen mit salzbildenden Gruppen.

3. Verbindungen der Formel I nach Anspruch 1, worin $R^1$ den Acylrest eines Dipeptids, bestehend aus zwei in der Natur vorkommenden (L)-Aminosäuren oder deren D-Isomeren, den Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder einen anderen aliphatischen, aromatischen oder aromatisch-aliphatischen Acylrest mit bis zu 18 C-Atomen, der verschieden ist von dem Acylrest von N-unsubstituiertem oder N-substituiertem (D)- oder (L)-Histidin oder Sarkosin, $R^2$ Alkyl oder Aralkyl mit jeweils nicht mehr als 18 C-Atomen, $R^3$ freies oder verestertes Hydroxy mit höchstens 18 C-Atomen und $R^4$ freies oder durch Arylniederalkyl mit höchstens 18 C-Atomen oder Niederalkyl substituiertes Amino, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, oder freies oder verethertes Hydroxy mit höchstens 12 C-Atomen bedeuten, mit Ausnahme der Verbindungen (H, Boc oder N-Isovaleryl)-Pro-His-Pro-Phe-His-Sta-Leu-Phe-$NH_2$ und (H, Boc, N-Acetyl oder N-Phenoxyacetyl)-Pro-Phe-His-Sta-Leu-Phe-$NH_2$, und Salze von solchen Verbindungen mit salzbildenden Gruppen.

4. Verbindungen der Formel I nach Anspruch 1, worin $R^1$ den Rest eines aus höchstens 6 in der Natur vorkommenden Aminosäuren und/oder ihren (D)-Isomeren aufgebauten Peptids, wobei in diesen Resten vorhandene freie funktionelle Gruppen gegebenenfalls in geschützter Form vorliegen, $R^2$ Niederalkyl oder Phenylniederalkyl, $R^3$ freies Hydroxy und $R^4$ freies Amino oder Amino, das durch gegebenenfalls durch Phenyl substituiertes Niederalkyl substituiert ist, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter

Form vorliegen, freies Hydroxy oder Niederalkoxy bedeuten, und Salze von solchen Verbindungen mit salzbildenden Gruppen mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, und Salze von solchen Verbindungen mit salzbildenden Gruppen.

5. Verbindungen der Formel I nach Anspruch 1, worin $R^1$ den Acylrest eines aus in der Natur vorkommenden Aminosäuren aufgebauten Dipeptids oder Niederalkanoyl, $R_2$ verzweigtes Niederalkyl, $R^3$ freies Hydroxy und $R^4$ freies oder durch Niederalkyl oder Benzyl substituiertes Amino, oder den N-terminalen Rest einer in der Natur vorkommenden Aminosäure oder eines aus solchen Aminosäuren aufgebauten Di- oder Tripeptids bedeuten, mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, und Salze von solchen Verbindungen mit salzbildenden Gruppen.

6. Verbindungen der Formel I nach Anspruch 1, worin $R^1$ den Acylrest eines aus in der Natur vorkommenden Aminosäuren aufgebauten Dipeptids, $R^2$ 2-Methyl-propyl, $R^3$ freies Hydroxy und $R^4$ den N-terminalen Rest eines aus in der Natur vorkommenden Aminosäuren aufgebauten Di- oder Tripeptids bedeuten, mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, und pharmazeutisch verwendbare Salze dieser Verbindungen.

7. Verbindungen der Formel I nach einem der Ansprüche 1-6, worin in den Resten $R^1$ und $R^4$ vorkommende Aminosäurereste sich von jenen 20 Aminosäuren in ihrer natürlichen Konfiguration ableiten, die regelmässig in Proteinen vorkommen, und pharmazeutisch verwendbare Salze dieser Verbindungen.

8. Verbindungen der Formel I nach Anspruch 1, worin $R^1$ den Rest H-Pro-His-, $R^2$ 2-Methyl-propyl, $R^3$ freies Hydroxy und $R^4$ den Rest -Val-Tyr-Lys-OH, -Ile-His-Lys-OH, -Ile-His-Ser-OH, -Val-Tyr-Ser-OH, -Val-Tyr-OH, -Ile-His-OH, -Val-Tyr-$NH_2$, -Ile-His-$NH_2$ oder -Ala-Sta-OH bedeuten, und pharmazeutisch verwendbare Salze dieser Verbindungen.

9. Verbindungen der Formel I nach Anspruch 1, worin $R^1$ den Rest H-Ile-His-, $R^2$ 2-Methyl-propyl, $R^3$ freies Hydroxy und $R^4$ den Rest -Val-Tyr-Lys-OH, -Ile-His-Lys-OH, -Ile-His-Ser-OH, -Val-Tyr-Ser-OH, -Val-Tyr-OH, -Ile-His-OH, -Val-Tyr-$NH_2$, -Ile-His-$NH_2$ oder -Ala-Sta-OH bedeuten, und pharmazeutisch verwendbare Salze dieser Verbindungen.

10. Verbindungen der Formel I nach einem der Ansprüche 1-9, die mindestens zwei Aminosäuren enthalten, die von α-Aminosäuren verschieden sind, und Salze von solchen Verbindungen mit salzbildenden Gruppen.

11. Verbindungen der Formel I nach Anspruch 10, die zwei Statinmoleküle enthalten, und Salze von solchen Verbindungen mit salzbildenden Gruppen.

12. Verbindungen der Formel I nach Anspruch 11, die die Sequenz -Sta-Ala-Sta- aufweisen, und Salze von solchen Verbindungen mit salzbildenden Gruppen.

13. Verbindungen der Formel I nach einem der Ansprüche 1-7, die die Sequenz -Sta-Gly-Sta- aufweisen, und Salze von solchen Verbindungen mit salzbildenden Gruppen.

14. Verbindungen der Formel I nach einem der Ansprüche 1-7, die die Sequenz -Sta-Ile-Sta aufweisen, und Salze von solchen Verbindungen mit salzbildenden Gruppen.

15. Verbindungen der Formel I nach einem der Ansprüche 1-7, die die Sequenz -Sta-Sar-Sta- aufweisen, und Salze von solchen Verbindungen mit salzbildenden Gruppen.

16. Verbindungen der Formel I nach einem der Ansprüche 1-7, die β-Alanin enthalten, und Salze von solchen Verbindungen mit salzbildenden Gruppen.

17. Verbindungen der Formel I nach einem der Ansprüche 1-7, die γ-Amino-buttersäure enthalten, und Salze von solchen Verbindungen mit salzbildenden Gruppen.

18. Verbindungen der Formel I nach einem der Ansprüche 1-7, die die Sequenz -Sta-β-Ala-His- enthalten, und Salze von solchen Verbindungen mit salzbildenden Gruppen.

19. Verbindungen der Formel I nach einem der Ansprüche 1-7, die die Sequenz -Sta-Sta- aufweisen, und Salze von solchen Verbindungen mit salzbildenden Gruppen.

20. Verbindungen der Formel I nach einem der Ansprüche 1-19, die mindestens eine Hydroxycarbonsäure an Stelle einer Aminosäure aufweisen, und Salze von solchen Verbindungen mit salzbildenden Gruppen.

21. Verbindungen der Formel I nach Anspruch 20, die ein Molekül L-Milchsäure enthalten, und Salze von solchen Verbindungen mit salzbildenden Gruppen.

22. Verbindungen der Formel I nach Anspruch 20, die ein Molekül Glykolsäure enthalten, und Salze von solchen Verbindungen mit salzbildenden Gruppen.

23. Verbindungen der Formel I nach einem der Ansprüche 20-22, die die Hydroxysäure im Rest $R^4$ im Anschluss an die γ-Aminosäure enthalten, und Salze von solchen Verbindungen mit salzbildenden Gruppen.

24. Verbindungen der Formel I nach einem der Ansprüche 1-3, 7 und 10-23, worin $R^3$ für verestertes Hydroxy mit höchstens 18 C-Atomen steht, und Salze von solchen Verbindungen mit salzbildenden Gruppen.

25. Verbindungen der Formel I nach einem der Ansprüche 1-7 und 10-24, worin $R^1$ für den Rest H-Arg-Arg- steht, und Salze von solchen Verbindungen mit salzbildenden Gruppen.

26. Verbindungen der Formel I nach einem der Ansprüche 1-25, worin freie Aminogruppen in geschützter Form vorliegen, und Salze von solchen Verbindungen mit salzbildenden Gruppen.

27. Verbindungen der Formel I nach einem der Ansprüche 1-26, worin freie Carboxylgruppen in geschützter Form vorliegen, und Salze von solchen Verbindungen mit salzbildenden Gruppen.

28. Verbindungen der Formel I nach Anspruch 27, worin freie Carboxylgruppen durch Schutzgruppen mit mehr als 4 C-Atomen geschützt sind, und Salze von solchen Verbindungen mit salzbildenden Gruppen.

29. Verbindungen der Formel I nach einem der Ansprüche 1-28, worin die in Formel I verwendeten Abkürzungen -Pro-, -Phe- und -His- für die Reste der entsprechenden (L)-Aminosäuren stehen, und Salze von solchen Verbindungen mit salzbildenden Gruppen.

30. Die Verbindung H-Pro-His-Pro-Phe-His-Sta-Val-Tyr-Lys-OH oder H-Pro-His-Pro-Phe-His-Sta-Ile-His-Lys-OH und ihre pharmazeutisch verwendbaren Salze nach Anspruch 1.

31. Die Verbindung Z-Arg-Arg-Pro-Phe-His-Sta-Ile-His-Lys(Boc)-OMe und ihre pharmazeutisch verwendbaren Salze nach Anspruch 1.

32. Eine Verbindung, ausgewählt aus der Gruppe Z-Arg-Arg-Pro-Phe-His-Sta-Ile-His-Lys-OMe, H-Arg-Arg-Pro-Phe-His-Sta-Ile-His-Lys-OMe und H-Arg-Arg-Pro-Phe-His-Sta-Ile-His-Lys-OH, und ihre pharmazeutisch verwendbaren Salze nach Anspruch 1.

33. Die Verbindung H-Arg-Arg-Pro-Phe-His-Sta-Ile-His-Lys(Boc)-OMe und ihre pharmazeutisch verwendbaren Salze nach Anspruch 1.

34. Eine Verbindung, ausgewählt aus der Gruppe Z-Pro-Phe-His-Sta-Ala-Sta-OMe, H-Pro-Phe-His-Sta-Ala-Sta-OMe und H-Pro-Phe-His-Sta-Ala-Sta-$NH_2$, und ihre pharmazeutisch verwendbaren Salze nach Anspruch 1.

35. Eine Verbindung, ausgewählt aus der Gruppe Z-Arg-Arg-Pro-Phe-His-Sta-Sar-His-Lys(Boc)-OMe, H-Arg-Arg-Pro-Phe-His-Sta-Sar-His-Lys(Boc)-OMe, H-Arg-Arg-Pro-Phe-His-Sta-Sar-His-Lys-OMe und H-Arg-Arg-Pro-Phe-His-Sta-Sar-His-Lys-OH, und ihre pharmazeutisch verwendbaren Salze nach Anspruch 1.

36. Eine Verbindung, ausgewählt aus der Gruppe Z-Arg-Arg-Pro-Phe-His-Sta-ß-Ala-His-$NH_2$ und H-Arg-Arg-Pro-Phe-His-Sta-ß-Ala-His-$NH_2$, und ihre pharmazeutisch verwendbaren Salze nach Anspruch 1.

37. Eine Verbindung, ausgewählt aus der Gruppe Z-Arg-Arg-Pro-Phe-His-Sta-Lac-His-$NH_2$ und H-Arg-Arg-Pro-Phe-His-Sta-Lac-His-$NH_2$, und ihre pharmazeutisch verwendbaren Salze nach Anspruch 1.

38. Pharmazeutische Präparate, die eine wirksame Menge einer Verbindung nach einem der Ansprüche 1-37 zusammen mit einer signifikanten Menge eines pharmazeutischen Trägermaterials enthalten.

39. Eine Verbindung nach einem der Ansprüche 1-37 zur Verwendung in einem Verfahren zur Behandlung des menschlichen oder tierischen Körpers.

40. Verwendung einer Verbindung nach einem der Ansprüche 1-37 zur Herstellung von pharmazeutischen Präparaten.

41. Verwendung einer Verbindung nach einem der Ansprüche 1-37 zur Hemmung der Wirkung des Enzyms Renin.

42. Verfahren zur Herstellung einer Verbindung der Formel I

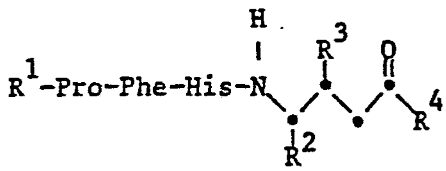

(I)

worin $R^1$ Wasserstoff oder Acyl, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, und eines Salzes einer solchen Verbindung mit mindestens einer salzbildenden Gruppe, dadurch gekennzeichnet, dass man

eine Amidbindung einer Verbindung der Formel I durch Umsetzung eines entsprechenden Bruchstücks mit einer freien Carboxylgruppe oder eines reaktionsfähigen Säurederivats davon mit einem komplementierenden Bruchstück mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der obengenannten Reaktion teilnehmenden Gruppen gegebenenfalls in geschützter Form vorliegen, herstellt und, wenn nötig, vorhandene Schutzgruppen abspaltet und/oder, wenn erwünscht, eine Verbindung der Formel I mit mindestens einer salzbildenden Gruppe in ihr Salz oder ein Salz in die freie Verbindung oder in ein anderes Salz überführt und/oder gegebenenfalls erhaltene Stereoisomerengemische auftrennt und/oder eine Verbindung der Formel I epimerisiert.

43. Verfahren zur Herstellung einer Verbindung der Formel I

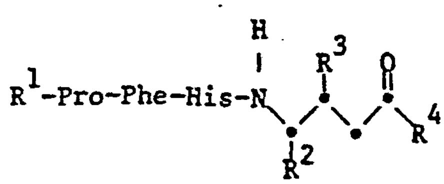

(I)

worin $R^1$ Wasserstoff oder Acyl, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, und eines Salzes einer solchen Verbindung mit mindestens einer salzbildenden Gruppe, dadurch gekennzeichnet, dass man

a) zur Herstellung von Verbindungen der Formel I, worin $R^3$ freies Hydroxy bedeutet, die Ketogruppe in einer Verbindung der Formel II,

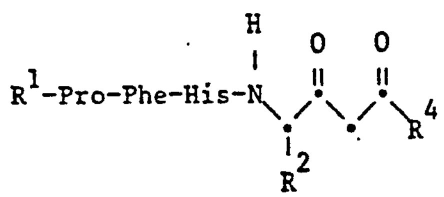

(II)

worin die Substituenten die obengenannten Bedeutungen haben, mit der Massgabe, dass in der Verbindung vorhandene freie funktionelle Gruppen mit Ausnahme der an der Reaktion teilnehmenden Ketogruppe gegebenenfalls in geschützter Form vorliegen, durch Umsetzung mit einem regioselektiven Reduktionsmittel zu einer Hydroxygruppe reduziert und, wenn nötig, vorhandene Schutzgruppen abspaltet oder

b) an die C=C-Doppelbindung einer Verbindung der Formel III,

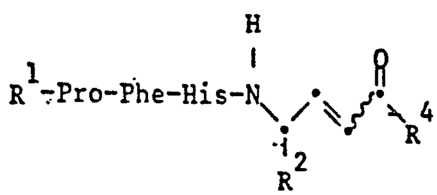

(III)

worin die Substituenten die obengenannten Bedeutungen haben, mit der Massgabe, dass in der Verbindung vorhandene freie funktionelle Gruppen mit Ausnahme der an der Reaktion teilnehmenden Doppelbindung gegebenenfalls in geschützter Form vorliegen, regioselektiv eine Verbindung der Formel $R^3$-H, worin $R^3$ die obengenannte Bedeutung hat, addiert und, wenn nötig, vorhandene Schutzgruppen abspaltet oder

c) in einer Verbindung der Formel IV,

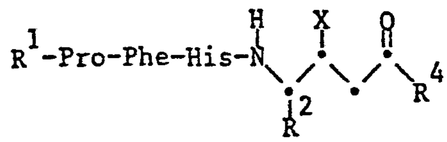

(IV)

worin X für eine gute Abgangsgruppe steht und die übrigen Substituenten die obengenannten Bedeutungen haben mit der Massgabe, dass in der Verbindung vorhandene freie funktionelle Gruppen mit Ausnahme der an der Reaktion teilnehmenden Gruppe gegebenenfalls in geschützter Form vorliegen, den Substituenten X mit einem, den Substituenten $R^3$ in nucleophiler Form bereitstellenden Reagenz gegen $R^3$ austauscht und, wenn nötig, vorhandene Schutzgruppen abspaltet oder

d) zur Herstellung einer Verbindung der Formel I, worin $R^4$ für freies oder substituiertes Amino steht, die Cyanogruppe in einer Verbindung der Formel V,

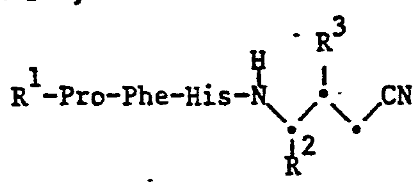

(V)

worin die Substituenten die obengenannten Bedeutungen haben, in eine gegebenenfalls N-substituierte Amidgruppe überführt oder

e) zur Herstellung einer Verbindung der Formel I, worin $R^3$ für freies Hydroxy steht, ein Epoxid der Formel VI,

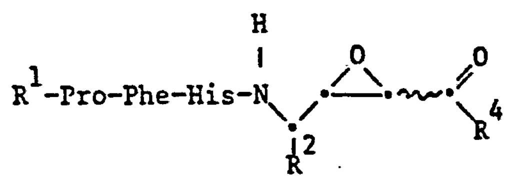

(VI)

worin die Substituenten die obengenannten Bedeutungen haben, mit der Massgabe, dass in der Verbindung vorhandene freie funktionelle Gruppen mit Ausnahme der an der Reaktion teilnehmenden Epoxygruppe gegebenenfalls in geschützter Form vorliegen, mit einem regioselektiven Reduktionsmittel zum entsprechenden Alkohol reduziert und, wenn nötig, vorhandene Schutzgruppen abspaltet oder

f) eine Verbindung der Formel VII,

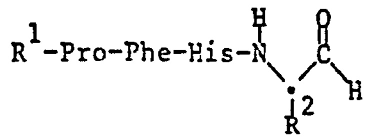

(VII)

worin die Substituenten die obengenannten Bedeutungen haben, mit der Massgabe, dass in der Verbindung vorhandene freie funktionelle Gruppen mit Ausnahme der an der Reaktion teilnehmenden Aldehydgruppe gegebenenfalls in geschützter Form vorliegen, mit einer Verbindung der Formel VIII,

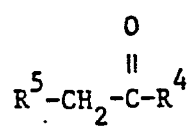

(VIII),

worin $R^5$ für Halogen mit einem Atomgewicht zwischen 35 und 127 steht und $R^4$ die obengenannte Bedeutung hat, mit der Massgabe, dass in einer Verbindung der Formel VIII vorhandene freie funktionelle Gruppen mit Ausnahme der an der Reaktion beteiligten Gruppe gegebenenfalls in geschützter Form vorliegen, nach Aktivierung mit Zink (analog einer Reformatsky-Reaktion) umsetzt und, wenn nötig, vorhandene Schutzgruppen abspaltet oder

g) in einer Verbindung der Formel I, worin die Substituenten die obengenannten Bedeutungen haben, mit der Massgabe, dass in einer Verbindung der Formel I mindestens eine freie Amino-, Hydroxy-, Mercapto- oder Carboxygruppe vorhanden ist und die übrigen funktionellen Gruppen gegebenenfalls in geschützter Form vorliegen, mindestens eine freie Amino-, Hydroxy- oder Mercaptogruppe mit einer den einzuführenden Rest enthaltenden Carbonsäure oder einem reaktionsfähigen Derivat davon acyliert oder mindestens eine freie Carboxygruppe oder ein reaktionsfähiges Derivat davon verestert und, wenn nötig, vorhandene Schutzgruppen abspaltet oder

h) in einer Verbindung der Formel I, worin die Substituenten die obengenannten Bedeutungen haben, mit der Massgabe, dass in einer Verbindung der Formel I mindestens eine freie Amino-, Hydroxy- oder Mercaptogruppe vorhanden ist und die übrigen funktionellen Gruppen gegebenenfalls in geschützter Form vorliegen, mindestens eine freie Aminogruppe alkyliert, oder eine freie Hydroxy- oder Mercaptogruppe verethert und, wenn nötig, vorhandene Schutzgruppen abspaltet oder

i) zur Herstellung einer Verbindung der Formel I, worin $R^4$ für den N-terminalen Rest einer gegebenenfalls entsprechend substituierten Aminosäure steht, ein Lacton der Formel IX,

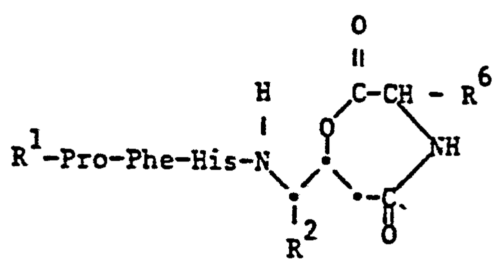

(IX),

worin $R^1$ und $R^2$ die obengenannten Bedeutungen haben und $R^6$ den entsprechenden Rest in einer in der Natur vorkommenden Aminosäure der Formel X oder ihrem (D)-Isomeren

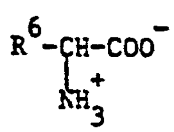

(X)

die über eine Carboxyl- oder Aminogruppe im Rest $R^6$ amidisch mit einer weiteren in der Natur vorkommenden Aminosäure oder mit einem aus in der Natur vorkommenden Aminosäuren oder ihren (D)-Isomeren aufgebauten Peptid mit 2-5 Aminosäuren verbunden sein kann, wobei freie funktionelle Gruppen in diesen Aminosäureresten gegebenenfalls in abgewandelter Form vorliegen, bedeutet, aufspaltet oder

j) zur Herstellung einer Verbindung mit mindestens einer freien funktionellen Gruppe in einer Verbindung der Formel I, worin die Substituenten die obengenannten Bedeutungen haben, mit der Massgabe, dass in einer Verbindung der Formel I mindestens eine funktionelle Gruppe durch eine leicht abspaltbare Schutzgruppe geschützt ist, die vorhandenen Schutzgruppen, gegebenenfalls stufenweise, abspaltet, und, wenn erwünscht, nach Ausführung eines der vorstehend genannten Verfahren a-j) oder eines beliebigen anderen Verfahrens zur Herstellung einer Verbindung der Formel I eine erhaltene Verbindung der Formel I mit mindestens einer salzbildenden Gruppe in ihr Salz oder ein erhaltenes Salz in die freie Verbindung oder in ein anderes Salz überführt und/oder gegebenenfalls erhaltene Stereoisomerengemische auftrennt und/oder eine erhaltene Verbindung der Formel I epimerisiert.

44. Die nach einem der Verfahren des Anspruchs 42 oder 43 erhältlichen Verbindungen und ihre Salze.

FO 7.4/VBU/am*

Patentansprüche für den Vertragsstaat AT:

1. Verfahren zur Herstellung einer Verbindung der Formel I

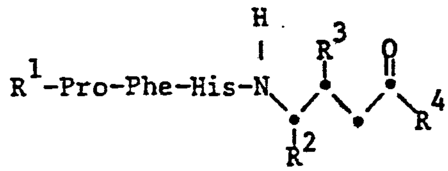 (I)

worin $R^1$ Wasserstoff oder Acyl, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, und eines Salzes einer solchen Verbindung mit mindestens einer salzbildenden Gruppe, dadurch gekennzeichnet, dass man

a) eine Amidbindung einer Verbindung der Formel I durch Umsetzung eines entsprechenden Bruchstücks mit einer freien Carboxylgruppe oder eines reaktionsfähigen Säurederivats davon mit einem komplementierenden Bruchstück mit einer freien Aminogruppe oder einem reaktionsfähigen Derivat davon mit aktivierter Aminogruppe, wobei in den Reaktionskomponenten vorhandene freie funktionelle Gruppen mit Ausnahme der an der obengenannten Reaktion teilnehmenden Gruppen gegebenenfalls in geschützter Form vorliegen, herstellt und, wenn nötig, vorhandene Schutzgruppen abspaltet oder

b) zur Herstellung von Verbindungen der Formel I, worin $R^3$ freies Hydroxy bedeutet, die Ketogruppe in einer Verbindung der Formel II,

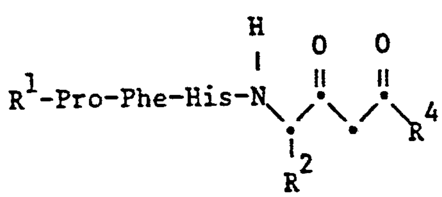 (II)

worin die Substituenten die obengenannten Bedeutungen haben, mit der Massgabe, dass in der Verbindung vorhandene freie funktionelle

Gruppen mit Ausnahme der an der Reaktion teilnehmenden Ketogruppe gegebenenfalls in geschützter Form vorliegen, durch Umsetzung mit einem regioselektiven Reduktionsmittel zu einer Hydroxygruppe reduziert und, wenn nötig, vorhandene Schutzgruppen abspaltet oder

c) an die C=C-Doppelbindung einer Verbindung der Formel III,

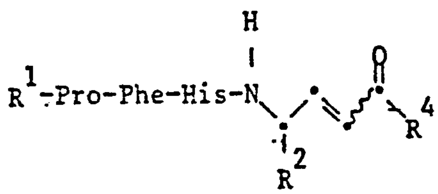

(III)

worin die Substituenten die obengenannten Bedeutungen haben, mit der Massgabe, dass in der Verbindung vorhandene freie funktionelle Gruppen mit Ausnahme der an der Reaktion teilnehmenden Doppelbindung gegebenenfalls in geschützter Form vorliegen, regioselektiv eine Verbindung der Formel $R^3$-H, worin $R^3$ die obengenannte Bedeutung hat, addiert und, wenn nötig, vorhandene Schutzgruppen abspaltet oder

d) in einer Verbindung der Formel IV,

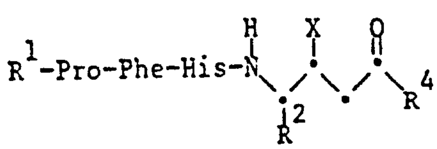

(IV)

worin X für eine gute Abgangsgruppe steht und die übrigen Substituenten die obengenannten Bedeutungen haben mit der Massgabe, dass in der Verbindung vorhandene freie funktionelle Gruppen mit Ausnahme der an der Reaktion teilnehmenden Gruppe gegebenenfalls in geschützter Form vorliegen, den Substituenten X mit einem, den Substituenten $R^3$ in nucleophiler Form bereitstellenden Reagenz gegen $R^3$ austauscht und, wenn nötig, vorhandene Schutzgruppen abspaltet oder

e) zur Herstellung einer Verbindung der Formel I, worin $R^4$ für freies oder substituiertes Amino steht, die Cyanogruppe in einer Verbindung der Formel V,

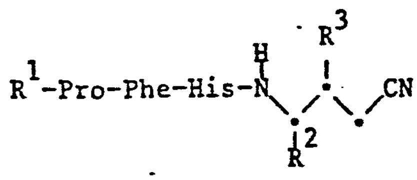

(V)

worin die Substituenten die obengenannten Bedeutungen haben, in eine gegebenenfalls N-substituierte Amidgruppe überführt oder

f) zur Herstellung einer Verbindung der Formel I, worin $R^3$ für freies Hydroxy steht, ein Epoxid der Formel VI,

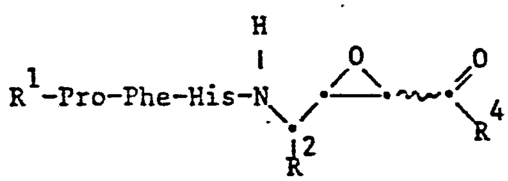

(VI)

worin die Substituenten die obengenannten Bedeutungen haben, mit der Massgabe, dass in der Verbindung vorhandene freie funktionelle Gruppen mit Ausnahme der an der Reaktion teilnehmenden Epoxygruppe gegebenenfalls in geschützter Form vorliegen, mit einem regioselektiven Reduktionsmittel zum entsprechenden Alkohol reduziert und, wenn nötig, vorhandene Schutzgruppen abspaltet oder

g) eine Verbindung der Formel VII,

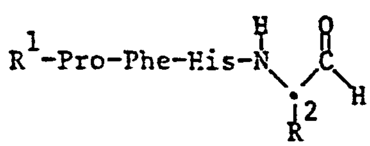

(VII)

worin die Substituenten die obengenannten Bedeutungen haben, mit der Massgabe, dass in der Verbindung vorhandene freie funktionelle Gruppen mit Ausnahme der an der Reaktion teilnehmenden Aldehydgruppe gegebenenfalls in geschützter Form vorliegen, mit einer Verbindung der Formel VIII,

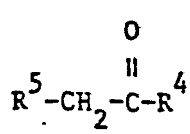

(VIII),

worin $R^5$ für Halogen mit einem Atomgewicht zwischen 35 und 127 steht und $R^4$ die obengenannte Bedeutung hat, mit der Massgabe, dass in einer Verbindung der Formel VIII vorhandene freie funktionelle Gruppen mit Ausnahme der an der Reaktion beteiligten Gruppe gegebenenfalls in geschützter Form vorliegen, nach Aktivierung mit Zink (analog einer Reformatsky-Reaktion) umsetzt und, wenn nötig, vorhandene Schutzgruppen abspaltet oder

h) in einer Verbindung der Formel I, worin die Substituenten die obengenannten Bedeutungen haben, mit der Massgabe, dass in einer Verbindung der Formel I mindestens eine freie Amino-, Hydroxy-, Mercapto- oder Carboxygruppe vorhanden ist und die übrigen funktionellen Gruppen gegebenenfalls in geschützter Form vorliegen, mindestens eine freie Amino-, Hydroxy- oder Mercaptogruppe mit einer den einzuführenden Rest enthaltenden Carbonsäure oder einem reaktionsfähigen Derivat davon acyliert oder mindestens eine freie Carboxygruppe oder ein reaktionsfähiges Derivat davon verestert und, wenn nötig, vorhandene Schutzgruppen abspaltet oder

i) in einer Verbindung der Formel I, worin die Substituenten die obengenannten Bedeutungen haben, mit der Massgabe, dass in einer Verbindung der Formel I mindestens eine freie Amino-, Hydroxy- oder Mercaptogruppe vorhanden ist und die übrigen funktionellen Gruppen gegebenenfalls in geschützter Form vorliegen, mindestens eine freie Aminogruppe alkyliert, oder eine freie Hydroxy- oder Mercaptogruppe verethert und, wenn nötig vorhandene Schutzgruppen abspaltet oder

j) zur Herstellung einer Verbindung der Formel I, worin $R^4$ für den N-terminalen Rest einer gegebenenfalls entsprechend substituierten Aminosäure steht, ein Lacton der Formel IX,

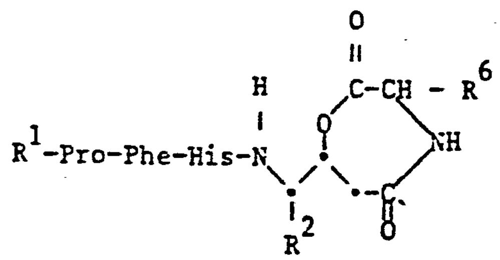

(IX),

worin $R^1$ und $R^2$ die obengenannten Bedeutungen haben und $R^6$ den entsprechenden Rest in einer in der Natur vorkommenden Aminosäure der Formel X oder ihrem (D)-Isomeren

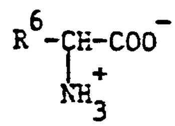

(X)

die über eine Carboxyl- oder Aminogruppe im Rest $R^6$ amidisch mit einer weiteren in der Natur vorkommenden Aminosäure oder mit einem aus in der Natur vorkommenden Aminosäuren oder ihren (D)-Isomeren aufgebauten Peptid mit 2-5 Aminosäuren verbunden sein kann, wobei freie funktionelle Gruppen in diesen Aminosäureresten gegebenenfalls in abgewandelter Form vorliegen, bedeutet, aufspaltet oder

k) zur Herstellung einer Verbindung mit mindestens einer freien funktionellen Gruppe in einer Verbindung der Formel I, worin die Substituenten die obengenannten Bedeutungen haben, mit der Massgabe, dass in einer Verbindung der Formel I mindestens eine funktionelle Gruppe durch eine leicht abspaltbare Schutzgruppe geschützt ist, die vorhandenen Schutzgruppen, gegebenenfalls stufenweise, abspaltet, und, wenn erwünscht, nach Ausführung eines der vorstehend genannten Verfahren a-k) oder eines beliebigen anderen Verfahrens zur Herstellung einer Verbindung der Formel I eine erhaltene Verbindung der Formel I mit mindestens einer salzbildenden Gruppe in ihr Salz oder ein erhaltenes Salz in die freie Verbindung oder in ein anderes Salz überführt und/oder gegebenenfalls erhaltene Stereoisomerengemische auftrennt und/oder eine erhaltene Verbindung der Formel I epimerisiert.

2. Verfahren nach Anspruch 1, dadurch gekennzeichnet, dass man die Ausgangsstoffe so wählt, dass man eine Verbindung der Formel I, worin $R^1$ Wasserstoff, einen aliphatischen, aromatischen oder aromatisch-aliphatischen Acylrest mit bis zu 18 C-Atomen, einen heterocyclischen oder heterocyclisch-aliphatischen Acylrest mit jeweils fünf oder sechs Ringgliedern und ein oder zwei Stickstoffatomen im gegebenenfalls benzoannellierten heterocyclischen Ring und insgesamt nicht mehr als 18 C-Atomen oder den Acylrest eines Oligopeptids mit mehr als 18 und höchstens 60 C-Atomen, das aus höchstens 6 in der Natur vorkommenden Aminosäuren und/oder ihren (D)-Isomeren aufgebaut ist, wobei in solchen Aminosäuren vorhandene freie funktionelle Gruppen gegegebenenfalls in geschützter Form vorliegen, $R^2$ Alkyl oder Aralkyl mit jeweils nicht mehr als 18 C-Atomen, $R^3$ freies oder verestertes Hydroxy mit höchstens 18 C-Atomen und $R^4$ freies oder durch Arylniederalkyl mit höchstens 18 C-Atomen oder Niederalkyl substituiertes Amino, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, oder freies oder verethertes Hydroxy mit höchstens 12 C-Atomen bedeuten, oder ein Salz einer solchen Verbindung mit mindestens einer salzbildenden Gruppe herstellt.

3. Verfahren nach Anspruch 1, dadurch gekennzeichnet, dass man die Ausgangsstoffe so wählt, dass man eine Verbindung der Formel I, worin $R^1$ Wasserstoff, den Rest einer in der Natur vorkommenden Aminosäure oder ihres (D)-Isomeren, oder den Rest eines aus höchstens 6 in der Natur vorkommenden Aminosäuren und/oder ihren (D)-Isomeren aufgebauten Peptids, wobei in diesen Resten vorhandene freie funktionelle Gruppen gegebenenfalls in geschützter Form vorliegen, $R^2$ Niederalkyl oder Phenylniederalkyl, $R^3$ freies Hydroxy und $R^4$ freies Amino oder

Amino, das durch gegebenenfalls durch Phenyl substituiertes Niederalkyl substituiert ist, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, freies Hydroxy oder Niederalkoxy bedeuten, oder ein Salz einer solchen Verbindung mit mindestens einer salzbildenden Gruppe herstellt.

4. Verfahren nach Anspruch 1, dadurch gekennzeichnet, dass man die Ausgangsstoffe so wählt, dass man eine Verbindung der Formel I, worin $R^1$ Wasserstoff, den Acylrest einer in der Natur vorkommenden Aminosäure, den Acylrest eines aus in der Natur vorkommenden Aminosäuren aufgebauten Dipeptids oder Niederalkanoyl, $R^2$ verzweigtes Niederalkyl, $R^3$ freies Hydroxy und $R^4$ freies oder durch Niederalkyl oder Benzyl substituiertes Amino, oder den N-terminalen Rest einer in der Natur vorkommenden Aminosäure oder eines aus solchen Aminosäuren aufgebauten Di- oder Tripeptids bedeuten, oder ein Salz einer solchen Verbindung mit mindestens einer salzbildenden Gruppe herstellt.

5. Verfahren nach Anspruch 1, dadurch gekennzeichnet, dass man die Ausgangsstoffe so wählt, dass man eine Verbindung der Formel I, worin $R^1$ den Acylrest eines aus in der Natur vorkommenden Aminosäuren aufgebauten Dipeptids, $R^2$ 2-Methyl-propyl, $R^3$ freies Hydroxy und $R^4$ den N-terminalen Rest eines aus in der Natur vorkommenden Aminosäuren aufgebauten Di- oder Tripeptids bedeuten, mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, oder ein pharmazeutisch verwendbares Salz einer solchen Verbindung mit mindestens einer salzbildenden Gruppe herstellt.

6. Verfahren nach einem der Ansprüche 1-5, dadurch gekennzeichnet, dass man die Ausgangsstoffe so wählt, dass man eine Verbindung der Formel I, worin in den Resten $R^1$ und $R^4$ vorkommende Aminosäurereste

sich von jenen 20 Aminosäuren in ihrer natürlichen Konfiguration ableiten, die regelmässig in Proteinen vorkommen, oder ein pharmazeutisch verwendbares Salz einer solchen Verbindung mit mindestens einer salzbildenden Gruppe herstellt.

7. Verfahren nach Anspruch 1, dadurch gekennzeichnet, dass man die Ausgangsstoffe so wählt, dass man eine Verbindung der Formel I, worin $R^1$ den Rest H-Pro-His-, $R^2$ 2-Methyl-propyl, $R^3$ freies Hydroxy und $R^4$ den Rest -Val-Tyr-Lys-OH, -Ile-His-Lys-OH, -Ile-His-Ser-OH, -Val-Tyr-Ser-OH, -Val-Tyr-OH, -Ile-His-OH, -Val-Tyr-$NH_2$, -Ile-His-$NH_2$ oder -Ala-Sta-OH bedeuten, oder ein pharmazeutisch verwendbares Salz einer solchen Verbindung mit mindestens einer salzbildenden Gruppe herstellt.

8. Verfahren nach Anspruch 1, dadurch gekennzeichnet, dass man die Ausgangsstoffe so wählt, dass man eine Verbindung der Formel I, worin $R^1$ den Rest H-Ile-His-, $R^2$ 2-Methyl-propyl, $R^3$ freies Hydroxy und $R^4$ den Rest -Val-Tyr-Lys-OH, -Ile-His-Lys-OH, -Ile-His-Ser-OH, -Val-Tyr-Ser-OH, -Val-Tyr-OH, -Ile-His-OH, -Val-Tyr-$NH_2$, -Ile-His-$NH_2$ oder -Ala-Sta-OH bedeuten, oder ein pharmazeutisch verwendbares Salz einer solchen Verbindung mit mindestens einer salzbildenden Gruppe herstellt.

9. Verfahren nach Anspruch 1, dadurch gekennzeichnet, dass man die Ausgangsstoffe so wählt, dass man eine Verbindung der Formel I, worin $R^1$ den Rest eines aus höchstens 6 in der Natur vorkommenden Aminosäuren und/oder ihren (D)-Isomeren aufgebauten Peptids, wobei in diesen Resten vorhandene freie funktionelle Gruppen gegebenenfalls in geschützter Form vorliegen, $R^2$ Niederalkyl oder Phenylniederalkyl, $R^3$ freies Hydroxy und $R^4$ freies Amino oder Amino, das durch gegebenenfalls durch Phenyl substituiertes Niederalkyl substituiert ist, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter

Form vorliegen, freies Hydroxy oder Niederalkoxy bedeuten, und Salze von solchen Verbindungen mit salzbildenden Gruppen mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, oder ein Salz einer solchen Verbindung mit mindestens einer salzbildenden Gruppe herstellt.

10. Verfahren nach Anspruch 1, dadurch gekennzeichnet, dass man die Ausgangsstoffe so wählt, dass man eine Verbindung der Formel I, worin $R^1$ den Acylrest eines aus in der Natur vorkommenden Aminosäuren aufgebauten Dipeptids oder Niederalkanoyl, $R^2$ verzweigtes Niederalkyl, $R^3$ freies Hydroxy und $R^4$ freies oder durch Niederalkyl oder Benzyl substituiertes Amino, oder den N-terminalen Rest einer in der Natur vorkommenden Aminosäure oder eines aus solchen Aminosäuren aufgebauten Di- oder Tripeptids bedeuten, mit Ausnahme der Verbindungen H-Pro-His-Pro-Phe-His-Sta-Leu-Phe-$NH_2$, N-Isovaleryl-Pro-His-Pro-Phe-His-Sta-Leu-Phe-$NH_2$ und N-tert.Butyloxycarbonyl-Pro-His-Pro-Phe-His-Sta-Leu-Phe-$NH_2$, oder ein Salz einer solchen Verbindung mit mindestens einer salzbildenden Gruppe herstellt.

11. Verfahren nach Anspruch 1, dadurch gekennzeichnet, dass man die Ausgangsstoffe so wählt, dass man eine Verbindung der Formel I, worin $R^1$ den Acylrest eines Dipeptids, bestehend aus zwei in der Natur vorkommenden (L)-Aminosäuren oder deren (D)-Isomeren, den Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder einen anderen aliphatischen, aromatischen oder aromatisch-aliphatischen Acylrest mit bis zu 18 C-Atomen, der verschieden ist von dem Acylrest von N-unsubstituiertem oder N-substituiertem (L)- oder (D)-Histidin oder Sarkosin, $R^2$ Alkyl oder Aralkyl, $R^3$ freies oder funktionell abgewandeltes Hydroxy, $R^4$ freies oder substituiertes Amino oder freies oder verethertes Hydroxy und -Pro-, -Phe- sowie -His- die bivalenten Reste der Aminosäuren Prolin, Phenylalanin beziehungsweise Histidin oder ihrer (D)-Isomeren bedeuten, mit Ausnahme der Verbindungen (H, Boc oder N-Isovaleryl)-Pro-His-Pro-Phe-His-Sta-Leu-Phe-$NH_2$ und (H, Boc, N-Acetyl oder

N-Phenoxyacetyl)-Pro-Phe-His-Sta-Leu-Phe-$NH_2$, oder ein Salz einer solchen Verbindung mit mindestens einer salzbildenden Gruppe herstellt.

12. Verfahren nach Anspruch 1, dadurch gekennzeichnet, dass man die Ausgangsstoffe so wählt, dass man eine Verbindung der Formel I, worin $R^1$ den Acylrest eines Dipeptids, bestehend aus zwei in der Natur vorkommenden (L)-Aminosäuren oder deren (D)-Isomeren, den Acylrest eines Oligopeptids mit 3 bis 6 in der Natur vorkommenden Aminosäuren und höchstens 60 C-Atomen, wobei freie funktionelle Gruppen in diesen Aminosäureresten gegebenenfalls in geschützter Form vorliegen, oder einen anderen aliphatischen, aromatischen oder aromatisch-aliphatischen Acylrest mit bis zu 18 C-Atomen, der verschieden ist von dem Acylrest von N-unsubstituiertem oder N-substituiertem (L)- oder (D)-Histidin oder Sarkosin, $R^2$ Alkyl oder Aralkyl mit jeweils nicht mehr als 18 C-Atomen, $R^3$ freies oder verestertes Hydroxy mit höchstens 18 C-Atomen und $R^4$ freies oder durch Arylniederalkyl mit höchstens 18 C-Atomen oder Niederalkyl substituiertes Amino, den N-terminalen Rest einer in der Natur vorkommenden Aminosäure oder ihres (D)-Isomeren oder eines aus in der Natur vorkommenden Aminosäuren und/oder ihren (D)-Isomeren aufgebauten Peptids mit 2-6 Aminosäuren, wobei in solchen Aminosäuren vorhandene freie funktionelle Gruppen gegebenenfalls in geschützter Form vorliegen, oder freies oder verethertes Hydroxy mit höchstens 12 C-Atomen bedeuten, mit Ausnahme der Verbindungen (H, Boc oder N-Isovaleryl)-Pro-His-Pro-Phe-His-Sta-Leu-Phe-$NH_2$ und (H, Boc, N-Acetyl oder N-Phenoxyacetyl)-Pro-Phe-His-Sta-Leu-Phe-$NH_2$, oder ein Salz einer solchen Verbindung mit mindestens einer salzbildenden Gruppe herstellt.

13. Verfahren nach einem der Ansprüche 1-6 und 8-12, dadurch gekennzeichnet, dass man die Ausgangsstoffe so wählt, dass man eine Verbindung der Formel I, die mindestens zwei Aminosäuren enthält, die von α-Aminosäuren verschieden sind, oder ein Salz einer solchen Verbindung mit mindestens einer salzbildenden Gruppe herstellt.

14. Verfahren nach Anspruch 13, dadurch gekennzeichnet, dass man die Ausgangsstoffe so wählt, dass man eine Verbindung der Formel I, die zwei Statinmoleküle enthält, oder ein Salz einer solchen Verbindung mit mindestens einer salzbildenden Gruppe herstellt.

15. Verfahren nach Anspruch 14, dadurch gekennzeichnet, dass man die Ausgangsstoffe so wählt, dass man eine Verbindung der Formel I, die die Sequenz -Sta-Ala-Sta- aufweist, oder ein Salz einer solchen Verbindung mit mindestens einer salzbildenden Gruppe herstellt.

16. Verfahren nach Anspruch 14, dadurch gekennzeichnet, dass man die Ausgangsstoffe so wählt, dass man eine Verbindung der Formel I, die die Sequenz -Sta-Gly-Sta- aufweist, oder ein Salz einer solchen Verbindung mit mindestens einer salzbildenden Gruppe herstellt.

17. Verfahren nach Anspruch 14, dadurch gekennzeichnet, dass man die Ausgangsstoffe so wählt, dass man eine Verbindung der Formel I, die die Sequenz -Sta-Ile-Sta- aufweist, oder ein Salz einer solchen Verbindung mit mindestens einer salzbildenden Gruppe herstellt.

18. Verfahren nach Anspruch 14, dadurch gekennzeichnet, dass man die Ausgangsstoffe so wählt, dass man eine Verbindung der Formel I, die die Sequenz -Sta-Sar-Sta- aufweist, oder ein Salz einer solchen Verbindung mit mindestens einer salzbildenden Gruppe herstellt.

19. Verfahren nach Anspruch 13, dadurch gekennzeichnet, dass man die Ausgangsstoffe so wählt, dass man eine Verbindung der Formel I, die ß-Alanin enthält, oder ein Salz einer solchen Verbindung mit mindestens einer salzbildenden Gruppe herstellt.

20. Verfahren nach Anspruch 13, dadurch gekennzeichnet, dass man die Ausgangsstoffe so wählt, dass man eine Verbindung der Formel I, die γ-Amino-buttersäure enthält, oder ein Salz einer solchen Verbindung mit mindestens einer salzbildenden Gruppe herstellt.

21. Verfahren nach Anspruch 19, dadurch gekennzeichnet, dass man die Ausgangsstoffe so wählt, dass man eine Verbindung der Formel I, die die Sequenz -Sta-ß-Ala-His enthält, oder ein Salz einer solchen Verbindung mit mindestens einer salzbildenden Gruppe herstellt.

22. Verfahren nach Anspruch 14, dadurch gekennzeichnet, dass man die Ausgangsstoffe so wählt, dass man eine Verbindung der Formel I, die die Sequenz -Sta-Sta- aufweist, oder ein Salz einer solchen Verbindung mit mindestens einer salzbildenden Gruppe herstellt.

23. Verfahren nach einem der Ansprüche 1-22, dadurch gekennzeichnet, dass man die Ausgangsstoffe so wählt, dass man eine Verbindung der Formel I, die mindestens eine Hydroxycarbonsäure an Stelle einer Aminosäure aufweist, oder ein Salz einer solchen Verbindung mit mindestens einer salzbildenden Gruppe herstellt.

24. Verfahren nach Anspruch 23, dadurch gekennzeichnet, dass man die Ausgangsstoffe so wählt, dass man eine Verbindung der Formel I, die ein Molekül L-Michsäure enthält, oder ein Salz einer solchen Verbindung mit mindestens einer salzbildenden Gruppe herstellt.

25. Verfahren nach Anspruch 23, dadurch gekennzeichnet, dass man die Ausgangsstoffe so wählt, dass man eine Verbindung der Formel I, die ein Molekül Glykolsäure enthält, oder ein Salz einer solchen Verbindung mit mindestens einer salzbildenden Gruppe herstellt.

26. Verfahren nach einem der Ansprüche 23-25, dadurch gekennzeichnet, dass man die Ausgangsstoffe so wählt, dass man eine Verbindung der Formel I, die die Hydroxysäure im Rest $R^4$ im Anschluss an die γ-Aminsäure enthält, oder ein Salz einer solchen Verbindung mit mindestens einer salzbildenden Gruppe herstellt.

27. Verfahren nach einem der Ansprüche 1, 2, 6 und 13-26, dadurch gekennzeichnet, dass man die Ausgangsstoffe so wählt, dass man eine Verbindung der Formel I, worin $R^3$ für verestertes Hydroxy mit höchstens 18 C-Atomen steht, oder ein Salz einer solchen Verbindung mit mindestens einer salzbildenden Gruppe herstellt.

28. Verfahren nach einem der Ansprüche 1-6 und 9-27, dadurch gekennzeichnet, dass man die Ausgangsstoffe so wählt, dass man eine Verbindung der Formel I, worin $R^1$ für den Rest H-Arg-Arg- steht, oder ein Salz einer solchen Verbindung mit mindestens einer salzbildenden Gruppe herstellt.

29. Verfahren nach einem der Ansprüche 1-28, dadurch gekennzeichnet, dass man die Ausgangsstoffe so wählt, dass man eine Verbindung der Formel I, worin freie Aminogruppen in geschützter Form vorliegen, oder ein Salz einer solchen Verbindung mit mindestens einer salzbildenden Gruppe herstellt.

30. Verfahren nach einem der Ansprüche 1-29, dadurch gekennzeichnet, dass man die Ausgangsstoffe so wählt, dass man eine Verbindung der Formel I, worin freie Carboxylgruppen in geschützter Form vorliegen, oder ein Salz einer solchen Verbindung mit mindestens einer salzbildenden Gruppe herstellt.

31. Verfahren nach Anspruch 30, dadurch gekennzeichnet, dass man die Ausgangsstoffe so wählt, dass man eine Verbindung der Formel I, worin freie Carboxylgruppen durch Schutzgruppen mit mehr als 4 C-Atomen geschützt sind, oder ein Salz einer solchen Verbindung mit mindestens einer salzbildenden Gruppe herstellt.

32. Verfahren nach einem der Ansprüche 1-31, dadurch gekennzeichnet, dass man die Ausgangsstoffe so wählt, dass man eine Verbindung der Formel I, worin die in Formel I verwendeten Abkürzungen -Pro-, -Phe- und -His- für die Reste der entsprechenden (L)-Aminosäuren stehen, oder ein Salz einer solchen Verbindung mit mindestens einer salzbildenden Gruppe herstellt.

33. Verfahren nach Anspruch 1, dadurch gekennzeichnet, dass man die Ausgangsstoffe so wählt, dass man die Verbindung H-Pro-His-Pro-Phe-His-Sta-Val-Tyr-Lys-OH oder H-Pro-His-Pro-Phe-His-Sta-Ile-His-Lys-OH oder ein pharmazeutisch verwendbares Salz einer solchen Verbindung mit mindestens einer salzbildenden Gruppe herstellt.

34. Verfahren nach Anspruch 1, dadurch gekennzeichnet, dass man die Ausgangsstoffe so wählt, dass man die Verbindung Z-Arg-Arg-Pro-Phe-His-Sta-Ile-His-Lys(Boc)-OMe oder ein pharmazeutisch verwendbares Salz davon herstellt.

35. Verfahren nach Anspruch 1, dadurch gekennzeichnet, dass man die Ausgangsstoffe so wählt, dass man eine Verbindung der Formel I, ausgewählt aus der Gruppe Z-Arg-Arg-Pro-Phe-His-Sta-Ile-His-Lys-OMe, H-Arg-Arg-Pro-Phe-His-Sta-Ile-His-Lys-OMe und H-Arg-Arg-Pro-Phe-His-Sta-Ile-His-Lys-OH, oder ein pharmazeutisch verwendbares Salz einer solchen Verbindung mit mindestens einer salzbildenden Gruppe herstellt.

36. Verfahren nach Anspruch 1, dadurch gekennzeichnet, dass man die Ausgangsstoffe so wählt, dass man die Verbindung H-Arg-Arg-Pro-Phe-His-Sta-Ile-His-Lys(Boc)-OMe oder ein pharmazeutisch verwendbares Salz davon herstellt.

37. Verfahren nach Anspruch 1, dadurch gekennzeichnet, dass man die Ausgangsstoffe so wählt, dass man eine Verbindung der Formel I, ausgewählt aus der Gruppe Z-Pro-Phe-His-Sta-Ala-Sta-OMe, H-Pro-Phe-His-Sta-Ala-Sta-OMe und H-Pro-Phe-His-Sta-Ala-Sta-$NH_2$, oder ein pharmazeutisch verwendbares Salz einer solchen Verbindung mit mindestens einer salzbildenden Gruppe herstellt.

38. Verfahren nach Anspruch 1, dadurch gekennzeichnet, dass man die Ausgangsstoffe so wählt, dass man eine Verbindung der Formel I, ausgewählt aus der Gruppe Z-Arg-Arg-Pro-Phe-His-Sta-Sar-His-Lys(Boc)-OMe, H-Arg-Arg-Pro-Phe-His-Sta-Sar-His-Lys(Boc)-OMe, H-Arg-Arg-Pro-Phe-His-Sta-Sar-His-Lys-OMe und H-Arg-Arg-Pro-Phe-His-Sta-Sar-His-Lys-OH, oder ein pharmazeutisch verwendbares Salz einer solchen Verbindung mit mindestens einer salzbildenden Gruppe herstellt.

39. Verfahren nach Anspruch 1, dadurch gekennzeichnet, dass man die Ausgangsstoffe so wählt, dass man eine Verbindung der Formel I, ausgewählt aus der Gruppe Z-Arg-Arg-Pro-Phe-His-Sta-ß-Ala-His-$NH_2$ und H-Arg-Arg-Pro-Phe-His-Sta-ß-Ala-His-$NH_2$, oder ein pharmazeutisch verwendbares Salz einer solchen Verbindung mit mindestens einer salzbildenden Gruppe herstellt.

40. Verfahren nach Anspruch 1, dadurch gekennzeichnet, dass man die Ausgangsstoffe so wählt, dass man eine Verbindung der Formel I, ausgewählt aus der Gruppe Z-Arg-Arg-Pro-Phe-His-Sta-Lac-His-$NH_2$ und H-Arg-Arg-Pro-Phe-His-Sta-Lac-His-$NH_2$, oder ein pharmazeutisch verwendbares Salz einer solchen Verbindung mit mindestens einer salzbildenden Gruppe herstellt.

FO 7.4/VBU/am*